# EUROPÄISCHE PATENTSCHRIFT

(11) **EP 3 337 801 B1**
(45) Veröffentlichungstag und Bekanntmachung des Hinweises auf die Patenterteilung: **12.06.2019**
(21) Anmeldenummer: 16753390.0
(22) Anmeldetag: 18.08.2016
(51) Int. Cl.: C07D 471/04, C25B 3/04, C25B 15/08, C25B 3/02

(54) **VERFAHREN ZUR HERSTELLUNG VON (4S)-4-(4-CYANO-2-METHOXYPHENYL)-5-ETHOXY-2,8-DIMETHYL-1,4-DIHYDRO-1,6-NAPHTHYRIDIN-3-CARBOXAMID UND WIEDERGEWINNUNG VON (4S)-4-(4-CYANO-2-METHOXYPHENYL)-5-ETHOXY-2,8-DIMETHYL-1,4-DIHYDRO-1,6-NAPHTHYRIDIN-3-CARBOXAMID MITTELS ELEKTROCHEMISCHER METHODEN**
METHOD FOR THE PREPARATION OF (4S)-4-(4-CYANO-2-METHOXYPHENYL)-5-ETHOXY-2,8-DIMETHYL-1,4-DIHYDRO-1-6-NAPHTHYRIDINE-3-CARBOXAMIDE AND RECOVERY OF (4S)-4-(4-CYANO-2-METHOXYPHENYL)-5-ETHOXY-2,8-DIMETHYL-1,4-DIHYDRO-1-6-NAPHTHYRIDINE-3-CARBOXAMIDE BY ELECTROCHEMICAL METHODS
PROCÉDÉ DE PRÉPARATION DE (4S)-4-(4-CYANO-2-MÉTHOXYPHÉNYL)-5-ÉTHOXY-2,8-DIMÉTHYL-1,4-DIHYDRO-1,6-NAPHTYRIDINE-3-CARBOXAMIDE ET DE RÉCUPÉRATION DE (4S)-4-(4-CYANO-2-MÉTHOXYPHÉNYL)-5-ÉTHOXY-2,8-DIMÉTHYL-1,4-DIHYDRO-1,6-NAPHTYRIDINE-3-CARBOXAMIDE AU MOYEN DE MÉTHODES ÉLECTROCHIMIQUES

(30) Priorität: 21.08.2015 EP 15182040; 21.08.2015 EP 15182042
(43) Veröffentlichungstag der Anmeldung: 27.06.2018
(73) Patentinhaber: Bayer Pharma Aktiengesellschaft, 13353 Berlin (DE)
(72) Erfinder: PLATZEK, Johannes, 12621 Berlin (DE); GOTTFRIED, Kathrin, 42349 Wuppertal (DE); ASSMANN, Jens, 42781 Haan (DE); LOLLI, Giulio, 51069 Köln (DE)
(74) Vertreter: BIP Patents
(86) Internationale Anmeldenummer: PCT/EP2016/069567
(87) Internationale Veröffentlichungsnummer: WO 2017/032678

(56) Entgegenhaltungen:
- WO-A2-2008/104306
- A Straub ET AL: "Inversion of Optically Active Dihydropyridines by Oxidation and Electroreduction", Angew. Chem. Int. Ed., 1. Januar 1996 (1996-01-01), Seiten 2662-2664, XP055311016, Gefunden im Internet: URL:http://onlinelibrary.wiley.com/store/1 0.1002/anie.199626621/asset/199626621_ftp. pdf?v=1&t=iu9wbw1e&s=a44eeff2d938b70344fd2 42c91b520f832cbdc69 [gefunden am 2016-10-14] in der Anmeldung erwähnt
- KITA Y ET AL: "Selective and facile electroreductive synthesis of dihydro- and tetrahydropyridine dicarboxylic acid derivatives", TETRAHEDRON LETTERS, PERGAMON, GB, Bd. 40, Nr. 49, 3. Dezember 1999 (1999-12-03), Seiten 8587-8590, XP004184851, ISSN: 0040-4039, DOI: 10.1016/S0040-4039(99)01789-X in der Anmeldung erwähnt

## Beschreibung

Die vorliegende Erfindung betrifft ein neues Verfahren Herstellung zur von (4S)-4-(4-Cyano-2-methoxyphenyl)-5-ethoxy-2,8-dimethyl-1,4-dihydro-1,6-naphthyridin-3-carboxamid der Formel (I) und zur Wiedergewinnung von (4S)-4-(4-Cyano-2-methoxyphenyl)-5-ethoxy-2,8-dimethyl-1,4-dihydro-1,6-naphthyridin-3-carboxamid der Formel (I) 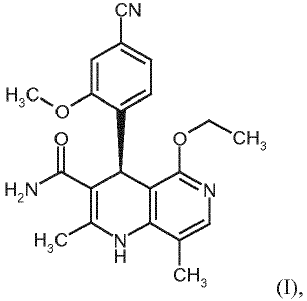 ausgehend von (4R)-4-(4-Cyano-2-methoxyphenyl)-5-ethoxy-2,8-dimethyl-1,4-dihydro-1,6-naphthyridin-3-carboxamid der Formel ent-(I)

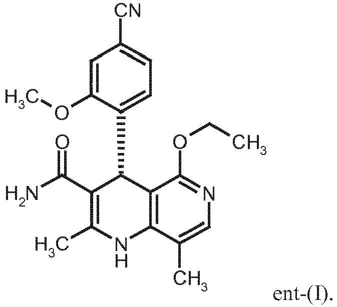

Die Verbindung der Formel (I) wirkt als nicht steroidaler Antagonist des Mineralcorticoid Rezeptors und kann als Mittel zur Prophylaxe und/oder Behandlung von kardiovaskulären und renalen Erkrankungen wie beispielsweise Herzinsuffizienzen und diabetische Nephropathie eingesetzt werden.

Die Verbindung der Formel (I) und deren Herstellungsprozess sind in der WO2008/104306 und ChemMedChem 2012, 7, 1385 beschrieben, wobei in beiden Publikationen eine ausführliche Diskussion der Forschungs-Synthese offenbart ist. Nachteilig an der dort beschriebenen Synthese ist die Tatsache, dass sich diese Synthese nicht für ein großtechnisches Verfahren eignet, da viele Schritte in sehr hoher Verdünnung, mit sehr hohen Reagenz-Überschüssen und damit zu einer relativ geringen Gesamtausbeute verlaufen. Des Weiteren sind viele chromatographische Zwischenreinigungen notwendig, die in der Regel technisch sehr aufwendig sind und einen hohen Verbrauch an Lösungsmitteln erfordern, kostenintensiv sind und damit nach Möglichkeit vermieden werden sollten. Einige Stufen lassen sich aufgrund von sicherheits- und verfahrenstechnischen Schwierigkeiten nicht realisieren.

Es bestand daher der Bedarf nach einer großtechnisch praktikablen Synthese, die die Verbindung der Formel (I) reproduzierbar in hoher Gesamtausbeute, niedrigen Gestehungskosten und hoher Reinheit liefert und allen regulatorischen Anforderungen entspricht, um die klinischen Prüfungen mit Wirkstoff zu beliefern und für eine spätere behördliche Einreichung verwendet zu werden.

Es wurde eine sehr effiziente Synthese gefunden, die es erlaubt die oben genannten Anforderungen zu erfüllen.

In der Publikation ChemMedChem 2012, 7, 1385 in der die Forschungs-Synthese der Verbindung der Formel (I) offenbart ist, wird ausgehend von Vanillin die Verbindung der Formel (I) in 10 Stufen mit einer Gesamtausbeute von 3,76 % der Theorie hergestellt. Die Verbindung der Formel (I) wurde durch Eindampfen von Chromatographie-Fraktionen als amorpher Feststoff erhalten, ein definiertes Kristallisations-Verfahren der Endstufe zur Polymorphie-Einstellung ist bisher nicht beschrieben.

Das nachfolgende Schema 1 zeigt das bekannte Verfahren zur Herstellung der Verbindung der Formel (I).

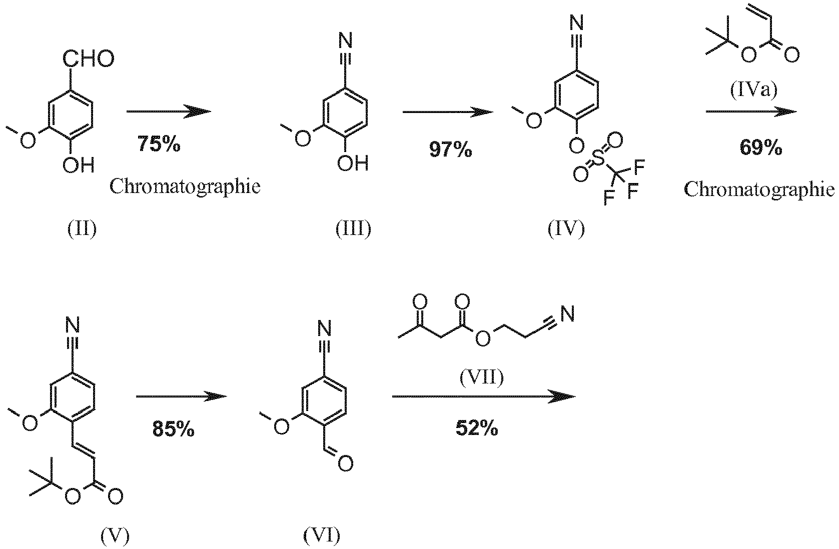

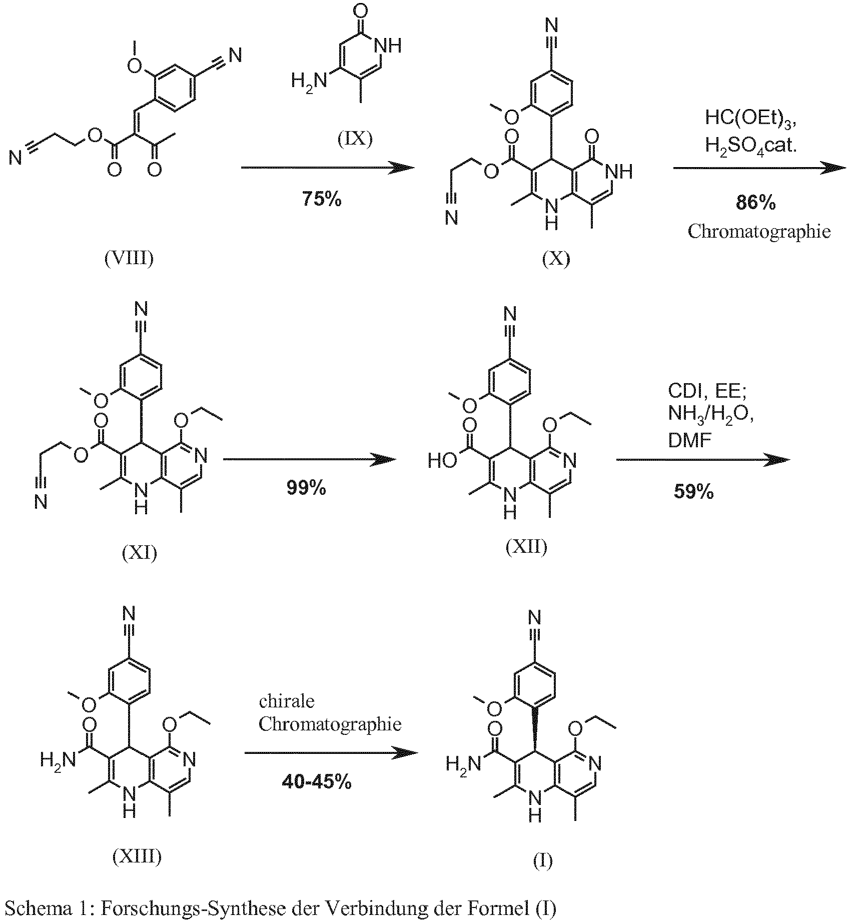

Es werden 3 chromatographische Aufreinigungen benutzt, sowie eine chirale Chromatographie-Stufe zur Auftrennung der Enantiomere des Racemates der Formel (XIII). Die Stufen laufen teilweise in sehr starker Verdünnung und unter Einsatz sehr großer Reagenz-Mengen.

So ist insbesondere die Sequenz der Herstellung der Nitril-Aldehyd Zwischenstufe (VI), die eine zentrale Rolle in dieser Synthese einnimmt aus atomökonomischer Sicht nicht akzeptabel.

Des Weiteren ist dieses Verfahren nicht in den technischen Maßstab zu übertragen, da zum einen sehr teure Reagenzien, wie Trifluormethansulfonsäureanhydrid [(III)=>(IV)] und Überschüsse an Acrylsäure-tert.butylester verwendet werden. Beim Upscaling der Heck-Reaktion (IV)=>(V) bildet sich im Kessel ein Plastik-ähnlicher Rückstand, der aus der Polymerisation des im Überschuss eingesetzten Acrylsäure-tert.butylester herrührt. Dieses ist in der technischen Durchführung nicht akzeptabel, da die Gefahr besteht, dass es zu einem Rührerbruch kommen kann und es zu stark zu entfernenden Rückständen in den Rührwerken führen würde.

Auch die anschließende Spaltung der Doppelbindung mit Natriumperiodat und dem hochgiftigen Osmiumtetroxid ist zu vermeiden, da es unter den beschriebenen Versuchsbedingungen zu einer Verzögerung der Reaktion und dadurch bedingt zu einer starken Exothermie und damit verbunden einem Runaway kommt.

Schema 2 veranschaulicht ein neues Verfahren, welches die Verbindung der Formel (I) in 9 Stufen in 27.7 % d. Th. Gesamtausbeute ohne eine chromatographische Aufreinigung von Zwischenstufen liefert.

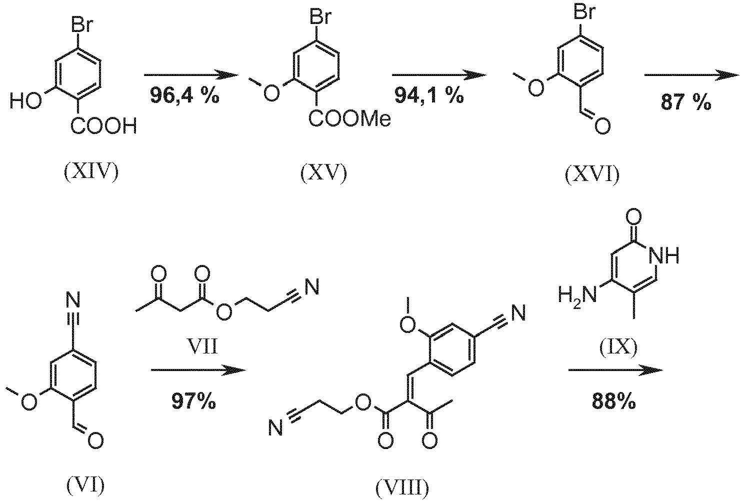

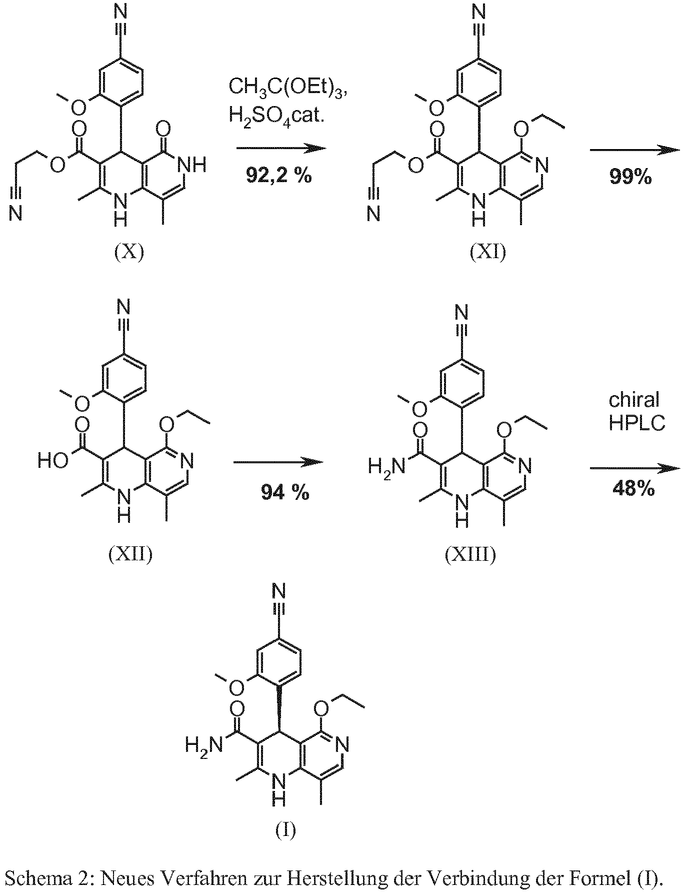

Der Methylester (XV) sowie der Aldehyd (XVI) werden nicht isoliert, sondern direkt in Lösung weiter umgesetzt, wobei dadurch nur 7 zu isolierende Stufen resultieren. Zur Enantiomeren-Trennung wird eine präparative chirale HPLC-Methode (z.B. SMB-Technologie, Varicol) benutzt.

Der Aldehyd (VI) ist literaturbekannt (J. Med. Chem. 2007, 50, 2468-2485) und stellt eine wichtige Zwischenstufe dieser Synthese dar. Es gibt mittlerweile auch die Möglichkeit die Verbindung käuflich zu erwerben.

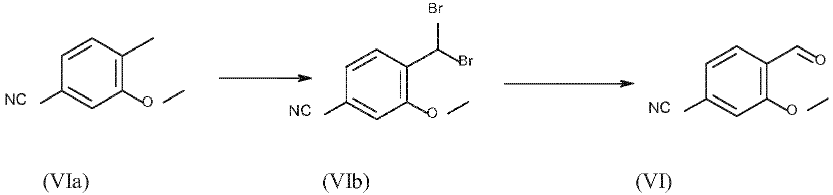

Ausgehend vom 4-Cyano-2-methoxytoluol (VIa) wird mit NBS ein Dibromid (VIb) hergestellt, welches in Ethanol mit 2.46 eq. Silbernitrat (in Wasser) zum Zielaldehyd (VI) umgesetzt wird. Diese in der Literatur beschriebene Synthese sowie das in der Forschungs-Synthese beschriebene Verfahren sind für ein up-scaling in den Multi-Tonnen-Maßstab völlig ungeeignet, sodass ein hoher Bedarf nach einer neuen effizienteren und ökonomisch günstigeren Synthese bestand.

Die Halogenbenzoesäuren (XIV) und (XIVa) 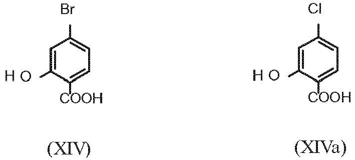 sind in größeren Mengen kommerziell erhältlich. Es wurde ein sehr effizienter und preiswerter Prozess entwickelt, bei dem die Zwischenstufen (XV) und (XVI) 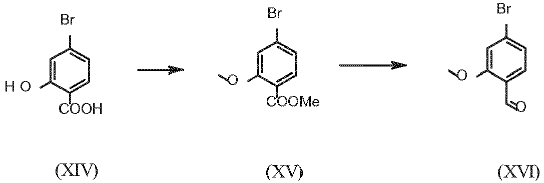 nicht isoliert, sondern in Lösung gelöst, weiter umgesetzt werden. Dies ist nur dadurch möglich, da die Ausbeute und Reinheit der jeweiligen Umsetzung sehr hoch ist (>95%.Th.). Der Methylether-Ester (XV) ist literaturbekannt (Journal of Medicinal Chemistry, 1992, vol. 35, p. 734-740) und wird durch Umsetzung mit dem sehr leichtflüchtigen, gesundheitsschädlichen und teuren Methyliodid durchgeführt.

Mit dem neuen Verfahren konnte gezeigt werden, dass das schwerflüchtige, preiswertere Dimethylsulfat analog verwendet werden kann. Ausgehend von der Säure (XIV) wird in einem Lösungsmittel wie Aceton, 2-Butanon, THF, 2-Methyl-THF, DMF, DMA oder NMP mit Dimethylsulfat unter Zuhilfenahme einer Hilfsbase wie Kaliumcarbonat, Natriumcarbonat, Calciumcarbonat, Lithiumcarbonat, N-Methylimidazol, Triethylamin, Pyridin, 2,6-Lutidin bei Temperaturen von 50-100°C zum Methylether-Ester (XV) umgesetzt. Hierbei handelt es sich um eine dem Fachmann bekannte Methode der Veresterung von Säuren und Veretherung von Phenolen (Tetrahedron, 2013, vol. 69, p. 2807-2815, Journal of the American Chemical Society, 2013, vol. 135, p. 5656-5668). Als besonders bevorzugt hat sich die Umsetzung in Aceton unter Rückfluss (56°C) unter Verwendung von Dimethylsulfat und Kaliumcarbonat herausgestellt. Hierzu wird Dimethylsulfat innerhalb von 4 Stunden zur siedenden Reaktionsmischung zu dosiert. Das Aceton wird abdestilliert und durch Toluol ersetzt (Umdestillation). Zur Aufarbeitung wird Wasser zugesetzt (Zerstörung des überschüssigen Dimethylsulfates), die Toluolphase abgetrennt und mit Wasser und gesättigter Kochsalz-Lösung gewaschen und die Toluol-Lösung anschließend bis auf ein bestimmtes Volumen abdestilliert (dient zur azeotropen Trocknung, d.h. Entfernung von Wasser für die Folgestufe). Eine Gehaltsbestimmung der Lösung zeigt einen nahezu vollständigen Umsatz (> 96% d.Th.) an. Anstelle der Bromverbindung kann analog die Chlorverbindung eingesetzt werden, die erzielten Umsätze sind identisch mit der Bromverbindung.

Die Herstellung vom Aldehyd (XVI) ist in der Literatur beschrieben, beispielhaft genannt seien: Glaxo Group Limited US2008/312209 A1, 2008, European Journal of Medicinal Chemistry, 1986, vol. 21, p. 397-402, Journal of Medicinal Chemistry, 1992, vol. 35, p. 734-740, Journal of Materials Chemistry, 2011, vol. 21, p. 9523-9531. Jedoch sind die in den Umsetzungen eingesetzten Ausgangsstoffe sehr teuer und nicht in großen Mengen erhältlich, daher wurde ausgehend vom Methylether-Ester (XV) ein neues Verfahren entwickelt. Die Umsetzung von (XV) zum Aldehyd (XVI) gelingt mit REDAL (Natrium-bis-(2-methoxy-ethoxy)-aluminium-dihydrid) in Toluol unter Zusatz von N-Methylpiperazin. Diese Methode ist in der Literatur beschrieben (Synthesis 2003, No. 6, 823-828 und Tetrahedron 57 (2001) 2701-2710). Führt man die Reaktion analog der in der Literatur angegebenen Stöchiometrie durch, so wird neben dem Aldehyd noch eine weitere Verbindung im Ansatz gefunden. Es zeigte sich, dass es sich um den korrespondierenden Benzylalkohol handelt, der durch Überreduktion bis zu 10% entsteht. Es zeigte sich, dass es wichtig ist die Stöchiometrie des REDAL und N-Methylpiperazin exakt einzustellen, 1.21 eq. REDAL + 1.28 eq. N-Methylpiperazin, dann gelingt es, dieses Nebenprodukt, welches in der Folgestufe die Kristallisation stört, bis auf <1% zu reduzieren. Hierzu wird eine 65%ige REDAL-Lösung in Toluol bei 0-5°C vorgelegt (bevorzugt 1.21 eq.) und 1.28 eq. N-Methylpiperazin zudosiert. Die so erhaltene Lösung von REDAL mit N-Methylpiperazin wird innerhalb ca. 30 Minuten zur vorgelegten Brommethylester-Lösung (XIV) in Toluol zudosiert und anschließend eine Stunde bei 0°C gerührt. Die Reaktionslösung wird auf Wasser/Säure, bevorzugt aqu. Schwefelsäure gequenscht und die Toluol-Phase abgetrennt und mit Wasser und gesättigter Kochsalzlösung gewaschen. Das Toluol wird abdestilliert und auf DMF (Lösungsmittel der Folgestufe) umdestilliert. Die Ausbeute der Umsetzung beträgt in der Regel >94% d. Theorie. Die entsprechende Umsetzung mit der Chlorverbindung läuft analog, die Ausbeuten sind entsprechend. Die DMF-Lösung wird direkt in die Folgereaktion eingesetzt.

Im weiteren Verlauf der Synthese wird der Bromaldehyd (XVI) in an sich bekannter Weise nach dem Fachmann geläufigen Methoden (Synth. Commun. 1994, 887-890, Angew. Chemie 2003, 1700-1703, Tetrahedron Lett. 2007, 2555-2557, Tetrahedron Lett. 2004, 1441-1444, JACS 2003, 125, 2890-2891, Journal of Organometallic Chemistry 689 (2004), 4576-4583) in das Nitril überführt, hierbei erhält man den Nitrilaldehyd (VI). Im Falle der Bromverbindung hat es sich als besonders vorteilhaft erwiesen, eine Palladium katalysierte Umsetzung mit Kaliumhexacyanoferrat*3H₂O als Cyanid-Quelle durchzuführen (Tetrahedron Lett. 48 (2007), 1087-1090). Hierzu wird der Bromaldehyd (XVI) in DMF (8-10 fach) vorgelegt, 0.22 eq. Kaliumhexacyanoferrat*3H₂O und 1 eq. Natriumcarbonat vorgelegt und anschließend 0.005 eq. Palladiumacetat zugesetzt. Man erwärmt 3 Stunden auf 120°C. Die Lösung wird auf 20°C abgekühlt, anschließend Wasser und Essigester zugesetzt. Die Essigester Phase wird abgetrennt, die Wasser Phase erneut mit Essigester nachgewaschen und anschließend die vereinigten Essigester-Phasen auf Isopropanol umdestilliert. Man fällt das Produkt durch Wasser Fällung in der Siedehitze aus. Nach Isolierung wird im Vakuum getrocknet. In manchen Fällen wurde das Produkt direkt durch Zugabe von Wasser aus dem DMF ausgefällt und nach Isolierung und Trocknung direkt in die Folgestufe eingesetzt. Die Ausbeuten dieser Umsetzung sind in der Regel > 85% d.Th. Für die Umsetzung der Chlorverbindung reicht Palladiumacetat nicht aus, hier hat es sich als vorteilhaft erwiesen die dem Fachmann geläufigen Palladium-Katalysatoren, wie in Tetrahedron Lett. 48 (2007), 1087-1090 beschrieben, einzusetzen, die Ausbeuten sind etwas niedriger als bei der Bromverbindung, in der Regel 80-85% d.Th.

Den Zimtester (VIII a,b) erhält man ausgehend vom Aldehyd der Formel (VI) in einer Knoevenagel-Reaktion mit dem Cyanoester (III) als E/Z-Gemisch.

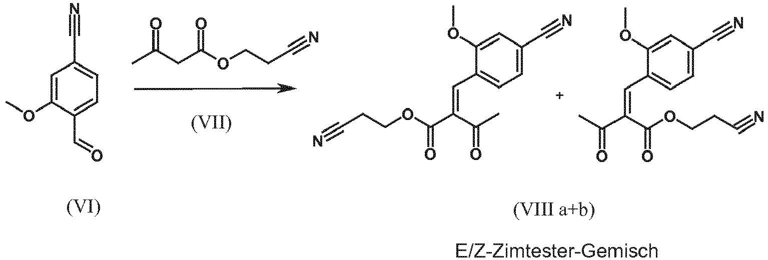

In der Forschungsvorschrift wurde in 16.6 fach Dichlormethan und 0.2 eq. Piperidin/0.2 eq. Eisessig 20 Stunden am Wasserabscheider erhitzt. Nach wässriger Aufarbeitung wird nach Eindampfen des Lösungsmittels aus Methanol kristallisiert, man erhält die Zielverbindung in 52% d.Theorie.

Die Reaktion läuft bevorzugt in siedendem Dichlormethan (10-fach) unter Zusatz von 5-20 mol% Piperidin, bevorzugt 10 mol% und 5-20 mol% Eisessig, bevorzugt 5-10 mol% am Wasserabscheider ab. Die Reaktionszeit beträgt 4-12 h, bevorzugt aber 5-6 h, besonders bevorzugt 6 h. Der Cyanoester (VII) wird in 1.0-1.5 eq, bevorzugt aber 1.1 bis 1.35 eq. zugesetzt. Besonders bevorzugt 1.1 eq. Die Herstellung des Cyanoesters (VII) ist in Pharmazie, 2000, vol. 55, p. 747-750 und Bioorg. Med. Chem. Lett. 16, 798-802 (2006) beschrieben. Nach beendeter Reaktion wird auf 20°C abgekühlt und die organische Phase 2mal mit Wasser gewaschen. Die organische Wasche wird auf 2-Butanol umdestilliert und das E/Z-Zimtestergemisch (VIII a+b) ohne Zwischenisolierung direkt die Folgereaktion mit dem Heterocyclus (IX) zum Dihydropyidin (X) eingesetzt:

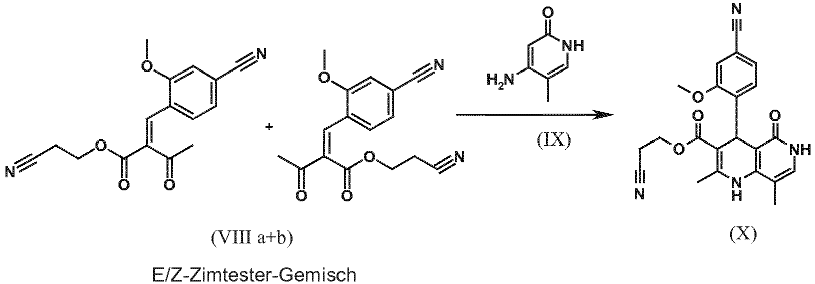

In der Forschungs-Synthese wurde für die weitere Umsetzung mit dem Heterocyclus (IX) in Isopropanol 40 Stunden unter Rückfluss erhitzt.

Es wurde gefunden, dass die Reaktion bevorzugt in einen sekundären Alkohol, wie Isopropanol, Isobutanol, 2-Amylalkohol, Cyclohexanol bei Temperaturen von 80-160°C, unter Normaldruck, sowie im Autoklaven (2-10 bar), Reaktionszeiten 8-40 h, bevorzugt aber wird 20-25 h in siedendem 2-Butanol unter Normaldruck oder aber in Isopropanol im Autoklaven (100°C, 2-10 bar, bevorzugt 3-5 bar, 8-24 h) durchgeführt werden kann. Zur Aufarbeitung wird auf 0°C bis 20°C abgekühlt, die Kristalle abfiltriert und mit Isopropanol nachgewaschen, anschließend getrocknet (im Vakuum, 60°C).

Wenn auf die Verwendung von Dichlormethan aus umweltökonomischen Gründen verzichtet werden soll, hat sich als vorteilhaft erwiesen den Zimtester (VIII a,b) in Isopropanol herzustellen, hierzu wird der Aldehyd (VI) in Isopropanol (3-9 fach, bevorzugt 5-7 fach) vorgelegt und 5-20 mol% Piperidin, bevorzugt 5-10 mol% und 5-20 mol% Eisessig, bevorzugt 5-10 mol% zugegeben. Bei 30°C wird 1.0-1.5 eq, bevorzugt 1.1-1.35 eq., besonders bevorzugt 1.1 eq. Cyanoester (VII), gegebenenfalls in wenig Isopropanol gelöst, über 3 Stunden zudosiert und 1 Stunde bei 30°C nachgerührt. Der Zimtester (VIIIa,b) kristallisiert während der Reaktion aus. Anschließend wird das Produkt, gegebenenfalls nach Abkühlen, bevorzugt bei 0°C abfiltriert, mit wenig Isopropanol (auf 0°C gekühlt) gewaschen und feucht in die Folgereaktion wie oben beschrieben eingesetzt. Die Ausbeute beträgt >96% d. Theorie. Bevorzugt wird in der Folgeumsetzung in 10-15 fach (bzg. auf Aldehyd (VI)), bevorzugt 11-12 fach Isopropanol 20-24 Stunden bei 100°C unter Druck gearbeitet. Das Produkt wird nach beendeter Reaktion und Abkühlen durch Filtration oder Zentrifugation isoliert. Anschließend wir bei 40-90°C im Vakuum getrocknet. Da der Umsatz zum Zimtester nahezu quantitativ verläuft, kann man den Prozess für die Folgestufe leicht standardisieren, ohne jeweils die Menge an Heterocylus (IX) anpassen zu müssen, daher kann das Produkt Isopropanol feucht eingesetzt werden. Die Ausbeuten liegen bei > 87% d. Theorie. Der Heterocyclus (IX) kann nach literaturbekannten Methoden, wie z.B. in Synthesis 1984, 765-766 beschrieben, hergestellt werden.

Ausgehend vom Dihydropyridin (X) wird der Ethylether (XI) durch Umsetzung unter saurerer Katalyse mit einem Orthoester erhalten, wobei R für -H und -Methyl steht:

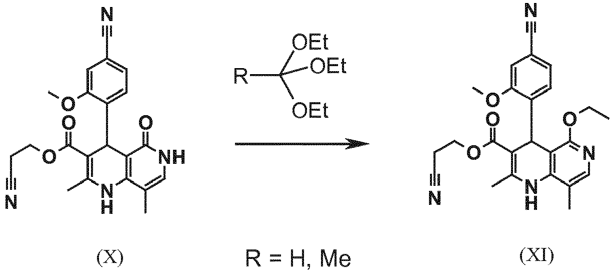

In der Forschungs-Synthese wurde hierzu in 25 fach DMF mit 20.2 equ. Triethylorthoformiat, katalytischer Menge konz. Schwefelsäure bei 135°C umgesetzt. Es wurde zur Trockene eingeengt und der Rückstand durch Chromatographie gereinigt, Ausbeute 86% d. Theorie. Dieses Verfahren ist aufgrund der starken Verdünnung und der Verwendung des bei niedriger Temperatur leicht entflammbaren Triethylorthoformiats, dass in sehr großem Überschuss eingesetzt wird und der anschließenden Chromatographie für eine technische Durchführung ungeeignet.

Es wurde überraschenderweise gefunden, dass die Reaktion viel konzentrierter (bis zu 1.5 g Lösungsmittel auf 1 g Edukt) in Lösungsmitteln wie Dimethylacetamid, NMP (1-Methyl-2-pyrrolidon), DMF (Dimethylformamid) unter Zusatz von 4-10 gew.% konz. Schwefelsäure, bevorzugt 6-8 gew.% durchgeführt werden kann. Die Reaktion läuft dann überraschenderweise bereits mit 2.5-5 eq. Orthoester. Es wurde gefunden, dass es viel günstiger ist, den entsprechenden Triethylorthoessigester in die Reaktion einzusetzen, da er zum einen viel sauberer reagiert und viel schwerer entflammbar ist, was ihn für die technische Durchführung besonders prädestiniert. Die Umsetzung erfolgt bevorzugt in DMA (Dimethylacetamid) und NMP (1-Methyl-2-pyrrolidon), bei Temperaturen von 100-120°C, bevorzugt 115°C. Es hat sich als vorteilhaft erwiesen, vor Start der eigentlichen Reaktion ein Teil des Lösungsmittels (DMD oder NMP) bei erhöhter Temperatur (100-120°C unter Vakuum) abzudestillieren, um gegebenenfalls vorhandene Reste an Isopropanol aus der Vorstufe zu entfernen, da ansonsten unerwünschte Nebenprodukte auftreten. Umsetzung: Man rührt 1.5-3 Stunden, bevorzugt 2 Stunden. Zur Aufarbeitung wird Wasser direkt in den Ansatz gegeben, wobei das Produkt auskristallisiert. Um ein besonders stabiles und reproduzierbares Verfahren in der Hand zu haben, wir erst eine Teilmenge Wasser (z.B. 1/3) zudosiert, dann angeimpft und die Restmenge Wasser zugegeben. Dieses Vorgehen garantiert, dass man immer die gleiche Kristallmodifikation erhält, die das beste Isolierverhalten zeigt. Das Produkt wird mit Wasser nachgewaschen und getrocknet. Die Ausbeuten liegen >92% d. Theorie.

Ausgehend vom Ethylether (XI) wird die Säure (XII) durch alkalische Verseifung und anschließender saurer Aufarbeitung erhalten:

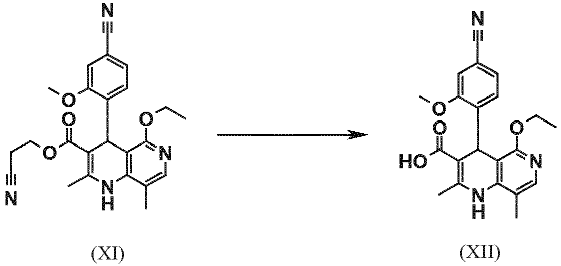

In der Forschungs-Synthese wurde die Verseifung unter starker Verdünnung (33.9 fach) in einem Gemisch aus DME/Wasser 3:1 durchgeführt. Hier galt es in erster Linie den Durchsatz zu erhöhen und das verwendete DME (Dimethoxyethan), welches einen sehr niedrigen Flammpunkt besitzt und damit für die großtechnische Verwendung als besonders kritisch zu bewerten ist, zu ersetzen. Es wurde überraschenderweise gefunden, dass man die Reaktion auch sehr leicht in Gemischen aus THF/Wasser viel konzentrierter durchführen kann. Hierzu wird bevorzugt in einem Gemisch aus THF/Wasser 2:1 (9-fach) gearbeitet, die Natronlauge wird bei 0-5 °C zu dosiert, anschließend 1-2 Stunden bei 0-5 °C gerührt. Es kann auch Kalilauge verwendet werden, bevorzugt wird aber NaOH verwendet. Zur Aufarbeitung wird mit MTBE (Methyl-tert-butylether) und Essigester extrahiert und zur Isolierung mit einer Mineralsäure, wie Salzsäure, Schwefelsäure oder Phosphorsäure, bevorzugt aber Salzsäure, auf pH 6.5 - 7.0 gestellt. Anschließend wird mit gesättigter Ammoniumsalz-Lösung der entsprechenden Säure, bevorzugt aber Ammoniumchlorid-Lösung, versetzt, wobei das Produkt quantitativ auskristallisiert. Nach Isolierung wird mit Wasser und mit Essigester oder Acetonitril oder Aceton, bevorzugt aber Acetonitril nachgewaschen und im Vakuum bei 40-50 °C getrocknet. Die Ausbeute ist nahezu quantitativ (99%). Alternative bevorzugte Aufarbeitung: Alternativ wird zur Aufarbeitung mit Toluol versetzt, Natriumacetat zugegeben und bei 20°C gerührt, anschließend die Phasen getrennt und die wässrige Phase bei 0°C mit 10%iger aqu. Salzsäure auf pH 6.5-7.0 gestellt (bei pH 9.5-10 kann gegebenenfalls angeimpft werden). Man lässt kurz nachrühren und filtriert das Produkt ab, wäscht mit wenig Wasser und Toluol nach und trocknet bei 40-50°C im Vakuum. Auch in diesem Fall sind die erzielten Ausbeuten quantitativ.

Die Folge-Umsetzung von der Säure zum Amid (XIII) wurde in der Forschung wie folgt durchgeführt: Die Säure (XII) wurde in ca. 10 fach DMF gelöst, 1.25 eq. 1,1'-Carbodiimidazol und 0.1 eq. DMAP (4-(Dimethylamino)-pyridin) zugegeben und 4 Stunden bei Raumtemperatur gerührt. Anschließend wurden 20 eq. Ammoniak in Form einer wässrigen 25%igen Lösung zugesetzt und diese Mischung in ein auf 110°C vortemperiertes Ölbad übertragen. Bei dieser Prozedur entstehen augenblicklich größerer Mengen an Ammoniak-Gas, die dem System entweichen und zusätzlich für einen starken Druckanstieg sorgen. Es wurde auf ca. 90 fach Wasser gegeben und durch Zugabe von Natriumacetat auf pH 7 gestellt. Das ausgefallene Produkt wurde abfiltriert und getrocknet (Ausbeute: 59% d. Theorie). Aus der Mutterlauge wurde durch aufwendige Extraktion (ca. 100 fach Essigester) eine weitere Teilmenge isoliert, die aus hochentzündlichem Diethylether ausgerührt wurde und ca. 14% DMF enthielt. Es liegt auf der Hand, dass ein solches Verfahren in keiner Weise im betrieblichen Rahmen realisiert werden kann und somit ein hoher Bedarf nach einer alternativen Verfahrensweise bestand. Der Aufwand zur Isolierung dieser Teilmenge steht in keinem Verhältnis zur dabei isolierten Menge.

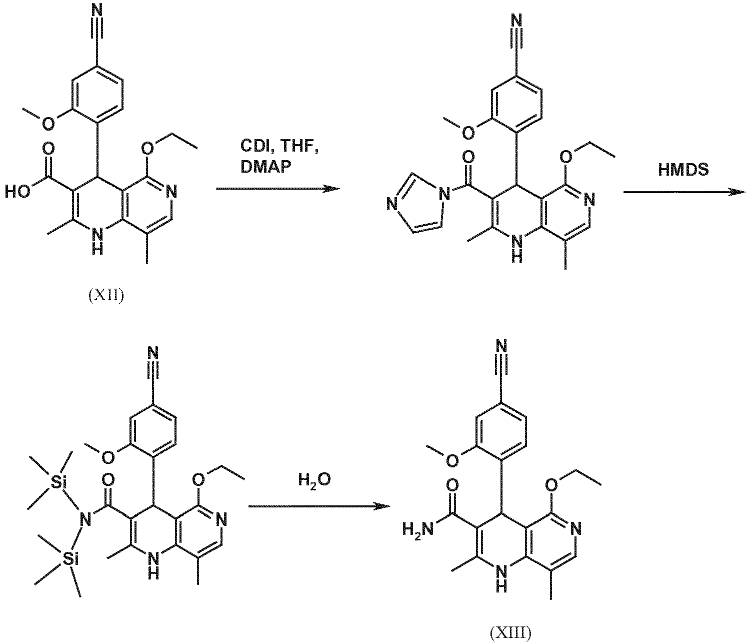

Es wurde überraschenderweise gefunden, dass bei einer Umsetzung der Säure (XII) in THF das Amid (XIII) direkt aus der Lösung auskristallisiert und in hoher Ausbeute und Reinheit erhalten werden kann. Hierzu wird die Carbonsäure (XII) mit 1.1 bis 1.6 eq., bevorzugt 1.3-1.4 eq. 1,1'-Carbodiimidazol unter DMAP-Katalyse (5-15 mol %, bevorzugt 10 mol %) in THF zum Imidazolid umgesetzt, dieses erfolgt bei Temperaturen zwischen 20-50°C, als bevorzugtes Vorgehen hat sich erwiesen, zuerst bei 20°C zu starten, anschließend 1 bis 2 Stunden bei dieser Temperatur zu rühren und dann 2 bis 3 Stunden bei 50°C nachzurühren. Nach beendeter Aktivierung setzt man 3-8 eq, bevorzugt 4.5 eq. Hexamethyldisilazan zu und kocht 16-24 Stunden, bevorzugt jedoch 16 Stunden unter Rückfluss. Die hierbei entstehende Disilylamid-Verbindung kann gegebenenfalls isoliert werden, es hat sich aber als vorteilhafter erwiesen in einer Eintopf-Reaktion weiter fortzufahren. Nach beendeter Reaktion wird daher auf 0-3°C abgekühlt und eine Mischung aus Wasser oder einem Gemisch aus Wasser und THF zudosiert, als vorteilhaft hat sich erwiesen eine Wassermenge von 0.5 bis 0.7 fach (bzg. auf Edukt) zu benutzen, besonders vorteilhaft ist eine Menge von 0.52 fach Wasser. Das Wasser kann direkt, oder im Gemisch mit ca. der einfach bis doppelten Volumenmenge THF zudosiert werden. Nach beendeter Quentschung wird, insgesamt 1-3 Stunden, bevorzugt 1 Stunde zum Rückfluss erhitzt. Man kühlt auf 0°C ab und rührt 1-5 Stunden, bevorzugt 3 Stunden bei dieser Temperatur nach, anschließend wird das Produkt durch Filtration oder Zentrifugieren isoliert. Man wäscht mit THF und Wasser nach und trocknet im Vakuum bei erhöhter Temperatur (30 bis 100°C, bevorzugt bei 60°C bis 90°C). Die Ausbeuten sind sehr hoch und betragen in der Regel > 93% d. Theorie. Die Reinheit ist in der Regel > 99% (HPLC, 100%-Methode). Die Verbindung (XIII) kann auch direkt durch Umsetzung mit Ammoniakgas im Autoklaven (ca. 25 bis 30 bar) erhalten werden. Dazu führt man die oben beschriebene Voraktivierung durch und erhitzt anschließend unter Ammoniak-Gas unter Druck. Nach beendeter Reaktion wird abgekühlt und das Produkt abfiltriert. Die so erzielten Ausbeuten und Reinheiten sind vergleichbar.

Um an die Verbindung der Formel (I) zu gelangen, muss das racemische Gemisch der Amide der Formel (XIII) in die Antipoden getrennt werden. In der publizierten Forschungs-Synthese wurde hierzu eine speziell synthetisierte chirale Phase verwendet (Eigenherstellung), die als chiralen Selektor N-(dicyclopropylmethyl)-N²-methacryloyl-D-leucinamid enthielt. Dieser Selektor wurde in einer Mehrstufen-Stufe hergestellt und dann auf spezielles Kieselgel aufpolymerisiert. Als Laufmittel diente Methanol/Essigester. Ein großer Nachteil dieser Methode war die sehr geringe Beladung, 30 mg pro Trennung auf einer 500*63 mm Chromatographie-Säule, sodass ein hoher Bedarf bestand eine möglichst effektive Trennmethode zu finden, die es erlaubt eine Antipoden-Trennung im multi Tonnen-Bereich vorzunehmen. Es wurde überraschenderweise gefunden, dass man die Trennung auch auf einer kommerziell leicht zugänglichen Phase vornehmen kann. Hierbei handelt es sich um die Phase Chiralpak AS-V, 20 µm. Als Laufmittel wurde eine Mischung aus Methanol/Acetonitril 60:40 verwendet. Diese Mischung hat den großen Vorteil, dass es nach destillativer Aufarbeitung als Laufmittel wieder zurückgewonnen werden kann, mit der identischen Zusammensetzung (60:40 entspricht dem Azeotrop). Auf diese Weise erreicht man einen sehr effizienten Prozess, bei der die Ausbeute der Trennung >47% d. Theorie beträgt (50% sind theoretisch möglich). Die optische Reinheit beträgt hier >93% e.e. bevorzugt aber >98.5% e.e. Hierbei kann die Chromatographie an einer handelsüblichen Chromatographie-Säule durchgeführt werden, bevorzugt werden aber mit dem Fachmann bekannten Techniken wie SMB oder Varicol (Computers and Chemical Engineering 27 (2003) 1883-1901) eingesetzt. So wurde beispielsweise mit einer SMB Anlage ca. 500 kg des racemischen Amids der Formel (XIII) getrennt, wobei eine Ausbeute von 48% erzielt wurde. Das Produkt bringt man als 3-8%ige, bevorzugt 5-7%ige Lösung in einem Gemisch aus Methanol/Acetonitril 60:40 aus und kann es direkt ins "Final Processing" einsetzen. Auch andere Lösungsmittelgemisch-Verhältnisse zwischen Acetonitril und Methanol sind denkbar (90:10 bis 10:90). Es können aber alternativ auch andere Lösungsmittel-Gemische, wie Acetonitril/Ethanol in Gemisch-Verhältnissen von 10:90 bis 90:10 zur SMB Trennung verwendet werden. Das jeweilige Verhältnis der Lösungsmittel hängt teilweise von den technischen Eigenschaften der SMB-Anlage ab und muss gegebenenfalls angepasst werden (z.B. verschiedene Flussrate, Recycling der Lösungsmittel am Dünnschicht-Verdampfer).

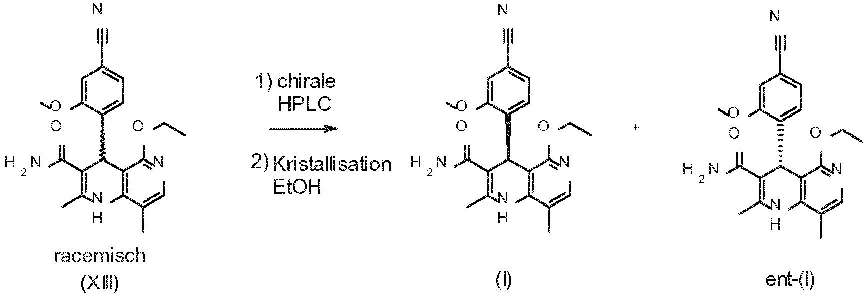

Neben der Zielverbindung der Formel (I) wird auch die enantiomere Verbindung der Formel ent-(I) in nahezu gleicher Ausbeute gewonnen. Aufgrund kostenökonomischer Gesichtspunkte bestand der Bedarf dieses Enantiomer der Formel ent-(I) nicht zu vernichten, sondern ein Verfahren zu erfinden, das es ermöglicht die Verbindung der Formel ent-(I) in ein racemisches Gemisch der Formel (XIII) zu überführen, um es erneut einer Enantiomeren-Trennung mittels SMB zu unterziehen.

Diese Aufgabe konnte durch Anwendung des erfindungsgemäßen indirekten elektrochemischen Verfahrens zur Oxidation von 1,4-Dihydropyridin-Derivaten der Formel (A) zu Pyridin-Analoga der Formel (B) 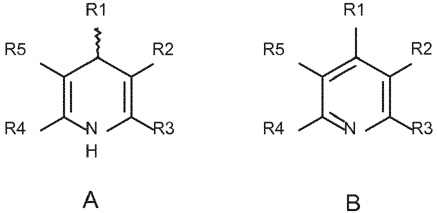 und nachfolgender Reduktion gelöst werden.

Zur Synthese von pharmazeutischen Wirkstoffen wird die Oxidation von 1,4-Dihydropyridin-Derivaten, wie dem in Formel (A) beschriebenen, in Pyridin-Analoga (B) 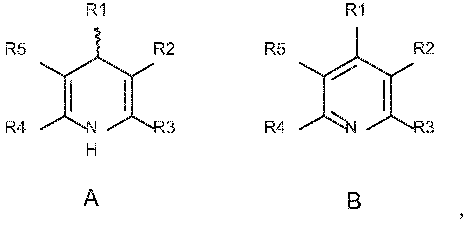 wobei R1-R5 unabhängig voneinander Wasserstoff, Fluor, Chlor, Brom, Iod, Carboxyl, Carboxylester, Hydroxyl, Hydroxyether, Cyano, Nitro, substituiertes und unsubstituiertes Amid, (C₁-C₆)-Alkyl, Halo(C₁-C₆)-Alkyl, Formyl, substituiertes und unsubstituiertes Phenyl, substituiertes und unsubstituiertes Benzyl, substituiertes und unsubstituiertes Naphthyl, substituiertes und unsubstituiertes 5- oder 6-gliedriger Heterocyclus mit mindestens einem Heteroatom ausgewählt aus der Gruppe N, S, O, benzokondensierter 5- oder 6-gliedrieger Heterocyclus bedeuten, durch Einsatz chemischer Oxidationsmittel durchgeführt.

Han et al. [Org. Lett. 2014, 16, 4142-4145] beschreiben einen Oxidationsschritt eines 1,4-Dihydropyridin-Derivats (C) [4-(3,6-Dihydro-2H-pyran-4-yl)-7,7-dimethyl-5-oxo-2-(propan-2-yl)-1,4,5,6,7,8-hexahydrochinolin-3-carbonsäuremethylester] unter Verwendung von 1.2 Äquivalenten DDQ [2,3-Dichlor-5,6-dicyano-1,4-benzochinon]. Dabei wird eine isolierte Ausbeute des Pyridin-Derivats (D) [4-(3,6-Dihydro-2H-pyran-4-yl)-7,7-dimethyl-5-oxo-2-(propan-2-yl)-5,6,7,8-tetrahydrochinolin-3-carbonsäuremethylester] von 93.5 Gew.-% erhalten.

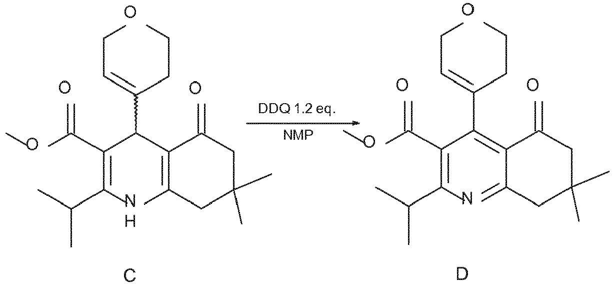

Nachteilig ist bei diesem Verfahren die große Menge an Oxidationsmittel (DDQ), die zur Abstraktion von zwei Protonen und zwei Elektronen von dem Substrat benötigt wird. Im besten Fall werden zur Vervollständigung der Reaktion stöchiometrische Mengen chemischer Oxidationsmittel benötigt. In den meisten Fällen wird ein Überschuss chemischer Reagenzien verwendet, um vollständige Umwandlung und maximale Ausbeute zu gewährleisten. Somit fällt eine Menge Abfall an, und außerdem werden durch die Verwendung großer Mengen von Oxidationsmittel auch die Herstellungskosten erhöht.

In Analogie zu der Arbeit von Han et al. kann man davon ausgehen, dass diese Methode für alle 1,4-Dihydropyridin-Derivate (DHP) der Formel A und die entsprechenden Pyridine (PYR) der Formel (B) gültig ist.

Eine erfindungsgemäße Anwendung des oben beschriebenen Oxidationsverfahrens ist ein neuartiges Verfahren zur Herstellung von (4S)-4-(4-Cyano-2-methoxyphenyl)-5-ethoxy-2,8-dimethyl-1,4-dihydro-1,6-naphthyridin-3-carboxamid (I) durch einen Recycling-Prozess aus dem Enantiomer der Formel ent-(I), das beim Herstellungs-Prozess von Verbindung (I) anfällt.

Dies gelingt, indem man das Fehlenantiomer der Formel ent-(I) zuerst zum Pyridin der Formel (XVII) oxidiert (aromatisiert) und anschließend elektrochemisch reduziert:

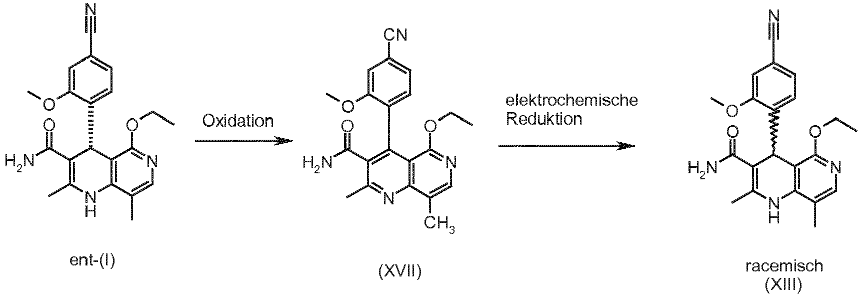

Die nachfolgende Beschreibung erläutert das neue erfinderische Verfahren:

Im ersten Schritt erfolgt eine Oxidation (Aromatisierung) der Verbindung der Formel ent-(I);

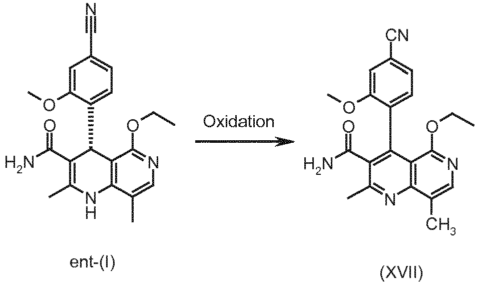

Als Oxidationsmittel können die dem Fachmann geläufigen Oxidationsmittel zur Aromatisierung von Piperidinen und Dihydropyridinen verwendet werden, diese sind beispielhaft im Buch: Pyridines: From Lab to Production; Edited by Eric F.V. Scriven, Elsevier Verlag 2013, Kapitel 8, Seite 116-144 beschrieben. Beispielhaft genannt seien DDQ in Dichlormethan, Chloranil in Dichlormethan, Mangandioxid in Dichlormethan, Kaliumpermanganat in Aceton, Mangan(III)acetat in Eisessig, Cerammoniumacetat in Acetonitril, Pyridiniumchlorochromat in Dichlormethan, konz. Salpetersäure in Dichlormethan, Jod in Methanol. Besonders bevorzugt ist DDQ oder konz. Salpetersäure in Dichlormethan. Die Ausbeuten sind im Allgemeinen sehr hoch, in der Regel > 86% d.Th.

Aus früheren Arbeiten (A. Straub, Tetrahedron Asymmetry 12 (2001) 341-345) gab es Hinweise, dass die oxidierten Dihydropyridine, also die Pyridyl-Aryle eine gehinderte Drehbarkeit zeigen. Die Rotations-Barriere ist so hoch ist, dass man die Antipoden bei Raumtemperatur trennen kann (axiale Chiralität → Atropisomerie). Daher wurden ausgehend von den Racematen präparative chirale Chromatographie-Methoden entwickelt, um diese in die Antipoden zu trennen. Dieses gelang überraschender Weise auch im vorliegenden Fall

| Trennung der racemischen Verbindung der Formel (XVII) | Chirale stationäre Phase | Laufmittel |
|---|---|---|
| | Chiralpak AS-H (250 x 4 mm) | i-Hexan : Ethanol = 50 : 50 |

Bei den beiden anfallenden Atropisomeren handelt es sich auch um die Haupt-Metabolite (Verbindungen der Formeln Mla(S) und M1b(R)), die nach Applikation der Verbindung der Formel (I) in vivo beobachtet werden. Ihre Absolut-konfiguration konnte durch Röntgenkristallstrukturanalyse bestimmt werden (siehe Beispiel-Teil)

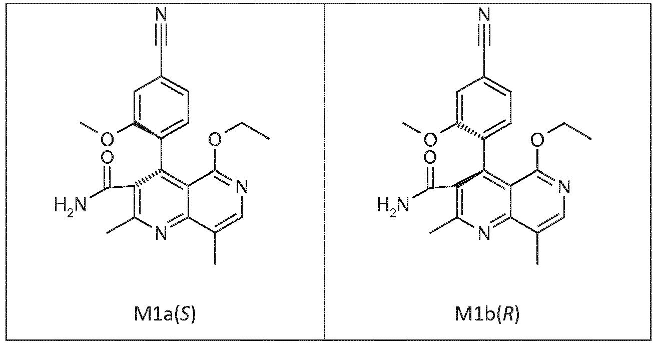

Überraschend ist die Tatsache, dass die optisch aktive Titelverbindung der Formel (I) mit der S-Konfiguration in Nagern und Säugetieren, sowie dem Menschen (Hund, Ratte, Maus, Mensch) hauptsächlich zu Mla(S) metabolisiert wird. Bietet man das R-Enantiomer der Formel ent-(I) an, 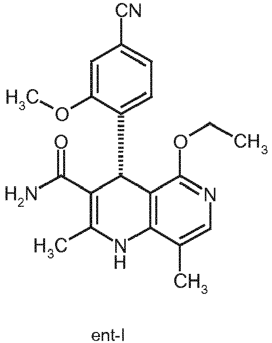 so wird hauptsächlich der Metabolit der Formel Mlb(R) gebildet.

Wird zum Beispiel eine Oxidation mit chemischen Oxidationsmitteln durchgeführt, so entsteht überwiegend der Metabolit der anderen Serie, aus Titelverbindung der Formel (I) (S-konfiguriert) entsteht überwiegend die Verbindung der Formel Mlb(R), aus der Verbindung der Formel ent-(I) (R-konfiguriert) entsteht überwiegend die Verbindung der Formel Mla(S).

Setzt man die optisch aktive Verbindung der Formel ent-(I) mit verschiedenen dem Fachmann geläufigen Oxidationsmitteln um, so erhält man folgende Ergebnisse:
Falls kein Lösungsmittel angegeben ist, wurde standardmäßig Dichlormethan als Lösungsmittel benutzt. Das Verhältnis wurde mittels einer chiralen HPLC-Methode gemessen, M1a(S)/M1b(R) wurde auf 100% normiert. Die Ausbeute wurde als Umsatz mittels HPLC (achirale Methode) gemessen

| Oxidationsmittel | M1b(R) Metabolit | M1a(S) Metabolit | Ausbeute/% (M1a(S)+M1b(R)) HPLC | Reaktions-Dauer (h) |
|---|---|---|---|---|
| TEMPO* BF₄ | 7,3% | 92,7% | 77 | 1 |
| DDQ, rt | 10,1% | 89,9% | 96 | 0,5 |
| DDQ, 0 °C | 10,3% | 89,7% | 97 | 4 |
| DDQ, -20 °C | 10,7% | 89,3% | 97 | 4 |
| Chloranil | 14,3% | 85,7% | 98 | 68 |
| NHPT, Co(II) cat. (MeCN) | 37,5% | 62,5% | 90 | 18 |
| MnO₂, 10x | 19,0% | 81,0% | 92 | 23 |
| KMnO₄ (Aceton) | 8,2% | 91,2% | 89 | 19 |
| KMnO₄ (AcOH) | 23,6% | 76,4% | 75 | 18 |
| RuCl₃ (AcOH) | 11,8% | 88,2% | 42 | 72 |
| Mn(III)OAc (AcOH) | 17,8% | 82,2% | 98 | 17 |
| CAN | 42,5% | 57,5% | 90 | 18 |
| CAN (MeCN) | 13,5% | 86,5% | 96 | 64 |
| Bi(NO₃)₄ | 47,2% | 52,8% | 84 | 18 |
| PCC | 20,0% | 80,0% | 97 | 40 |
| I₂ (MeOH) | 20,7% | 79,3% | 77 | 18 |
| HNO₃ (konz) | 25,5% | 74,5% | 97 | 95 |
| **A**/NaIO₄, rt | 40,0% | 60,0% | 48 | 48 |
| **A**/NBu₄IO₄, rt | 42,6% | 57,4% | 80 | 24 |
| **A**/NBu ₄IO ₄, 0 °C | 49,2% | 50,8% | 80 | 18 |
| **A**/NBu₄IO₄, -10 | 52,2% | 47,8% | 82 | 55 |

Die verwendeten Reagenzien sind in der nachfolgenden Tabelle wiedergegeben:

| | | |
|---|---|---|
| 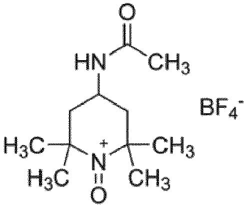 | 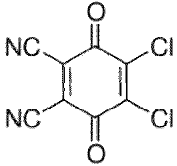 | 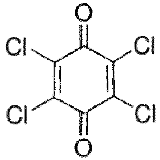 |
| TEMPO* BF4 | DDQ | Chloranil |
| 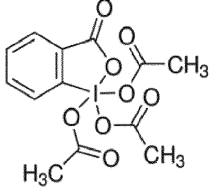 | 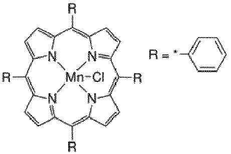 | 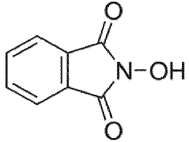 |
| DMP | A | NHPT |

Es konnte gezeigt werden, daß sich die einzelnen Antipoden auch thermisch racemisieren lassen, hierzu wird in einem Lösungsmittel mit einem erhöhten Siedepunkt >70°C erhitzt, man kann aber auch in einem niedrig siedenden Lösungsmittel arbeiten, muss dann aber unter Druck fahren. Als Lösungsmittel kommen alle gängigen Lösungsmittel, wie Ethanol, Methanol, Propanol, Isopropanol, THF, Dioxan, Methylenchlorid (unter Druck), DMF, DMA, NMP, Essigester, 2-Me-THF in Frage. Bevorzugt wird in 1-Butanol und Ethanol gearbeitet.

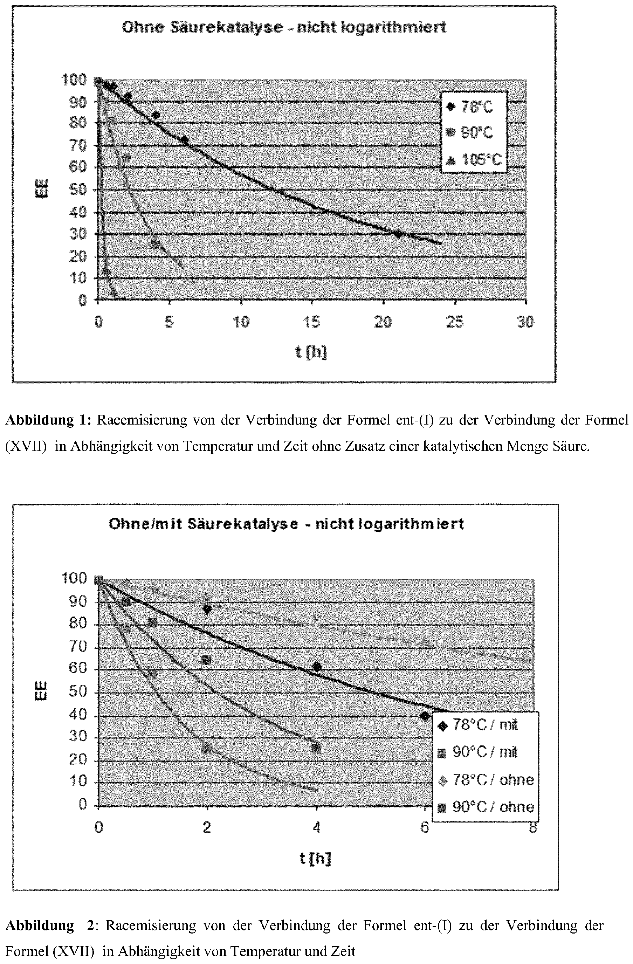

Beispielhaft sei die thermische Racemiserung in 1-Butanol (ca. 20 fach gelöst) erwähnt. Hierzu wurde der Enantiomeren-Überschuss e.e.% der Verbindung der Formel Mla(S) in Butanol bei 3 verschiedenen Temperaturen bestimmt (siehe Abbildung 1). Man erkennt, dass bei 105°C innerhalb von 1 h vollständige Racemisierung eintritt. Die Racemisierungs-Geschwindigkeit lässt sich durch Säure-Zusatz (katalyt. Mengen Methan-sulfonsäure in 1-Butanol) beschleunigen (siehe Abbildung 2), Durch den Zusatz einer katalytischen Menge an Säure kann die Thermische Racemisierung auch bei tieferen Temperaturen durchgeführt werden. Als Säuren kommen Methansulfonsäure, Schwefelsäure, Salzsäure, p-Toluolsulfonsäure, sowie die meisten aromatischen Sulfonsäuren in Frage. Bevorzugt werden aber die Sulfonsäuren, besonders bevorzugt Methansulfonsäure verwendet.

Ein großer Nachteil der oben angeführten Oxidations-Methoden ist, dass man stöchiometrische, bzw. überstöchiometrische Mengen an Oxidationsmittel einsetzen muss und man auf diese Weise viel Abfall erzeugt. Es bestand daher der Wunsch die Abfallmenge an Oxidationsmittel so gering wie möglich zu halten. Dieses gelingt mit der vorliegenden Erfindung. Der Einsatz katalytischer Mengen DDQ reduziert die Abfallmenge deutlich auf ein Minimum, was ein erheblicher Vorteil des neuen erfinderischen Verfahrens darstellt.

Die beste Alternative zur chemischen Oxidation wäre die elektrochemische Oxidation unter Ersatz von chemischen Oxidationsmitteln durch Elektronen. Durch Einsatz von Elektrochemie ist es möglich, das Oxidationspotential fein einzustellen und auf die Verwendung von chemischen Reagentien zu verzichten. Arguello et al. [Electrochemica Acta 49 (2004) S. 4849-4856] und Lopez-Alarcon et al. [Electrochimica Acta 48 (2003) S. 2505-2516] beschreiben die voltammetrische Oxidation von Hantzsch-1,4-Dihydropyridinen in protischen und aprotischen Medien. Sie berichteten jedoch über hohe Oxidationspotentiale, die zwischen +915 mV und +1093 mV gegen Ag/AgCl-Referenzelektrode in aprotischem Medium variierten. Bei diesem hohen Oxidationspotential treten bekanntlich Oxidationen von funktionellen Gruppen, z.B. Aminogruppen oder Phenolgruppen, auf [a) Handbook of electrochemistry, Elsevier, Herausgeber C.G. Zoski, 2007; b) Fundamentals and Applications of organic Electrochemistry; Fuchigami et al., 2015 John Wiley & Sons, Ltd; c) David et al., Tetrahedron 51 (1995) 3181-3196]. Somit ist die direkte elektrochemische Oxidation von Dihydropyridin-Derivaten nur beschränkt anwendbar.

Als Alternative zur direkten elektrochemischen Oxidation beschreiben Francke und Little die Anwendung von indirekten elektrochemischen Reaktionen unter Verwendung verschiedener Arten von Mediatoren im Allgemeinen [Chem. Soc. Rev. 43(8) 2014 S. 2492-2521]. Es werden keine Beispiele angeführt, in denen Dihydropyridine erfolgreich zu ihren Pyridin-Analoga oxidiert werden konnten. Der Einsatz von DDQ bei der indirekten Elektrosynthese wird erwähnt, ist aber gemäß dem Kommentar der Autoren noch nicht vollständig erforscht. Beispiele beschränken sich auf benzylische Oxidation, d.h. Seitenkettenfunktionalisierung in wässriger Essigsäure. Bei Verwendung trockener aprotischer Lösungsmittel verlief die Reaktion nicht erfolgreich.

Eine Aufgabe der Erfindung bestand in der Entwicklung eines Verfahrens zur Oxidation von Dihydropyridinen (A) zum Pyridin-Analogon (B), 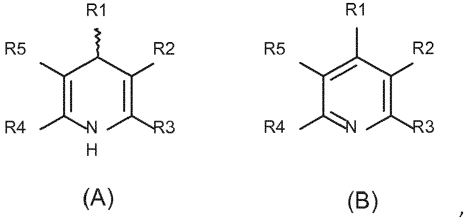 wobei
R1-R5 unabhängig voneinander Wasserstoff, Fluor, Chlor Brom, Iod, Carboxyl, Carboxylester, Hydroxyl, Hydroxyether, Cyano, Nitro, substituiertes und unsubstituiertes Amid, (C₁-C₆)-Alkyl, Halo(C₁-C₆)-Alkyl, Formyl, substituiertes und unsubstituiertes Phenyl, substituiertes und unsubstituiertes Benzyl, substituiertes und unsubstituiertes Naphthyl, substituiertes und unsubstituiertes 5- oder 6-gliedriger Heterocyclus mit mindestens einem Heteroatom ausgewählt aus der Gruppe N, S, O, benzokondensierter 5- oder 6-gliedrieger Heterocyclus bedeuten,
dadurch gekennzeichnet, dass
i) unterstöchiometrischen Oxidationsreagenzien eingesetzt werden und das Verfahren
ii) für Seitenketten und eine Reihe von Substituenten unter milden Bedingungen tolerierbar ist.

Zur Lösung dieser Aufgabe wurde überraschenderweise gefunden, dass Dihydropyridinderivate unter Verwendung von indirekter elektrochemischer Oxidation mit unterstöchiometrischen Mengen von Mediatoren erfolgreich in hohen Ausbeuten in ihr Pyridin-Analogon oxidiert werden können.

Optimale Reaktionsbedingungen für das erfindungsgemäße Verfahren sind die Temperatur von 1-100°C, bevorzugt 10-50°C, besonders bevorzugt 20-30°C bei Normaldruck und Oxidationspotentiale von -0.1 V bis +0.6 V gegen Ag/Ag+-Referenzelektrode (10 mmol/l), bevorzugt 0.0 V bis +0.5 V und besonders bevorzugt 0.1 V bis 0.4 V gegen Ag/Ag+-Referenzelektrode (10mmol/l) (gemessen in aprotischen organischen Lösungsmitteln).

Unter sehr milden Bedingungen, d.h. Raumtemperatur (25°C) und Normaldruck und kleinen Oxidationspotentialen (+0.4 V gegen Ag/Ag+ 10 mmol/l) im Vergleich zur direkten elektrochemischen Oxidation (>+1 V gegen Ag/Ag+ 10 mmol/1), wurden hohe Ausbeuten an Pyridin-Derivat erreicht. Es gab keine Anzeichen für Seitenkettenoxidation, und die Reaktion konnte auch in aprotischen Lösungsmittel gefahren werden. Darüber ist unseres Wissens nach in der Literatur noch nie zuvor berichtet worden. Das nächstliegende Beispiel aus der Literatur ist eine benzylische Oxidation. Diese war in wässriger Essigsäure erfolgreich, scheiterte aber, sobald trockene aprotische Lösungsmittel (wie das von uns eingesetzte) verwendet wurden. [Chem. Soc. Rev. 43(8) 2014 S. 2492-2521].

Die Menge an Mediator, z.B. DDQ, konnte auf weniger als 10 Mol-% (idealerweise etwa 2% unter Beibehaltung einer Produktausbeute >95%) verringert werden, und ein Oxidationspotential von nur 0.3-0.4 V gegen Ag/Ag+-Referenzelektrode war für einen hohen Umsatz, eine hohe Ausbeute sowie eine hohe Stromeffizienz ausreichend.

Geeignete Mediatoren auf die die Erfindung nicht beschränkt sein soll sind: Triarylamine (Typ Ar3N), TEMPO und andere N-oxyl Radikale, Halogenid-Salze (Typ HX mit X = Cl, Br, I), Metall-Salze (Cr(VI)/Cr(III), Fe(III)/Fe(II), V(IV)/V(III), Ce(IV)/Ce(III), Co(III)Co(II), Ru(VIII)/Ru(IV), Os(VIII)/Os(VI), Mn(III)/Mn(II)), Iodbenzol und Iodbenzolderivate, Nitrat Salze und Triarylimidazol, in Chem. Soc. Rev. 43(8) 2014 S. 2492-2521 zitiert.

Für die elektro-organische Synthese werden dem Fachmann bekannte Elektrolyse-Apparate, die sogenannten "Drei-Elektroden-Systeme" eingesetzt [Handbook of Electrochemistry; Herausgeber C.G. Zoski; 2007 Elsevier B. V. & Fundamentals and Applications of Organic Electrochemistry: Synthesis, Materials, Devices, First Edition, T. Fuchigami, M. Atobe und S. Inagi; 2015 John Wiley & Sons, Ltd]. Hierbei werden dem Namen nach drei Elektroden eingesetzt, die Arbeits-, Gegen-, und Referenzelektrode. Es gibt eine Vielzahl an Referenz-Elektroden, wobei für nicht-wässrige Elektrolyte, d.h. organische Lösungsmittel, die Silber/Silber-Kation (Ag/Ag+) Referenzelektrode bedingt durch ihre Stabilität und hohe Reproduzierbarkeit der Messungen bevorzugt eingesetzt wird. Hierbei wird ein Silber-Draht in eine 10 mM oder 0.1 M AgNO3 Lösung eingetaucht. Als Lösungsmittel können Acetonitril, Dimethylformamid oder Dimethylsulfoxid verwendet werden. Als Leitsalz wird standardmäßig Tetrabutylammoniumperchlorat (BuN4ClO4) verwendet. Alternativ können aber auch andere Leitsalze eingesetzt werden: Et4NBF4, Bu4NBF4, Bu4NPF6, Bu4NX(mit X = I, Br) oder Perchlorate (NaClO4, LiClO4, Et4NClO4).

Eine räumliche Trennung zwischen Arbeits- und Gegenelektrode bzw. beider sogenannten Halbzellen ist in den meisten Fällen vorteilhaft, um zu verhindern, dass sowohl Edukte als auch das zu erzeugende Zielprodukt an die Gegenelektrode gelangen und dort unerwünschte Nebenreaktionen auslösen, wodurch Ausbeuteverluste resultieren würden.

Für die räumliche Trennung von Arbeits- und Gegenelektrode werden Separatoren eingesetzt, die durch eine begrenzte Porosität und oder auch durch ihren chemischen Aufbau bzw. Funktionalität einen beliebigen Austausch zwischen beiden Halbzellen verhindern. Bekannte Separatoren sind gesinterte Glasfritten, PTFE-Filtermembranen, Kationenaustauscher-Membranen, Polyvinylidenfluorid- oder Polypropylen-Filtermembranen sowie hier nicht weiter aufgelistete Materialien, die beständig gegenüber organischen Lösungsmitteln sind und deren Porengrößen klein genug sind, um einen Übertritt von Edukt und Produkt in die andere Halbzelle einzuschränken bzw. ganz zu verhindern.

Für die elektrochemische Oxidation des Dihydro-Pyridins (A) wird die Arbeitselektrode als Anode und die Gegenelektrode als Kathode geschaltet. Bei Kathode wird Wasserstoffentwicklung erwartet und beobachtet.

Bekannte Elektrodenmaterialien sind Platin, Palladium, Gold, Graphit, Glaskohlenstoff, Bor-dotierter Diamant, Zink, Kupfer, Nickel, Zinn, Samarium, Stahl, Quecksilber, Blei oder Legierungen bestehend aus Kupfer, Zinn und Blei, sogenannte Bleibronzen. Ferner sind dem Fachmann weitere Metall und Metalloxid Elektroden bekannt, die auch dotiert oder in Legierungen eingesetzt werden: Ru/RuO2, Ti/TiO2, RuO2/TiO2, Ir/IrO2, Pt/Ti, Platin/Iridium.

Insbesondere ist kathodisch die Bildung von gasförmigem Wasserstoff als konkurrierende Reaktion dem Fachmann bekannt. Daher sind Kathoden Materialien bevorzugt, die eine hohe Überspannung gegenüber der Wasserstoffbildung aufweisen. So nimmt die Überspannung für die H₂-Bildung in folgender Rangfolge zu: Pd<Au<Pt<Ni<Cu<Sn<Pb<Zn<Hg.

Typische und auch für elektro-organische Synthesen beschriebene Lösungsmittel sind Acetonitril, Ethanol, Tetrahydrofuran (THF), Aceton, N,N-Dimethylformamid (DMF), Methanol, Dichlormethan, Dimethylsulfoxid (DMSO), Hexamethylphosphoramide ([(CH3)2N]3PO; CAS:680-31-9). Dem Fachmann allgemein bekannte Lösungsmittel sind ferner NMP, N,N-Dimethylacetamid, Propanol, Isopropanol, Methylenchlorid, Essigester.

Leitsalze, die organischen Lösungsmittel zur Erhöhung der Leitfähigkeit zugefügt werden sind: Et4NBF4, Bu4NBF4, Bu4NPF6, Bu4NX(mit X = I, Br) oder Perchlorate (NaClO4, LiClO4, Et4NClO4, Bu4NClO4).

Die im Detail beschriebenen weitverbreiteten "Drei-Elektroden-Systemen", kommen in der Regel in dem Fachmann bekannten Becherglas-Zellen, H-Zellen oder anderen Behältnissen zur Anwendung. Mittels Magnetrührern können die Reaktionsansätze kontinuierlich gerührt werden. Es handelt sich mehrheitlich um Batch-Experimente, bei denen das Lösungsmittel/Leitsalzgemisch in beiden Halbzellen vorgelegt wird. Das Edukt wird nur in die Halbzelle eingefüllt, in der es auch elektrochemisch umgesetzt werden soll.

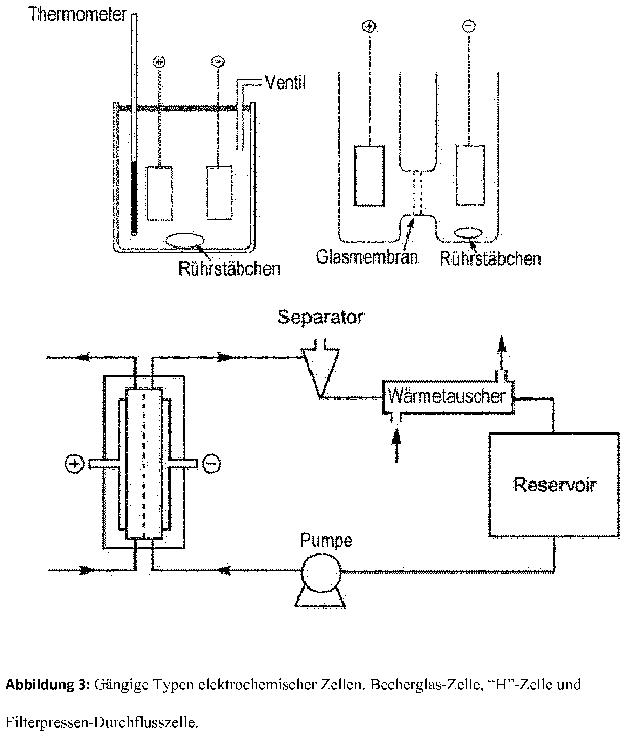

Durch kontinuierliche Zirkulation des Reaktionsgemisches mittels Kreislauf-Pumpen können solche Zellen auch im Durchfluss betrieben werden. Daneben sind in der Literatur sehr spezielle Geometrien für Durchflusszellen beschrieben [Handbook of Electrochemistry; Herausgeber C.G. Zoski; 2007 Elsevier B. V. & Fundamentals and Applications of Organic Electrochemistry: Synthesis, Materials, Devices, First Edition, T. Fuchigami, M. Atobe und S. Inagi; 2015 John Wiley & Sons, Ltd]. Siehe Abbildung 3. Besonders bevorzugt sind Durchflusszellen im Filterpressen Design mit Blick auf ein Scale-up der Synthese. Ausgehend von sehr kleinen Querschnittsflächen (10cm2) lässt sich das Scale-up einerseits durch eine Vergrößerung der Querschnittsfläche auf bis zu 0.4 m² pro Modul (kommerziell verfügbar bei der Firma Electrocell, Model MFC bis 0.001 m², Model MPC von 0.01 bis 0.2 m², Model ESC von 0.04 bis 1.04 m², Model EPC von 0.4 bis 16.0 m²) und andererseits durch ein Numbering-Up, also das Koppeln mehrerer identischer Module in einen Stack realisieren. Das Risiko eines solchen Scale-up Prozesses ist überschaubar, da die anderen geometrischen Dimensionen wie z.B. der Elektrodenabstand, das Elektrodenmaterial (für Anode und Kathode) als auch die Betriebsparameter (insbesondere die Stromdichte) nicht mehr verändert werden müssen.

Mittels regelbarer Durchflussgeschwindigkeit lässt sich die Verweilzeit in der Zelle kontrollieren. Typische Verweilzeiten liegen im Bereich 0.1-100s pro Durchlauf ("Single pass"). Für das erfindungsgemäße Verfahren sind bei Anwendung von Durchflusszellen bei der elektrochemischen Reduktion Verweilzeiten bevorzugt von 0.5-50s und besonders bevorzugt sind Verweilzeiten pro Durchlauf von 1-10s.

Die Auswahl der Stromdichte hängt sowohl von der Verweilzeit als auch der Kinetik der Zielreaktion als auch von unerwünschten Nebenreaktionen ab. Eine zu hohe Stromdichte bei gleichzeitig langer Verweilzeit und Gasbildung (z.B. H₂) würde zur Abschirmung der Elektrodenfläche durch die Ausbildung eines Gaspolsters in der Zelle führen. Für die elektrochemische Oxidation von (XIII) zu (XVII) mit DDQ als Mediator sind Stromdichten von 1-100 mA/cm² denkbar. Bevorzugt sind jedoch Stromdichten im Bereich 5-50 mA/cm² und besonders bevorzugt im Bereich 10-30 mA/cm² um bei ausreichender Raumzeit-Ausbeute maximale Selektivität zu erzielen. Die Anwendung unterschiedlicher Lösungsmittel aus obiger Liste ist grundsätzlich möglich. Bevorzugte Lösungsmittel sind DMF, DMA, NMP, Acetonitril und deren Gemische.

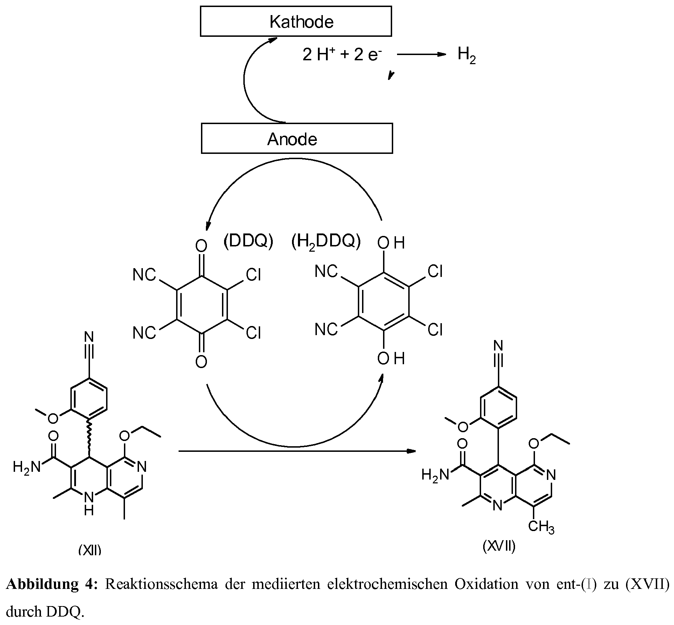

Zur Durchführung des erfindungsgemäßen Verfahrens im Falle von Verbindung ent-(I) hat sich folgendes Vorgehen bewährt:
Die Oxidation von Verbindung ent-(I) in das entsprechende Derivat (XVII) mit DDQ als Mediator verläuft gemäß dem folgenden Schema in Abbildung 4, sie lautet also ent-(I) → (XVII) + H₂ unter Anwendung von Spannung und elektrischem Strom (siehe Abbildung 4).

Zur Untersuchung und zum besseren Verständnis des Systems wurde Cyclovoltammetrie in einer ungeteilten Zelle vom Becher-Typ mit einem Durchmesser von 5 cm mit einer Pt-Käfigelelctrode (Arbeitselektrode) auf der Außenseite und einer Pt-Drahtelektrode (Gegenelektrode) in der Mitte durchgeführt. In der Nähe der Arbeitselektrode wurde eine Ag/Ag⁺-Referenzelektrode (10 mmol/l in Acetonitril) angeordnet. Die Zelle wurde mit 100 ml Acetonitril gefüllt, worin 2.17 g (10 mmol) Tetraethylammoniumtetrafluoroborat (Et4NBF4) zusammen mit 22.7 mg (0.1 mmol) DDQ und 378.4 mg (1 mmol) der Verbindung der Formel ent-(I) gelöst wurden. Für die Cyclovoltammetrien ohne die Verbindung der Formel ent-(I) oder ohne DDQ wurde die entsprechende Menge nicht zugegeben. Cyclovoltammetrien wurden unter Verwendung eines Potentiostaten der Bauart Gamry Interface 1000 mit einer Abtastrate von 250 mV/s und 100 mV/s über 10 Zyklen zwischen -0.5 und +1 V gegen Referenzelektrode aufgenommen. Nach Ausschluss des ersten und des letzten Zyklus wurde das Ergebnis gemittelt. Die Cyclovoltammographie ist dem Fachmann als eine Möglichkeit zur Untersuchung von elektrochemischen Reaktionen an der Elektrodenoberfläche bekannt.

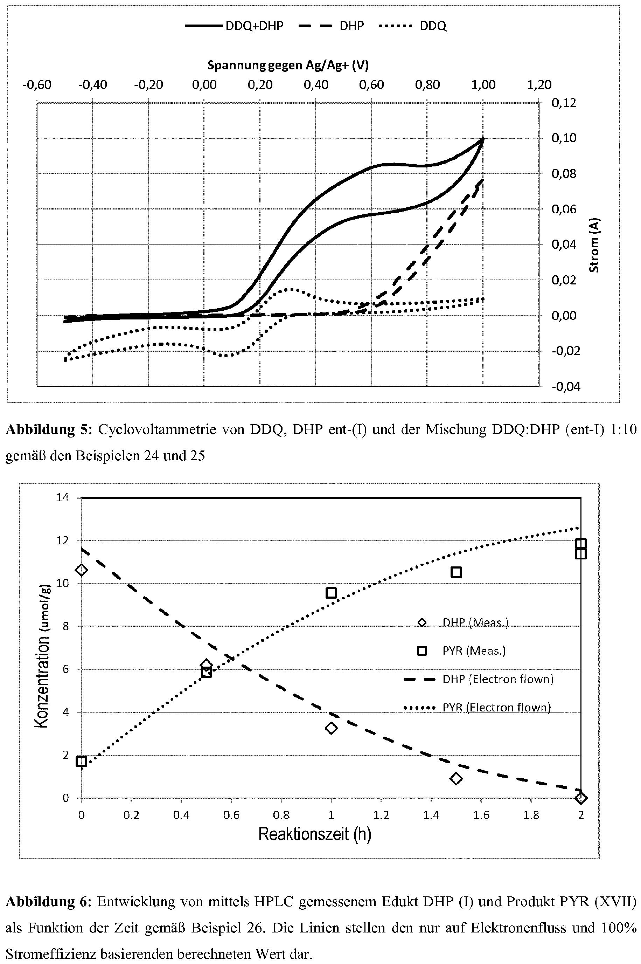

Die Ergebnisse der Cyclovoltammographie-Tests sind in Abbildung 5 angegeben. Es ist klar ersichtlich, dass im Fall von DDQ ohne das Substrat ent-(I) - gepunktete Linie - 2 Peaks klar erkennbar sind. Ein Reduktionspeak (negativ) bei etwa +0.1 V gegen Ag/Ag⁺, der mit der Reaktion DDQ→H₂DDQ assoziiert ist, und ein Oxidationspeak (positiv) bei etwa +0.3 V gegen Ag/Ag⁺, der mit der Reaktion H₂DDQ→DDQ assoziiert ist. Außerdem ist das Cyclovoltammogramm vollkommen symmetrisch, was bedeutet, dass die Reaktionen vollkommen reversibel sind.

Bei Betrachtung des Cyclovoltammogramms des Substrats ent-(I) ohne DDQ (gestrichelte Linie), also in Direktoxidationsmodus, ist ersichtlich, dass ent-(I) nur oberhalb von 0.6 V gegen Ag/Ag⁺ oxidiert werden kann und zum Erhalt eines annehmbaren Umsatzes mindestens 1 V benötigt wird (siehe Abbildung 5). Nach 1 h Elektrolyse bei +1.0 V gegen Ag/Ag waren eine Verfärbung der Lösung und das Vorliegen mehrerer Nebenkomponenten bei der HPLC festzustellen. Eine genaue Identifizierung und quantitative Bestimmung war nicht möglich. Dies war zu erwarten, da aus der Literatur [a) Handbook of electrochemistry, Elsevier, Herausgeber C.G. Zoski, 2007; b) Fundamentals and Applications of organic Electrochemistry; Fuchigami et al., 2015 John Wiley & Sons, Ltd] bekannt ist, dass Amine und Amide (die beispielsweise in dem Molekül vorliegen) zwischen +0.5 und +1.0 V gegen GKE oxidiert werden können (Handbook of Electrochemistry, Seite 819).

Beim Vergleich mit der mediierten Elektrolyse, also Substrat + 10 Mol-% DDQ (durchgezogene Linie) ist die Bildung eines sehr effektiven Charge-Transfer-Komplexes ersichtlich, wobei das Substrat bei dem gleichen Potential von DDQ (etwa 0.3 V) oxidiert werden kann und das Verfahren sehr effektiv ist und somit den höchsten Strom zeigt. Außerdem ist ersichtlich, dass die umgekehrte Reaktion (Reduktionspeak) vollkommen verschwunden ist, da DDQ nun nur mit dem Substrat reagieren kann und nicht mehr für die Elektrode verfügbar ist.

Es sei darauf hingewiesen, dass nach Reagierenlassen des Systems bei dem Potential von +0.4 V gegen Ag/Ag⁺ über einen Zeitraum von 2 h ein Umsatz des Substrats von ungefähr 98% erzielt wurde und keine Nebenkomponenten beobachtet wurden, sondern nur die gewünschte Komponente. Siehe auch Beispiele. Dies ist mit der Direktumwandlung zu vergleichen, bei der nur eine geringe Selektivität erreicht wurde.

Somit kann ein ideales Arbeitsfenster (Potential zwischen 0.3 und 0.5 V) definiert werden, in dem die Regeneration von DDQ (also die Oxidation H₂DDQ → DDQ) maximal ist (>0.3 V) und die direkte, unselektive Reaktion des Substrats mit der Elektrode (<0.5 V) vollkommen vermieden wird. Dies ist das ideale Arbeitsfenster, das die maximale Ausbeute und Selektivität erlaubt.

Es sei auch darauf hingewiesen, dass unter derartigen Bedingungen (d.h. mit +0.4 V gegen Ag/Ag⁺ als Referenz) ein hoher Strom von 65 mA und somit eine hohe Reaktionsrate von 1.2 mmol/h erreicht werden konnte. Zum Erhalt der gleichen Rate in einem nicht mediierten direkten elektrochemischen System ist eine Spannung von +1.0 V anzulegen, die das Molekül schädigen wird.

In Analogie zur Cyclovoltammetrie wurde in der gleichen Batch-Zelle mit der gleichen Lösung und Konfiguration ein Produktionstest durchgeführt. Danach wurde die Lösung bei einem konstanten Potential von +0.4 V 2 Stunden elektrolysiert und alle 15 Minuten eine Probe entnommen und mittels HPLC analysiert. Nach Ablauf der 2 Stunden erreichte der Umsatz überraschenderweise 98%, die Produktausbeute war > 97.5% (Selektivität >99%), und noch überraschender war nur eine Ladung von 2.1 F geflossen. In Anbetracht der Tatsache, dass die benötigte stöchiometrische (minimale) Strommenge 2 F beträgt, lag die Stromeffizienz über 95%. Wenn eine geringe Stromeffizienz kein Anzeichen für eine unselektive Reaktion ist, sind selektive Reaktionen eine notwendige Bedingung für hohe Stromeffizienzen. Hieraus geht zusammen mit der HPLC-Analytik eindeutig hervor, dass die mediierte Oxidation von (I) zu (XVII), oder allgemeiner von (A) zu (B), hinsichtlich Selektivität und Ausbeute ein viel günstigerer Prozess ist als die chemische und direkte elektrochemische Oxidation (siehe Abbildung 6).

Die Verbindung (XIII) existiert in 2 enantiomeren Formen: (I) und ent-(I). Das Produkt (XVII) existiert in 2 Axialchiralitätsformen, die als Mla(S) und Mlb(R) bekannt sind. Überraschenderweise wurde gefunden, dass durch Umwandlung mittels mediierter elektrochemischer Reduktion gemäß obiger Beschreibung die Verbindung der Formel (I) bevorzugt in Mlb(R) umgewandelt wird, wobei das Verhältnis Mla(S):Mlb(R) 13:87 beträgt (Beispiel 27), und ent-(I) bevorzugt in Mla(S) umgewandelt wird, wobei das Verhältnis Mla(S):Mlb(R) 90:10 beträgt (Beispiel 26). Diese Ergebnisse sind mit dem durch chemische Oxidation mit stöchiometrischen Mengen von DDQ erhaltenen Ergebnis (Beispiele 11a und 11b) vergleichbar. Überraschenderweise wird die Verbindung der Formel (I) in Tierzellen (einschließlich Menschen) bevorzugt zu der Verbindung der Formel Mla(S) metabolisiert, die Verbindung der Formel ent-(I) dagegen zu der Verbindung der Formel M1b(R).

Bei Verwendung eines racemischen Gemischs der Verbindung der Formel (I), also der Verbindung der Formel (XIII), führt die elektrochemische Oxidation wie erwartet zu einem racemischen Gemisch von (XVII) mit einem Verhältnis Mla(S):Mlb(R) von 50:50 (Beispiel 28).

Angesichts der beobachteten hohen Selektivität und Stromeffizienz könnte die obige Verfahrensweise auch ohne besondere Schwierigkeiten in Durchflusszellen des oben beschriebenen Typs (d.h. von Fa. Electrocell) durchgeführt werden. Dies erlaubt eine höhere Raumzeit-Ausbeute und eine größere Produktion im technischen Maßstab.

Isolierung von (XVII): Nachdem die elektrochemische Umsetzung erfolgt ist (Edukt (I) in der Regel < 1%) wird die Reaktionslösung aufgearbeitet. Die Umsetzung verläuft in hohen Ausbeuten (>98%) und überraschend sauber mit nahezu keinen Verunreinigungen. Es hat sich als vorteilhaft erwiesen, dass man das Lösungsmittel zuerst weitgehend abdestilliert und anschließend das Produkt durch eine Wasser-Fällung (Zugabe von Wasser) ausfällt, abfiltriert und trocknet. Das so erhaltene Produkt kann aus Ethanol oder Isopropanol oder 1-Butanol, oder 2-Butanol umkristallisert werden.

Im nächsten Schritt wird eine elektrochemische Reduktion des Pyridins der Formel (XVII) zum Dihydropyridin durchgeführt:

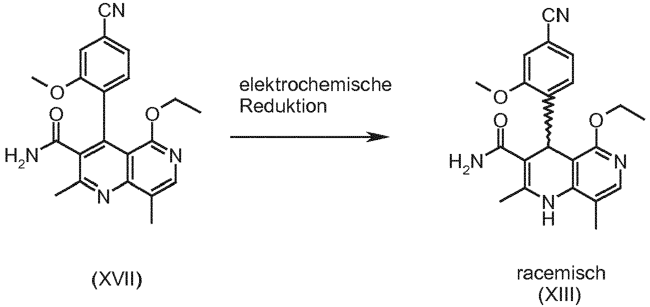

### Stand der Technik zur (elektro)chemischen Reduktion von Pyridinen

Straub und Goehrt [Alexander Straub und Axel Goehrt, Angew. Chem., 108 (1996), 2832-2834 (Titel: Inversion optisch aktiver Dihydropyridine durch Oxidation und Elektroreduktion)] beschreiben die elektrochemische Reduktion von Pyridin-Derivaten, die u.a. alle durch die Anwesenheit einer Ester-Gruppe (-CO2Et) charakterisiert sind, an Quecksilber-Elektroden. Auch die von Kita et al. [Yoshio Kita, Hirofumi Maekawa, Yasuhiro Yamasaki und Ikuzo Nishiguchi, Tetrahedron Letters 40 (1999) 8587-8590 (Titel: Selective and facile electroreductive synthesis of dihydro- and tetrahydropyridine dicarboxylic acid derivatives);Yoshio Kita, Hirofumi Maekawa, Yasuhiro Yamasaki und Ikuzo Nishiguchi, Tetrahedron 57 (2001) 2095-2102 (Titel: Highly selective and facile synthesis of dihydroand tetrahydropyridine dicarboxylic acid derivatives using electroreduction as a key step)] verwendeten Pyridine weisen sogar zwei Ester-Substituenten (-CO2Me) auf. Straub und Goehrt geben 83% Ausbeute für einen sehr kleinen Laboransatz von 0.72 mmol Pyridin-Derivat an.

Kita et al. beschreiben sowohl 1,2- als auch 1,4-Dihydropyridin als Produkte. Versuche an C- und Pb-Elektroden lieferten 0% Umsatz. An Pt-Kathoden wurden Ausbeuten von 36% erzielt. Nur bei Einsatz von Ammoniumchlorid und Temperaturen deutlich unter Raumtemperatur (5-10°C) konnten an Pt-Kathoden Ausbeuten von über 83% erzielt werden.

Eisner und Kuthan [Ulli Eisner und Josef Kuthan, Chem. Rev. (1972), 72, 1-42 (Titel: The Chemistry of Dihydropyridines)] beschreiben die chemische Reduktion von Pyridinen mittels NaBH4 oder durch katalytische Hydrierung. In beiden Fällen kommt es zu Ausbeute-Verlusten durch unselektive Reduktion von Substituenten (z.B. der Ester-Gruppe zum Alkohol) oder durch Reduktion der NitrilGruppe. Des Weiteren wurden auch hier 1,2-Dihyropyridine als Hauptprodukte beobachtet.

Der Stand der Technik offenbart somit kein allgemein gültiges Verfahren zur selektiven Reduktion von Pyridin-Derivaten, insbesondere nicht für Pyridin-Derivate, die keine Methylester- oder Ethylester-Substituenten aufweisen. Quecksilber-Elektroden sind bedingt durch ihren toxischen Charakter zudem für die Synthese pharmazeutischer Wirkstoffe ungeeignet. Zudem soll die Bildung von 1,2-Dihydropyridin-Derivaten, wie sie im Stand der Technik beschrieben wird, vermieden werden, da auch hierdurch Ausbeute-Verluste entstehen.

Die nachfolgende Beschreibung erläutert den zweiten Schritt des erfinderischen Verfahrens, die elektrochemische Reduktion des Pyridins (XVII) zum Amid (XIII):
Für die elektro-organische Synthese werden dem Fachmann bekannte Elektrolyse-Apparate, die sogenannten "Drei-Elektroden-Systeme" eingesetzt [Handbook of Electrochemistry; edited by C.G. Zoski; 2007 Elsevier B. V. & Fundamentals and Applications of Organic Electrochemistry: Synthesis, Materials, Devices, First Edition, T. Fuchigami, M. Atobe und S. Inagi; 2015 John Wiley & Sons, Ltd]. Hierbei werden dem Namen nach drei Elektroden eingesetzt, die Arbeits-, Gegen-, und Referenzelektrode. Es gibt eine Vielzahl an Referenz-Elektroden [Handbook of Electrochemistry; edited by C.G. Zoski; 2007 Elsevier B. V.], wobei für nicht-wässrige Elektrolyte, d.h. organische Lösungsmittel, die Silber/Silber-Kation (Ag/Ag+) Referenzelektrode bedingt durch ihre Stabilität und hohe Reproduzierbarkeit der Messungen bevorzugt eingesetzt wird. Hierbei wird ein Silber-Draht in eine 10 mM oder 0.1 M AgNO3 Lösung eingetaucht. Als Lösungsmittel können Acetonitril, Dimethylformamid oder Dimethylsulfoxid verwendet werden. Als Leitsalz wird standardmäßig Tetrabutylammoniumperchlorat (BuN4ClO4) verwendet. Alternativ können aber auch andere Leitsalze eingesetzt werden: Et4NBF4, Bu4NBF4, Bu4NPF6, Bu4NX (mit X = I, Br) oder Perchlorate (NaClO4, LiClO4, Et4NClO4).

Eine räumliche Trennung zwischen Arbeits- und Gegenelektrode bzw. beider sogenannten Halbzellen ist in den meisten Fällen vorteilhaft, um zu verhindern, dass sowohl Edukte als auch das zu erzeugende Zielprodukt an die Gegenelektrode gelangen und dort unerwünschte Nebenreaktionen auslösen, wodurch Ausbeuteverluste resultieren würden.

Für die räumliche Trennung von Arbeits- und Gegenelektrode werden Separatoren eingesetzt, die durch eine begrenzte Porosität und oder auch durch ihren chemischen Aufbau bzw. Funktionalität einen beliebigen Austausch zwischen beiden Halbzellen verhindern. Bekannte Separatoren sind gesinterte Glasfritten, PTFE-Filtermembranen, Kationenaustauscher-Membranen, Polyvinylidenfluorid- oder Polypropylen-Filtermembranen sowie hier nicht weiter aufgelistete Materialien, die beständig gegenüber organischen Lösungsmitteln sind und deren Porengrößen klein genug sind, um einen Übertritt von Edukt und Produkt in die andere Halbzelle einzuschränken bzw. ganz zu verhindern.

Für die elektrochemische Reduktion des Pyridins (XVII) wird die Arbeitselektrode als Kathode und die Gegenelektrode als Anode geschaltet.

Bekannte Elektrodenmaterialien sind Platin, Palladium, Gold, Graphit, Glaskohlenstoff, Bor-dotierter Diamant, Zink, Kupfer, Nickel, Zinn, Samarium, Stahl, Quecksilber, Blei oder Legierungen bestehend aus Kupfer, Zinn und Blei, sogenannte Bleibronzen. Ferner sind dem Fachmann weitere Metall und Metalloxid Elektroden bekannt, die auch dotiert oder in Legierungen eingesetzt werden: Ru/RuO2, Ti/TiO2, RuO2/TiO2, Ir/IrO2, Pt/Ti, Platin/Iridium.

Insbesondere in wässrigen Elektrolyten ist kathodisch die Bildung von gasförmigem Wasserstoff als konkurrierende Reaktion dem Fachmann bekannt. Daher sind Kathoden Materialien bevorzugt, die eine hohe Überspannung gegenüber der Wasserstoffbildung aufweisen. So nimmt die Überspannung für die H2-Bildung in folgender Rangfolge zu: Pd<Au<Pt<Ni<Cu<Sn<Pb<Zn<Hg.

In nicht-wässrigen Elektrolyten entscheidet die elektrochemische Stabilität des Lösungsmittels und Leitsalzes über das Auftreten und Ausmaß von Nebenreaktionen an den Elektroden.

Das sogenannte elektrochemische Fenster ist für ausgewählte Lösungsmittel/Leitsalzgemische tabelliert [Handbook of Electrochemistry; edited by C.G. Zoski; 2007 Elsevier B. V. & Fundamentals and Applications of Organic Electrochemistry: Synthesis, Materials, Devices, First Edition, T. Fuchigami, M. Atobe und S. Inagi; 2015 John Wiley & Sons, Ltd]. Beispielsweise sind die Kombinationen Acetonitril/0,1 M Bu4NPF6, Tetrahydrofuran/0,1 M Bu4NPF6, Acetonitril/0,1 M Et4NBF4, DMF/ 0,1 M Bu4NClO4 genannt, die auch bei negativeren Potentialen als -2,0 V (gegen gesättigte Kalomel-Elektrode) noch als elektrochemisch stabil gelten. Die Anwendung anderer Lösungsmittel ist dadurch nicht eingeschränkt oder grundsätzlich ausgeschlossen.

Typische und auch für elektro-organische Synthesen beschriebene Lösungsmittel sind Acetonitril, Ethanol, Tetrahydrofuran (THF), Aceton, N,N-Dimethylformamid (DMF), Methanol, Dichlormethan, Dimethylsulfoxid (DMSO), Hexamethylphosphoramide ([(CH3)2N]3PO; CAS:680-31-9). Dem Fachmann allgemein bekannte Lösungsmittel sind ferner NMP, N,N-Dimethylacetamid, Propanol, Isopropanol, Methylenchlorid, Essigester.

Leitsalze, die organischen Lösungsmittel zur Erhöhung der Leitfähigkeit zugefügt werden sind: Et4NBF4, Bu4NBF4, Bu4NPF6, Bu4NX (mit X = I, Br) oder Perchlorate (NaClO4, LiClO4, Et4NClO4, Bu4NClO4).

Die im Detail beschriebenen weitverbreiteten "Drei-Elektroden-Systemen", kommen in der Regel in dem Fachmann bekannten Becherglas-Zellen, H-Zellen oder anderen Behältnissen zur Anwendung. Mittels Magnetrührern können die Reaktionsansätze kontinuierlich gerührt werden. Es handelt sich mehrheitlich um Batch-Experimente, bei denen das Lösungsmittel/Leitsalzgemisch in beiden Halbzellen vorgelegt wird. Das Edukt wird nur in die Halbzelle eingefüllt, in der es auch elektrochemisch umgesetzt werden soll.

Durch kontinuierliche Zirkulation des Reaktionsgemisches mittels Kreislauf-Pumpen können solche Zellen auch im Durchfluss betrieben werden. Daneben sind in der Literatur sehr spezielle Geometrien für Durchflusszellen beschrieben [Handbook of Electrochemistry; edited by C.G. Zoski; 2007 Elsevier B. V.]. Besonders bevorzugt sind Durchflusszellen im Filterpressen Design mit Blick auf ein Scale-up der Synthese. Ausgehend von sehr kleinen Querschnittsflächen (10 cm²) lässt sich das Scale-up einerseits durch eine Vergrößerung der Querschnittsfläche auf bis zu 0.4 m² pro Modul (als Moduleinheit der "Electro Prod Cell", kommerziell verfügbar bei der Firma Electrocell) und andererseits durch ein Numbering-Up, also das Koppeln mehrerer identischer Module in einen Stack realisieren. Das Risiko eines solchen Scale-up Prozesses ist überschaubar, da die anderen geometrischen Dimensionen wie z.B. der Elektrodenabstand, das Elektrodenmaterial (für Anode und Kathode) als auch die Betriebsparameter (insbesondere die Stromdichte) nicht mehr verändert werden müssen. Für das erfindungsgemäße Verfahren wurden neben einfachen Becherglas-Zellen auch Durchflusszellen, wie die Micro-Flow Cell mit 10 cm² und die Multipurpose Cell mit 100 cm² aktiver Elektrodenquerschnittsfläche der Firma Electrocell erfolgreich eingesetzt.

Mittels regelbarer Durchflussgeschwindigkeit lässt sich die Verweilzeit in der Zelle kontrollieren. Typische Verweilzeiten liegen im Bereich 0.1-100s pro Durchlauf ("Single pass"). Für das erfindungsgemäße Verfahren sind bei Anwendung von Durchflusszellen bei der elektrochemischen Reduktion Verweilzeiten bevorzugt von 0.5-50s und besonders bevorzugt sind Verweilzeiten pro Durchlauf von 1-10s.

Die Auswahl der Stromdichte hängt sowohl von der Verweilzeit als auch der Kinetik der Zielreaktion und von unerwünschten Nebenreaktionen ab. Eine zu hohe Stromdichte bei gleichzeitig langer Verweilzeit und Gasbildung (z.B. H₂) würde zur Abschirmung der Elektrodenfläche durch die Ausbildung eines Gaspolsters in der Zelle führen. Für die elektrochemische Reduktion des Racemats M1 sind Stromdichten von 1-100 mA/cm² denkbar. Bevorzugt sind jedoch Stromdichten im Bereich 5-50 mA/cm² und besonders bevorzugt im Bereich 10-30 mA/cm² um bei ausreichender Raumzeit-Ausbeute maximale Selektivität zu erzielen, da überraschend gefunden wurde, dass zu hohe Stromdichten zu unerwünschten Nebenreaktionen führen und damit die Ausbeute sinkt.

Die Anwendung unterschiedlicher Lösungsmittel aus obiger Liste ist grundsätzlich möglich. Bevorzugte Lösungsmittel sind Methanol, DMF, DMA, NMP, Acetonitril und deren Gemische.

Es wurde überraschend gefunden, dass der Einsatz von Methanol als Lösungsmittel in Becherglaszellen Zielproduktausbeuten größer 97% ermöglicht. Überraschend wurde gefunden, dass eine Kombination aus aprotischem Lösungsmittel und protischem Lösungsmittel in der Durchflusszelle verbesserte Stromeffizienzen im Vergleich mit reinem Methanol zeigte. Umsätze und Ausbeuten über 94% konnten in der Durchflusszelle erzielt werden, wobei beide Halbzellen mittels Kationenaustauscher-Membran voneinander getrennt waren. Der erfolgreiche Transfer der elektrochemischen Reduktion von Pyridin der Formel (XVII) zum Amid der Formel (XIII) aus der Becherglaszelle in die Durchflusszelle ermöglicht die Skalierbarkeit des Verfahrens und damit die wirtschaftliche Nutzung.

Besonders bevorzugt sind Gemische mit einem gleich großen oder größeren Anteil an aprotischem Lösungsmittel und einem gleich großen bzw. kleineren Anteil an protischem Lösungsmittel.

Aprotische Lösungsmittel sind dem Fachmann allgemein bekannt. Bevorzugt sind insbesondere DMF, DMA und Acetonitril. Protische Lösungsmittel sind dem Fachmann ebenfalls allgemein bekannt. Bevorzugte protische Lösungsmittel sind Methanol, Ameisensäure, Ethanol und Essigsäure. Besonders bevorzugt ist die Kombination aus Methanol und DMF. Der Methanol Anteil sollte hierbei zwischen 0.1-50 gew.% liegen. Bevorzugt ist ein Methanol-Anteil von 0.5-25 gew.% und besonders bevorzugt von 1-10 gew.%. In diesem Gemisch befindet sich vorzugsweise neben Methanol auch Ethanol. Ebenfalls besonders bevorzugt ist die Kombination aus Ethanol und DMF. Der Ethanol Anteil sollte hierbei zwischen 0.1-50 gew.% liegen. Bevorzugt ist ein Ethanol-Anteil von 0.5-25 gew.% und besonders bevorzugt von 1-10 gew.%. Die Verwendung von Ethanol verhindert eine Umetherungsreaktion bei der der Ethylether zum Methylether umgeether werden kann.

Die im Nachgang aufgeführten Beispiele dokumentieren, dass ausgehend von racemischem Pyridin der Formel (XVII) das Zielprodukt, nämlich racemisches Amid der Formel (XIII) durch elektrochemische Reduktion erhalten wird und damit das erfindungsgemäße Verfahren mit dem nächsten Schritt (Trennung der beiden Enantiomere der Formeln (I) und ent-(I) z.B. in einer SMB Anlage) zur reinen Zielverbindung der Formel (I) führt. Überraschend wurde zudem gefunden, dass beim Einsatz der reinen Atropisomere Mlb(R) und Mla(S) die elektrochemische Reduktion in Becherglaszellen an Platin-Iridium-Netzelektroden nicht zu einem racemischen Produkt der Formel (XIII) führt. Bei der Reduktion der Verbindung der Formel Mlb(R) bildet sich bevorzugt das gewünschte Enantiomer (Zielprodukt) der Formel (I) im Verhältnis von ca. 78:22 [(I):ent-(I)]. Ausgehend von dem Atropsiomer der Formel Mla(S) wird das Fehlenantiomer der Formel ent-(I) im Überschuss erhalten: Verhältnis [(I):ent-(I)] = 22:78. Diese Beobachtung eröffnet die Möglichkeit durch selektive Oxidation der Verbindung der Formel ent-(I) zu der Verbindung der Formel Mlb(R) die Recycling-Ausbeute am Zielprodukt der Formel (I) pro Zyklus (Oxidation-Reduktion-Chirale-HPLC) weiter zu erhöhen.

Isolierung der Verbindung der Formel (XIII): Nachdem die elektrochemische Umsetzung erfolgt ist (Edukt der Formel (XVII) in der Regel <1%) wird die Reaktionslösung aufgearbeitet. Die Umsetzung verläuft in hohen Ausbeuten (>98%) und überraschend sauber mit nahezu keinen Verunreinigungen. Es hat sich als vorteilhaft erwiesen, dass man das Lösungsmittel zuerst weitgehend abdestilliert und anschließend das Produkt durch eine Wasser-Fällung (Zugabe von Wasser) ausfällt, abfiltriert und trocknet. Das so erhaltene Produkt kann aus Ethanol oder THF umkristallisiert werden und erneut einer Enantiomeren-Trennung mittels SMB unterzogen werden.

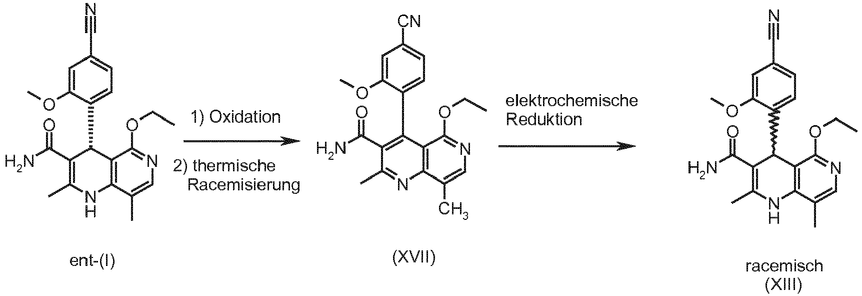

Zusammenfassend wird der Prozess wie folgt ablaufen: Zunächst wird das Fehlenantiomer der Formel ent-(I) oxidiert, dabei erhält man die Verbindung der Formel Mla(S) im Überschuss, im Rahmen der Aufarbeitung wird eine thermische Racemisierung (ggf. Säure katalysiert) durchgeführt. Anschließend wird die isolierte racemische Verbindung der Formel (XVII) einer elektrochemischen Reduktion unterzogen. Nach Aufarbeitung wird die racemische Verbindung der Formel (XIII) isoliert und umkristallisiert. Das so erhaltene Produkt der Formel (XIII) hat eine hohe Reinheit und wird in den SMB-Prozess eingespeist.

Der hier beschriebene Oxidations/Reduktions-Prozess kann mehrmals hintereinander durchgeführt werden und bietet somit die Möglichkeit in der Produktion in quasi kontinuierlicher Fahrweise das Fehlenantiomer der Formel ent-(I) in das richtige Produkt der Formel (I) zu überführen, was kostentechnisch große Vorteile bietet. Nach mehreren Prozess-Zyklen erreicht man fast eine vollständige Verwertung des Fehlenantiomers der Formel ent-(I).

Als ein besonders wichtiger Vorteil des neuen Verfahrens zur Rückgewinning der Verbindung der Formel (XIII) ist dessen hohe chemische Reinheit anzusehen. Da es sich um einen pharmazeutischen Wirkstoff handelt, werden alle Operationen unter GMP durchgeführt und erfordern hohe Reinheiten der Zwischenstufen.

Mit der neuen Synthese ist es gelungen in sehr effizienter Weise die Verbindung der Formel (I) herzustellen. Das Verfahren bietet gegenüber dem Stand der Technik erhebliche Vorteile, was die Skalierbarkeit und technische Durchführung betrifft. Die Gesamtausbeute ist verglichen mit publizierten Daten signifikant höher, außerdem werden exzellente Reinheiten beim Wirkstoff erzielt. Das neue Verfahren ermöglicht die reproduzierbare, ökonomische Herstellung der definierten Verbindung der Formel (I). Mit dem hier vorgestellten Verfahren wurden bereits erfolgreich 200 kg Material für klinische Prüfungen hergestellt.

Gegenstand der vorliegenden Erfindung ist ein Verfahren zur Herstellung von Verbindungen der Formel (B) 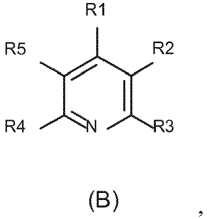 dadurch gekennzeichnet, dass Verbindungen der Formel (A) 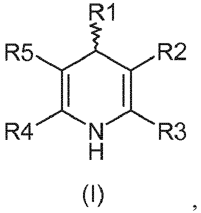 wobei
- R1-R5: unabhängig voneinander Wasserstoff, Fluor, Chlor Brom, Iod, Carboxyl, Carboxylester, Hydroxyl, Hydroxyether, Cyano, Nitro, substituiertes und unsubstituiertes Amid, (C₁-C₆)-Alkyl, Halo(C₁-C₆)-Alkyl, Formyl, substituiertes und unsubstituiertes Phenyl, substituiertes und unsubstituiertes Benzyl, substituiertes und unsubstituiertes Naphthyl, substituiertes und unsubstituiertes 5- oder 6-gliedriger Heterocyclus mit mindestens einem Heteroatom ausgewählt aus der Gruppe N, S, O, benzokondensierter 5- oder 6-gliedrieger Heterocyclus bedeuten,
elektrochemisch über eine indirekte elektrochemische Oxidation oxidiert werden.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-100°C und Normaldruck durchgeführt wird.

Ein weiterer Gegenstand der vorliegenden Erfindung ist ein Verfahren wie oben dargestellt, dadurch gekennzeichnet, dass die indirekte elektrochemische Oxidation bei einem Oxidationspotential von -0.1 V bis +0,6 V gegen Ag/Ag⁺-Referenzelektrode durchgeführt wird.

Ein weiterer Gegenstand der vorliegenden Erfindung ist ein Verfahren wie oben dargestellt, dadurch gekennzeichnet, dass die indirekte elektrochemische Oxidation unter Verwendung von DDQ als Mediator durchgeführt wird.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-110°C und Normaldruck bei einem Oxidationspotential von -0.1 V bis +0.6 V gegen Ag/Ag⁺-Referenzelektrode und unter Verwendung von DDQ als Mediator durchgeführt wird.

Ein weiterer Gegenstand der vorliegenden Erfindung ist ein Verfahren zur Herstellung von Verbindung der Formel (XVII) 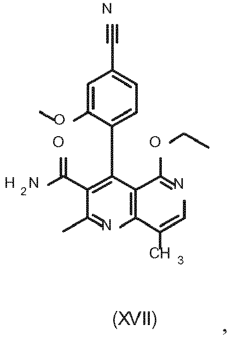 dadurch gekennzeichnet, dass Verbindungen der Formel ent-(I) 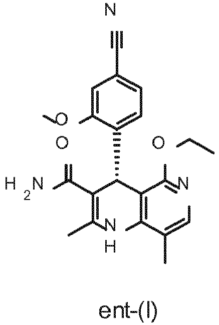 elektrochemisch über eine indirekte elektrochemische Oxidation oxidiert werden.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-100°C und Normaldruck durchgeführt wird.

Ein weiterer Gegenstand der vorliegenden Erfindung ist ein Verfahren wie oben dargestellt, dadurch gekennzeichnet, dass die indirekte elektrochemische Oxidation bei einem Oxidationspotential von -0.1 V bis +0.6 V gegen Ag/Ag⁺-Referenzelektrode durchgeführt wird.

Ein weiterer Gegenstand der vorliegenden Erfindung ist ein Verfahren wie oben dargestellt, dadurch gekennzeichnet, dass die indirekte elektrochemische Oxidation unter Verwendung von DDQ als Mediator durchgeführt wird.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-110°C und Normaldruck bei einem Oxidationspotential von -0.1 V bis +0.6 V gegen Ag/Ag⁺-Referenzelektrode und unter Verwendung von DDQ als Mediator durchgeführt wird.

Ein weiterer Gegenstand der vorliegenden Erfindung ist ein Verfahren zur Herstellung von Verbindung der Formel (XVII) 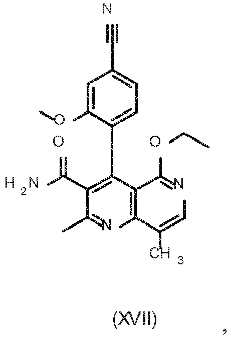 dadurch gekennzeichnet, dass Verbindungen der Formel (XIII) 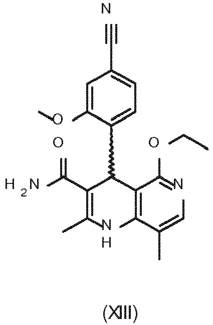 elektrochemisch über eine indirekte elektrochemische Oxidation oxidiert werden.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-100°C und Normaldruck durchgeführt wird.

Ein weiterer Ciegenstand der vorliegenden Erfindung ist ein Verfahren wie oben dargestellt, dadurch gekennzeichnet, dass die indirekte elektrochemische Oxidation bei einem Oxidationspotential von -0.1 V bis +0.6 V gegen Ag/Ag⁺-Referenzelektrode durchgeführt wird.

Ein weiterer Gegenstand der vorliegenden Erfindung ist ein Verfahren wie oben dargestellt, dadurch gekennzeichnet, dass die indirekte elektrochemische Oxidation unter Verwendung von DDQ als Mediator durchgeführt wird.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-110°C und Normaldruck bei einem Oxidationspotential von -0.1 V bis +0.6 V gegen Ag/Ag⁺-Referenzelektrode und unter Verwendung von DDQ als Mediator durchgeführt wird.

Ein weiterer Gegenstand der vorliegenden Erfindung ist ein Verfahren zur Herstellung von Verbindung der Formel (XVII) 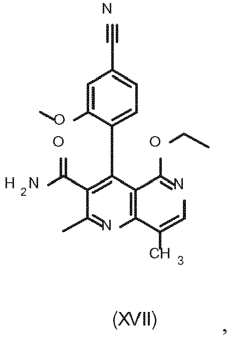 dadurch gekennzeichnet, dass Verbindungen der Formel (I) 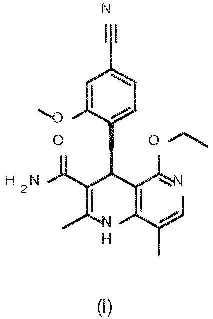 elektrochemisch über eine indirekte elektrochemische Oxidation oxidiert werden.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-100°C und Normaldruck durchgeführt wird.

Ein weiterer Gegenstand der vorliegenden Erfindung ist ein Verfahren wie oben dargestellt, dadurch gekennzeichnet, dass die indirekte elektrochemische Oxidation bei einem Oxidationspotential von -0.1 V bis +0,6 V gegen Ag/Ag⁺-Referenzelektrode durchgeführt wird.

Ein weiterer Gegenstand der vorliegenden Erfindung ist ein Verfahren wie oben dargestellt, dadurch gekennzeichnet, dass die indirekte elektrochemische Oxidation unter Verwendung von DDQ als Mediator durchgeführt wird.

Ein weiterer Gegenstand der vorliegenden Erfindung ist ein Verfahren wie oben dargestellt, dadurch gekennzeichnet, dass die indirekte elektrochemische Oxidation bei einer Temperatur von 1-110°C und Normaldruck bei einem Oxidationspotential von -0.1 V bis +0.6 V gegen Ag/Ag⁺-Referenzelektrode und unter Verwendung von DDQ als Mediator durchgeführt wird.

Gegenstand der vorliegenden Erfindung ist ein Verfahren zur Herstellung von Verbindungen der Formeln M1a(S) und M1b(R)

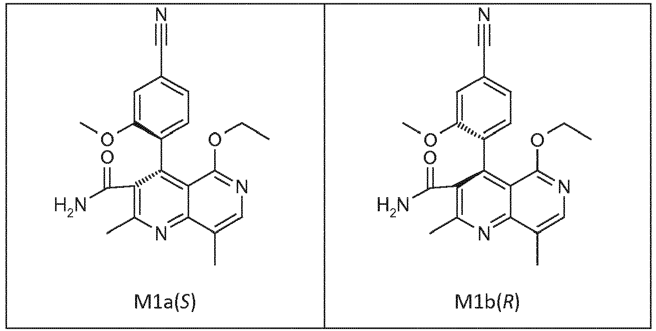

dadurch gekennzeichnet, dass die Verbindung der Formel ent-(I) 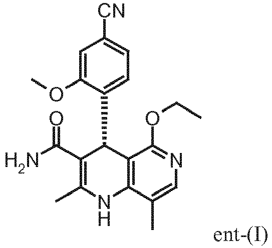 oxidiert wird.

Gegenstand der vorliegenden Erfindung ist ein Verfahren zur Herstellung von Verbindungen der Formeln Mla(S) und Mlb(R) wie oben beschrieben, dadurch gekennzeichnet, dass die Oxidation mit chemischen Oxidationsmitteln durchgeführt wird.

Ein weiterer Gegenstand der vorliegenden Erfindung ist ein Verfahren zur Herstellung der racemischen Verbindung der Formel (XVII) 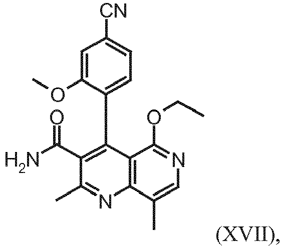 dadurch gekennzeichnet, dass man ein Gemisch aus den Verbindungen der Formeln Mla(S) und Mlb(R)

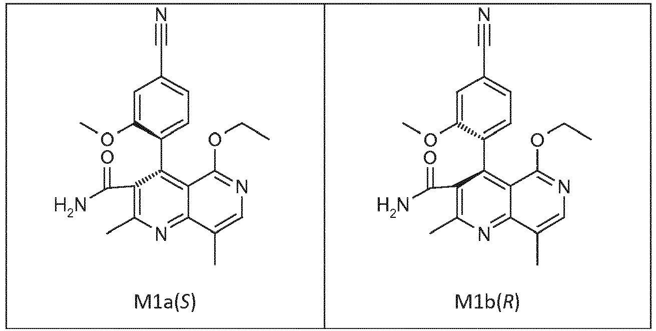

thermisch racemisiert wird.

Gegenstand der vorliegenden Erfindung ist ein Verfahren zur Herstellung der Verbindung der Formel (XVII) wie oben beschrieben, dadurch gekennzeichnet, dass ein Gemisch aus den Verbindungen der Formeln M1a(S) und Mlb(R) bei einer Temperatur von 70 bis 110°C mit oder ohne Zugabe einer Säure racemisiert wird.

Ein weiterer Gegenstand der vorliegenden Erfindung ist ein Verfahren zu Herstellung von Verbindungen der Formeln (I) und ent-(I)

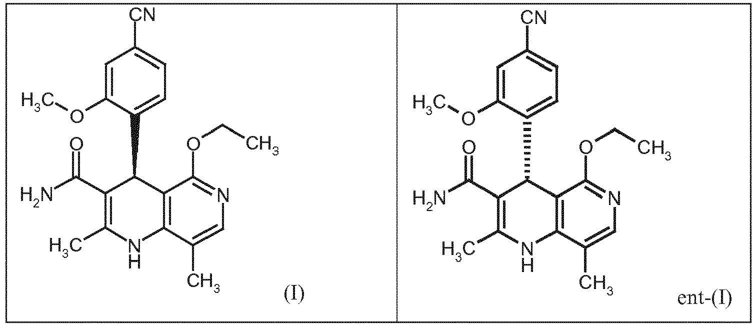

dadurch gekennzeichnet, dass Verbindungen der Formeln (XVII) oder Mla(S) oder Mlb(R) oder ein Gemisch aus Mla(S) und Mlb(R)

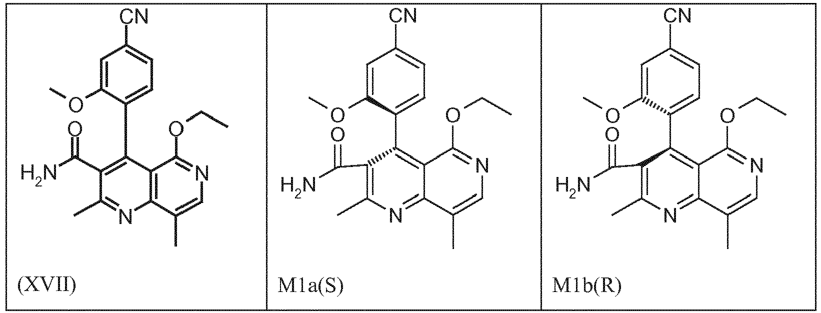

elektrochemisch reduziert werden.

Gegenstand der vorliegenden Erfindung ist ein Verfahren zu Herstellung der Verbindungen der Formeln (I) und ent-(I) wie oben beschrieben, dadurch gekennzeichnet, dass die elektrochemische Reduktion in einer Becherglaszelle oder Durchflusszelle in Anwesenheit von Methanol durchgeführt wird.

Gegenstand der vorliegenden Erfindung ist ein Verfahren zu Herstellung der Verbindungen der Formeln (I) und ent-(I) wie oben beschrieben, dadurch gekennzeichnet, dass die elektrochemische Reduktion in einer Becherglaszelle oder Durchflusszelle in Anwesenheit von Ethanol durchgeführt wird.

Ein weiterer Gegenstand der vorliegenden Erfindung ist Verfahren zur Herstellung von Verbindungen der Formeln (I) und ent-(I) wie oben beschreiben

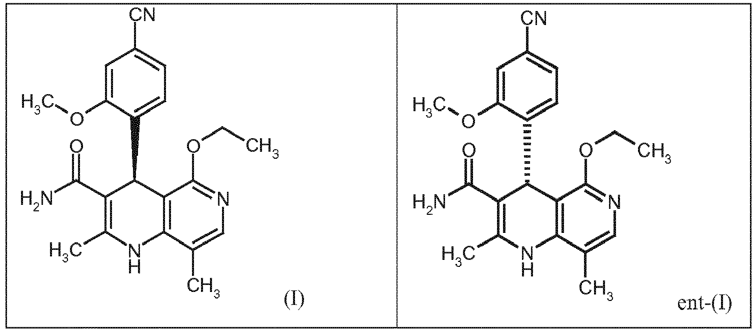

dadurch gekennzeichnet, dass Verbindungen der Formeln (XVII) oder Mla(S) oder Mlb(R) oder ein Gemisch aus Mla(S) und Mlb(R)

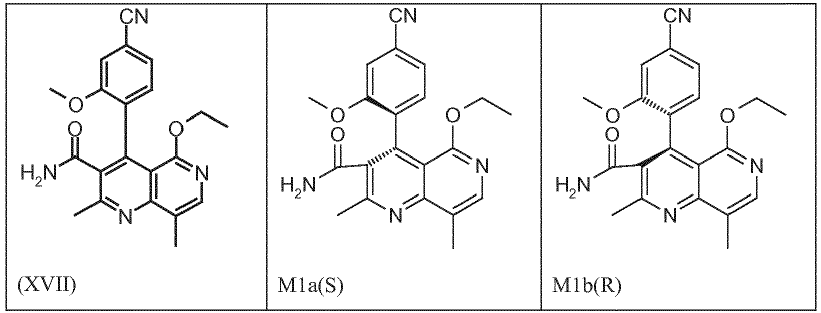

elektrochemisch reduziert werden,
und dadurch gekennzeichnet, dass die Verbindungen der Formeln (XVII), M1a (S) und Mlb (R) durch thermische Isomerisierung von Verbindungen der Formeln M1a (S) und Mlb (R)

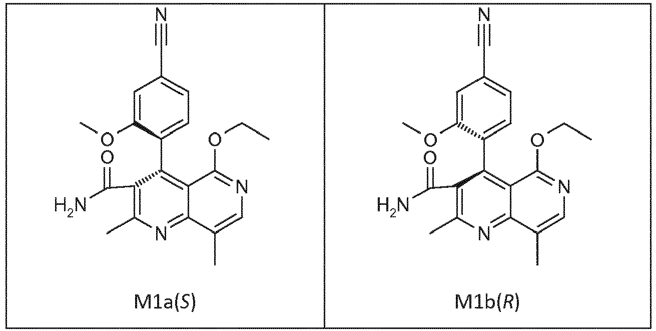

erhalten werden, und dadurch gekennzeichnet, dass man die Verbindung der Formel ent-(I) 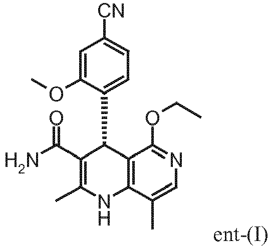 oxidiert.

Gegenstand der vorliegenden Erfindung ist ein Verfahren zur Herstellung von Verbindungen der Formeln (I) und ent-(I) wie oben beschrieben,

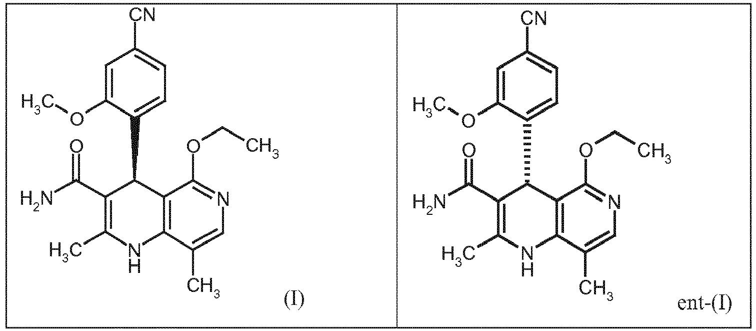

dadurch gekennzeichnet, dass Verbindungen der Formeln (XVII) oder Mla(S) oder Mlb(R) oder ein Gemisch aus Mla(S) und Mlb(R)

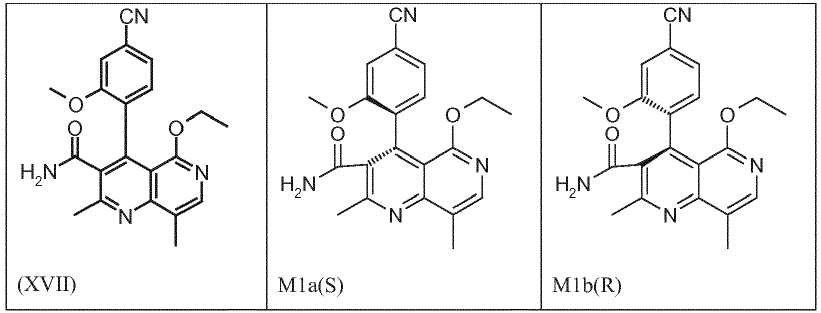

elektrochemisch in einer Becherglaszelle oder Durchflusszelle in Anwesenheit von Methanol reduziert werden,
und dadurch gekennzeichnet, dass die Verbindungen der Formeln (XVII), M1a(S) und M1b(R) durch thermische Isomerisierung von Verbindungen der Formeln M1a(S) und M1b(R)

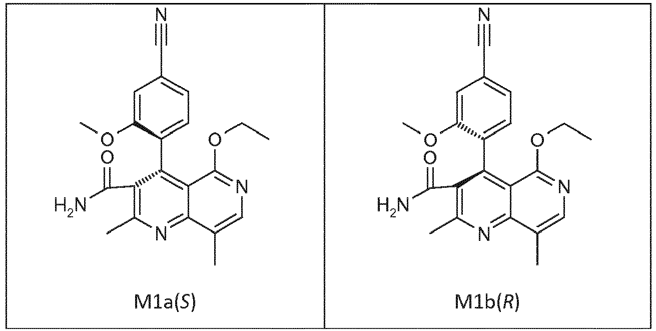

erhalten werden, und dadurch gekennzeichnet, dass man die Verbindung der Formel ent-(I) 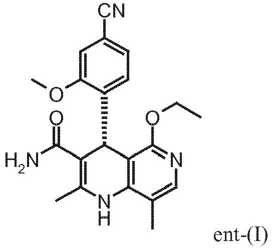 mit chemischen Oxidationsmitteln oxidiert.

Gegenstand der vorliegenden Erfindung ist ein Verfahren zur Herstellung von Verbindungen der Formeln (I) und ent-(I) wie oben beschrieben,

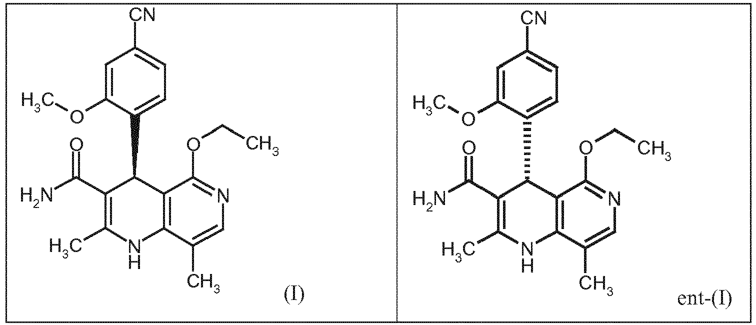

dadurch gekennzeichnet, dass Verbindungen der Formeln (XVII) oder Mla(S) oder Mlb(R) oder ein Gemisch aus Mla(S) und Mlb(R)

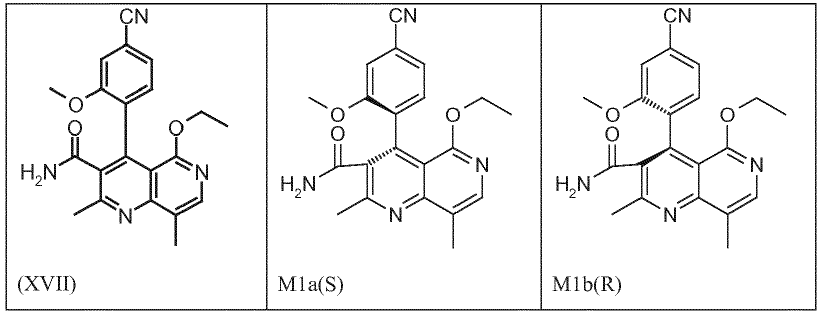

elektrochemisch in einer Becherglaszelle oder Durchflusszelle in Anwesenheit von Ethanol reduziert werden,
und dadurch gekennzeichnet, dass die Verbindungen der Formeln (XVII), Mla(S) und Mlb(R) durch thermische Isomerisierung von Verbindungen der Formeln Mla(S) und Mlb(R)

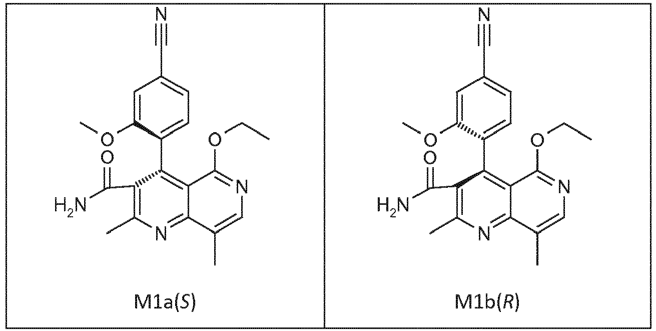

erhalten werden, und dadurch gekennzeichnet, dass man die Verbindung der Formel ent-(I) 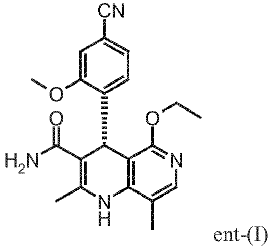 mit chemischen Oxidationsmitteln oxidiert.

### Experimenteller Teil

### Abkürzungen und Akronyme:

MS : Masse aus Massenspektrometrie
HPLC: Hochleistungsflüssigkeitschromatographie
DMF : Dimethylformamid
Red-Al Lösung in Toluol: Natrium-bis-(2-methoxy-ethoxy)-aluminium-dihydrid in Toluol
THF : Tetrahydrofuran
Aqu. HCl: wässrige Salzsäure
DMAP: 4-(Dimethylamino)-pyridin

### Beispiele

### Beispiel 1

### Methyl-4-brom-2-methoxybenzoat (XV)

3,06 kg (22,12 mol) Kaliumcarbonat wurden in 3,6 1 Aceton vorgelegt und zum Rückfluss erhitzt. Zu dieser Suspension dosierte man 1,2 kg 4-Brom-2-hydroxybenzoesäure (5,53 mol), suspendiert in 7,8 1 Aceton zu und spülte mit 0,6 1 Aceton nach. Man erhitzte eine Stunde unter Rückfluss (starke Gasentwicklung!). Anschließend wurde unter Sieden 2,65 kg (21,01 mol) Dimethylsufat über 4 Stunden zudosiert. Anschließend wurde 2,5 Stunden unter Rückfluss nachgerührt. Man destillierte das Lösungsmittel weitgehend ab (bis zur Rührbarkeit) und gab 12 l Toluol zu, anschließend wurde bei 110°C das restliche Aceton abdestilliert. Es wurden ca. 3 l Destillat abdestilliert, diese wurden durch Zugabe von weiteren 3 l Toluol zum Ansatz ergänzt. Man ließ auf 20°C abkühlen und gab 10,8 l Wasser dazu und rührte kräftig durch. Die organische Phase wurde abgetrennt und die Wasserphase nochmals mit 6,1 l Toluol nachextrahiert. Die vereinigten organischen Phasen wurden mit 3 l gesättigter Kochsalzlösung gewaschen und die Toluolphase bis auf ca. 4 l eingeengt. Eine Gehaltsbestimmung durch Eindampfen einer Teilmenge ergab umgerechnet eine Ausbeute 1,306 kg (96,4 % der Theorie). Die Lösung wurde direkt in die Folgestufe eingesetzt.
HPLC-Methode A: RT ca. 11,9 min.
MS (EIpos): m/z = 245 [M+H]⁺
¹H NMR (400 MHz, CD₂Cl₂): δ = 3.84 (s, 3H), 3.90 (s, 3H), 7.12-7.20 (m, 2H), 7.62 (d, 1H).

### Beispiel 2

### 4-Brom-2-methoxybenzaldehyd (XVI)

Man legte 1,936 kg (6,22 mol) 65%ige Red-Al Lösung in Toluol mit 1,25 l Toluol bei -5 °C vor. Zu dieser Lösung dosierte man 0,66 kg (6,59 mol) 1-Methylpiperazin zu und spülte mit 150 ml Toluol nach, die Temperatur hielt man dabei zwischen -7 bis -5°C. Man ließ 30 Minuten bei 0°C nachrühren. Diese Lösung dosierte man anschließend zu einer Lösung von 1,261 kg (5,147 mol) Methyl-4-brom-2-methoxybenzoat (XV), gelöst in 4 l Toluol zu, die Temperatur hielt man bei - 8 bis 0°C. Man spülte zweimal mit 0,7 l Toluol nach und rührte 1,5 Stunden bei 0°C nach. Zur Aufarbeitung dosierte man auf eine 0°C kalte wässrige Schwefelsäure (12,5 l Wasser + 1,4 kg konz. Schwefelsäure). Die Temperatur sollte dabei maximal auf 10°C steigen (langsame Dosierung). Der pH-Wert wurde ggf. durch Zugabe von weiterer Schwefelsäure auf pH l gestellt. Die organische Phase wurde abgetrennt und die wässrige Phase mit 7,6 l Toluol nachextrahiert. Die vereinigten organischen Phasen wurden mit 5,1 l Wasser gewaschen und anschließend weitgehend eingeengt und der Rückstand mit 10 l DMF aufgenommen. Man engte erneut auf ca. 5 l Volumen ein. Eine Gehaltsbestimmung durch Eindampfen einer Teilmenge ergab umgerechnet eine Ausbeute 1,041 kg (94,1 % der Theorie). Die Lösung wurde direkt in die Folgestufe eingesetzt.
HPLC-Methode A: RT ca. 12,1 min.
MS (EIpos): m/z = 162 [M+H]⁺
¹H-NMR (CDCl₃, 400MHz): δ = 3.93 (3H, s), 7.17 (2H, m), 7.68 (1H, d), 10.40 (1H, s)

### Beispiel 3

### 4-Formyl-3-methoxybenzonitril (VI)

719 g (3,34 mol) 4-Brom-2-methoxybenzaldehyd (XVI) als Lösung in 4,5 l DMF wurden mit 313 g (0,74 mol) Kaliumhexacyanoferrat (K₄[Fe(CN)₆]) und 354 g (3,34 mol) Natriumcarbonat und weiteren 1,2 l DMF vorgelegt und 3,8 g (0,017 mol) Palladiumacetat wurde zugegeben. Man rührte 3 Stunden bei 120°C. Man ließ auf 20°C abkühlen und gab 5,7 l Wasser zum Ansatz. Man extrahierte mit 17 l Essigester und wusch die wässrige Phase nochmals mit 17 l Essigester nach. Die organischen Phasen wurden vereinigt und weitgehend eingeengt mit 5 l Isopropanol aufgenommen und auf ca. 2 l eingeengt. Man erwärmte zum Sieden und tropfte 2 l Wasser dazu. Man ließ auf 50 °C abkühlen und gab erneut 2 l Wasser hinzu. Man kühlte auf 3°C ab und rührte eine Stunde bei dieser Temperatur. Das Produkt wurde abfiltriert und mit Wasser (2 mal 1,2 l) nachgewaschen. Man trocknete bei 40°C im Vakuum.
Ausbeute: 469 g (87 % d. Theorie) eines beigefarbenen Feststoffes.
HPLC-Methode A: RT ca. 8,3 min.
MS (EIpos): m/z = 162 [M+H]+
1H-NMR (300 MHz, DMSO-d6): δ = 3.98 (s, 3H), 7.53 (d, 1H), 7.80 (s, 1H), 7.81 (d, 1H), 10.37 (s, 1H).

### Beispiel 4

### 2-Cyanoethyl 4-(4-cyano-2-methoxyphenyl)-2,8-dimethyl-5-oxo-1,4,5,6-tetrahydro-1,6-naphthyridin-3-carboxylat (X)

### Variante A

1,035 kg (6,422 mol) 4-Formyl-3-methoxybenzonitril (VI), 1,246 kg (8,028 mol) 2-Cyanethyl-3-oxobutanoat, 54,6 g (0,642 mol) Piperidin und 38,5 g (0,642 mol) Eisessig wurden in 10 l Dichlormethan 6,5 Stunden unter Rückfluss am Wasserabscheider erhitzt. Man ließ auf Raumtemperatur abkühlen und wusch die organische Phase 2 mal mit je 5 l Wasser. Anschließend wurde das Dichlormethanphase unter Normaldruck eingeengt und der noch rührbare Rückstand mit 15,47 kg 2-Butanol aufgenommen und 0,717 kg (5,78 mol) 4-Amino-5-methylpyridon zugesetzt. Das restliche Dichlormethan wurde abdestilliert, bis eine Innentemperatur von 98°C erreicht wurde. Anschließend wurde 20 Stunden unter Rückfluss erhitzt. Man kühlte auf 0°C ab, ließ 4 Stunden bei dieser Temperatur nachrühren und filtrierte das Produkt ab. Es wurde bei 40°C im Vakuum unter Schleppgas getrocknet.
Ausbeute: 2,049 kg (87,6 % der Theorie bezogen auf 4-Amino-5-methylpyridon, da diese Komponente im Unterschuss eingesetzt wird) eines leicht gelbfarbigen Feststoffes.
HPLC-Methode A: RT ca. 9,7 min.
MS (EIpos): m/z = 405 [M+H]⁺
¹H-NMR (300 MHz, DMSO-d₆): δ = 2.03 (s, 3H), 2.35 (s, 3H), 2.80 (m, 2H), 3.74 (s, 3H), 4.04 (m, 1H), 4.11 (m, 1H), 5.20 (s, 1H), 6.95 (s, 1H), 7.23 (dd, 1H), 7.28-7.33 (m, 2H), 8.18 (s, 1H), 10.76 (s, 1H).

### Variante B

1,344 kg (8,34 mol) 4-Formyl-3-methoxybenzonitril (VI), 71 g (0,834 mol) Piperidin und 50,1 g (0,834 mol) Eisessig wurden in 6 l Isopropanol vorgelegt und bei 30°C innerhalb von 3 Stunden eine Lösung aus 1,747 kg (11,26 mol) 2-Cyanethyl-3-oxobutanoat in 670 ml Isopropanol zudosiert. Man rührte eine Stunde bei 30°C nach. Es wurde auf 0-3°C abgekühlt und 0,5 Stunden nachgerührt. Man filtrierte das Produkt ab und wusch 2-mal mit je 450 ml kaltem Isopropanol nach. Zur Ausbeute-Bestimmung wurde im Vakuum bei 50°C getrocknet (2,413 kg, 97% d. Th.); es wurde aber in der Regel, aufgrund der hohen Ausbeute direkt mit dem Isopropanol-feuchtem Produkt weitergearbeitet. Dazu wurde das Produkt mit 29 l Isopropanol aufgenommen und 1,277 kg (7,92 mol) 4-Amino-5-methylpyridon zugegeben und anschließend 24 h bei 100°C Innentemperatur unter ca. 1,4 Bar Überdruck im geschlossenen Kessel erhitzt. Man kühlte über eine Rampe innerhalb von 5 h auf 0°C ab und rührte 3 Stunden bei 0°C nach. Man filtrierte ab und wusch mit 2,1 l kaltem Isopropanol nach. Man trocknete im Vakuum bei 60°C.
Ausbeute: 2,819 kg (88 % der Theorie bezogen auf 4-Amino-5-methylpyridon, da diese Komponente im Unterschuss eingesetzt wird) eines leicht gelbfarbigen Feststoffes.
HPLC-Methode A: RT ca. 9,7 min.
MS (EIpos): m/z = 405 [M+H]⁺
¹H-NMR (300 MHz, DMSO-d₆): δ = 2.03 (s, 3H), 2.35 (s, 3H), 2.80 (m, 2H), 3.74 (s, 3H), 4.04 (m, 1H), 4.11 (m, 1H), 5.20 (s, 1H), 6.95 (s, 1H), 7.23 (dd, 1H), 7.28-7.33 (m, 2H), 8.18 (s, 1H), 10.76 (s, 1H).

### Beispiel 5

### 2-Cyanoethyl-4-(4-cyano-2-methoxyphenyl)-5-ethoxy-2,8-dimethyl-1,4-dihydro-1,6-naphthyridin-3-carboxylat (XI)

2,142 kg (5,3 mol) 2-Cyanoethyl 4-(4-cyano-2-methoxyphenyl)-2,8-dimethyl-5-oxo-1,4,5,6-tetrahydro-1,6-naphthyridin-3-carboxylat (X) und 4,70 kg (29 mol) Orthoessigsäuretriethylester wurden in 12,15 l Dimethylacetamid gelöst und 157,5 g konzentrierter Schwefelsäure zugegeben. Man erwärmte 1,5 Stunden bei 115°C und kühlte anschließend auf 50°C ab. Bei 50°C wurden über 30 Minuten 12,15 l Wasser zu getropft. Nach beendeter Zugabe wurde mit 10 g der Titelverbindung (XI) angeimpft und weitere 12,15 l Wasser über 30 Minuten bei 50°C zu getropft. Man kühlte auf 0°C (Rampe, 2 Stunden) ab und rührte 2 Stunden bei 0°C nach. Das Produkt wurde abfiltriert, 2mal mit je 7,7 l Wasser gewaschen und im Vakuum bei 50°C getrocknet.
Ausbeute: 2114,2 g (92,2 % der Theorie) eines leicht gelbfarbigen Feststoffes.
HPLC-Methode B: RT ca. 10,2 min.
MS (EIpos): m/z = 433 [M+H]⁺
¹H-NMR (300 MHz, DMSO-d₆): δ = 1.11 (t, 3H), 2.16 (s, 3H), 2.42 (s, 3H), 2.78 (m, 2H), 3.77 (s, 3H), 4.01-4.13 (m, 4H), 5.37 (s, 1H), 7.25 (d, 1H), 7.28-7.33 (m, 2H), 7.60 (s, 1H), 8.35 (s, 1H).
Alternativ kann die Reaktion in NMP (1-Methyl-2-pyrrolidon) durchgeführt werden

### 2-Cyanoethyl-4-(4-cyano-2-methoxyphenyl)-5-ethoxy-2,8-dimethyl-1,4-dihydro-1,6-naphthyridin-3-carboxylat (XI)

2,142 kg (5,3 mol) 2-Cyanoethyl 4-(4-cyano-2-methoxyphenyl)-2,8-dimethyl-5-oxo-1,4,5,6-tetrahydro-1,6-naphthyridin-3-carboxylat (X) und 2,35 kg (14,5 mol) Orthoessigsäuretriethylester wurden in 3,21 kg NMP (1-Methyl-2-pyrrolidon) gelöst und 157,5 g konzentrierter Schwefelsäure zugegeben. Man erwärmte 1,5 Stunden bei 115°C und kühlte anschließend auf 50°C ab. Bei 50°C wurden über 30 Minuten 2,2 l Wasser zu getropft. Nach beendeter Zugabe wurde mit 10 g der Titelverbindung (XI) angeimpft und weitere 4,4 l Wasser über 30 Minuten bei 50°C zu getropft. Man kühlte auf 0°C (Rampe, 2 Stunden) ab und rührte 2 Stunden bei 0°C nach. Das Produkt wurde abfiltiert, 2mal mit je 4 l Wasser gewaschen und im Vakuum bei 50°C getrocknet.
Ausbeute: 2180,7 g (95,1 % der Theorie) eines leicht gelbfarbigen Feststoffes.
HPLC-Methode B: RT ca. 10,2 min.

### Beispiel 6

### 4-(4-Cyano-2-methoxyphenyl)-5-ethoxy-2,8-dimethyl-1,4-dihydro-1,6-naphthyridin-3-carbonsäure (XII)

2,00 kg (4,624 mol) 2-Cyanoethyl 4-(4-cyano-2-methoxyphenyl)-5-ethoxy-2,8-dimethyl-1,4-dihydro-1,6-naphthyridin-3-carboxylat (XI) wurden in einer Mischung aus 12 l THF und 6 l Wasser gelöst und auf 0°C abgekühlt. Zu dieser Lösung tropfte man innerhalb 15 Minuten bei 0°C eine Natronlauge-Lösung (hergestellt aus 0,82 kg 45%iger aqu. NaOH (9,248 mol) und 4,23 l Wasser) und rührte 1,5 Stunden bei 0°C nach. Man extrahierte 2-mal mit je 4,8 l Methyl-tert.butylether und einmal mit 4,8 l Essigester. Die wässrige Lösung wurde bei 0°C mit verdünnter Salzsäure (hergestellt aus 0,371 kg 37%ig HCl und 1,51 l Wasser) auf pH 7 eingestellt. Man ließ auf 20°C erwärmen und gab eine wässrige Lösung aus 2,05 kg Ammoniumchlorid in 5,54 l Wasser zu. Es wurde l Stunde bei 20°C nachgerührt, das Produkt filtriert und je 2-mal mit je 1,5 l Wasser und einmal mit 4 l Acetonitril gewaschen. Man trocknete bei 40°C im Vakuum unter Schleppgas.
Ausbeute: 1736,9 g (99 % d. Th.) eines fast farblosen Pulvers (ganz leichter Gelbstich).
HPLC-Methode C: RT: ca. 6,8 min.
MS (EIpos): m/z = 380 [M+H]⁺
¹H-NMR (300 MHz, DMSO-d₆): δ = 1.14 (t, 3H), 2.14 (s, 3H), 2.37 (s, 3H), 3.73 (s, 3H), 4.04 (m, 2H), 5.33 (s, 1H), 7.26 (m, 2H), 7.32 (s, 1H), 7.57 (s, 1H), 8.16 (s, 1H), 11.43 (br. s, 1H).

Alternative Aufarbeitung mit Toluol zur Extraktion:

### 4-(4-Cyano-2-methoxyphenyl)-5-ethoxy-2,8-dimethyl-1,4-dihydro-1,6-naphthyridin-3-carbonsäure (XII)

2,00 kg (4,624 mol) 2-Cyanoethyl 4-(4-cyano-2-methoxyphenyl)-5-ethoxy-2,8-dimethyl-1,4-dihydro-1,6-naphthyridin-3-carboxylat (XI) wurden in einer Mischung aus 12 l THF und 6 l Wasser gelöst und auf 0°C abgekühlt. Zu dieser Lösung tropfte man innerhalb von 15 Minuten bei 0°C eine Natronlauge-Lösung (hergestellt aus 0,82 kg 45%iger aqu. NaOH (9,248 mol) und 4,23 l Wasser und rührte 1,5 Stunden bei 0°C nach. Man gab 5 L Toluol und 381,3 g Natriumacetat zu und rührte kräftig durch. Man ließ die Phasen absetzen und trennte die organische Phase ab. Die wässrige Phase wurde mit 10%iger Salzsäure auf pH 6,9 gestellt (bei ca. pH 9,5 impfte man mit 10 g der Titelverbindung an). Nach beendeter Ausfällung des Produktes wurde eine Stunde bei 0°C nachgerührt und anschließend wurde abfiltriert und zwei Mal mit je 4 l Wasser und zwei Mal mit je 153 ml Toluol gewaschen. Man trocknete bei 40°C im Vakuum unter Schleppgas (Stickstoff, 200 mbar). Ausbeute: 1719,5 g (98 % d. Th.) eines fast farblosen Pulvers (ganz leichter Gelbstich).
HPLC-Methode C: RT: ca. 6,8 min.

### Beispiel 7

### 4-(4-Cyano-2-methoxyphenyl)-5-ethoxy-2,8-dimethyl-1,4-dihydro-1,6-naphthyridin-3-carboxamid (XIII)

1,60 kg (4,22 mol) 4-(4-Cyano-2-methoxyphenyl)-5-ethoxy-2,8-dimethyl-1,4-dihydro-1,6-naphthyridin-3-carbon¬säure (XII) und 958 g (5,91 mol) 1,1-Carbodiimidazol wurden in 8 l THF vorgelegt und bei 20°C 51 g (0,417 mol) DMAP zugegeben. Man rührte eine Stunde bei 20°C (Gasentwicklung!) und erwärmte anschließend 2,5 Stunden auf 50°C. Zu dieser Lösung gab man 2,973 kg (18,42 mol) Hexamethyldisilazan und kochte 22 Stunden unter Rückfluss. Man gab weitere 1,8 l THF zu und kühlte auf 5°C ab. Es wurde eine Mischung aus 1,17 l THF und 835 g Wasser über 3 Stunden zu dosiert, sodass die Temperatur zwischen 5 und 20°C blieb. Anschließend kochte man eine Stunde unter Rückfluss, kühlte dann über eine Rampe (3 Stunden) auf 0°C ab und rührte eine Stunde bei dieser Temperatur nach. Das Produkt wurde abfiltriert und 2-mal mit je 2,4 l THF und zweimal mit je 3,2 l Wasser nachgewaschen. Man trocknete im Vakuum bei 70°C unter Schleppgas.
Ausbeute: 1,501 kg (94 % d. Theorie) eines fast farblosen Pulvers (ganz leichter Gelbstich).
HPLC-Methode B: RT ca. 6,7 min.
MS (EIpos): m/z = 379 [M+H]⁺
¹H-NMR (300 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).

### Beispiel 8

### (4S)-4-(4-Cyano-2-methoxyphenyl)-5-ethoxy-2,8-dimethyl-1,4-dihydro-1,6-naphthyridin-3-carboxamid (I) als Lösung in Acetonitril/Methanol 40:60

### Enantiomeren-Trennung auf einer SMB-Anlage

Als Feed-Lösung diente eine Lösung entsprechend einer Konzentration bestehend aus 50 g Racemat 4-(4-Cyano-2-methoxyphenyl)-5-ethoxy-2,8-dimethyl-1,4-dihydro-1,6-naphthyridin-3-carbox-amid (XIII), gelöst in 1 Liter einer Mischung aus Methanol/Acetonitril 60:40.

Es wurde über eine SMB-Anlage an einer stationären Phase: Chiralpak AS-V, 20 µm chromatographiert. Der Druck betrug 30 bar, als Eluent wurde eine Mischung aus Methanol/Acetonitril 60:40 verwendet.

9,00 kg 4-(4-Cyano-2-methoxyphenyl)-5-ethoxy-2,8-dimethyl-1,4-dihydro-1,6-naphthyridin-3-carbox-amid (XII) wurden in 180 l einer Mischung bestehend aus Methanol/Acetonitril 60:40 gelöst und mittels SMB chromatographiert. Nach aufkonzentrieren der produkthaltigen Fraktionen erhielt man 69,68 liter einer 6,2 %igen Lösung (entspricht 4,32 kg (4S)- 4-(4-Cyano-2-methoxyphenyl)-5-ethoxy-2,8-dimethyl-1,4-dihydro-1,6-naphthyridin-3-carbox-amid (I) als Lösung in Acetonitril/Methanol 40:60).
Ausbeute: 4,32 kg (S)-Enantiomer (48 % d. Theorie), gelöst in 69,68 liter Acetonitril/Methanol 40:60 als farblose Fraktion.
Enantiomerenreinheit: > 98,5% e.e. (HPLC, Methode D)
Eine Probe wird im Vakuum eingeengt und ergibt: MS (EIpos): m/z = 379 [M+H]⁺
¹H-NMR (300 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).
In analoger Weise wurde das (R)-Enantiomer ent-(I) isoliert
Ausbeute: 4,41 kg (R)-Enantiomer (48 % d. Theorie), gelöst in 71,00 liter Acetonitril/Methanol 40:60 als farblose Fraktion.
Enantiomerenreinheit: > 98,5% e.e. (HPLC, Methode D)
Eine Probe wurde im Vakuum eingeengt und ergibt: MS (EIpos): m/z = 379 [M+H]+
1H-NMR (300 MHz, DMSO-d6): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).

### Beispiel 9

### (4S)-4-(4-Cyano-2-methoxyphenyl)-5-ethoxy-2,8-dimethyl-1,4-dihydro-1,6-naphthyridin-3-carboxamid (I)

### Kristallisation und Polymorphie-Einstellung

64,52 liter einer 6,2 %igen Lösung aus Beispiel 8 in einer Mischung Acetonitiril/Methanol 40:60 (entspricht 4,00 kg Verbindung I) wurden über eine Filterkerze (1,2 um) filtriert und anschließend bei 250 mbar soweit eingeengt, sodaß die Lösung noch rührbar ist. Man fügte 48 l Ethanol, vergällt mit Toluol zu und destillierte erneut bei 250 mbar bis zur Rührbarkeitsgrenze ab (Umdestillation auf Ethanol). Man gab weitere 48 1 Ethanol, vergällt mit Toluol zu und destillierte anschließend bei Normaldruck bis auf ein Gesamtvolumen von ca. 14 1 ab (Manteltemperatur 98°C). Man kühlte über eine Rampe (4 Stunden) auf 0°C ab, rührte 2 Stunden bei 0°C nach und filtrierte das Produkt ab. Es wurde zweimal mit je 4 1 kaltem Ethanol nachgewaschen und anschließend im Vakuum bei 50°C getrocknet.
Ausbeute: 3,64 kg (91 % d. Theorie) eines farblosen kristallinen Pulvers
Enantiomerenreinheit: >> 99 % e.e. (HPLC-Methode D); Retentionszeiten/RRT: (4S)- 4-(4-Cyano-2-methoxyphenyl)-5-ethoxy-2,8-dimethyl-1,4-dihydro-1,6-naphthyridin-3-carbox-amid (1) ca. 11 min. RRT: 1,00 ; (4R)- 4-(4-Cyano-2-methoxyphenyl)-5-ethoxy-2,8-dimethyl-1,4-dihydro-1,6-naphthyridin-3-carbox-amid (I) ca. 9 min. RRT: 0,82
Reinheit: > 99,8% (HPLC-Methode B), RT: ca. 6,7 min.
Gehalt: 99,9 % (gegen externen Standard)
spezifischer Drehwert (Chloroform, 589 nm, 19.7°C, c = 0.38600 g / 100 ml): - 148.8°.
MS (EIpos): m/z = 379 [M+H]⁺
¹H-NMR (300 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).
Schmelzpunkt: 252 °C (Verbindung der Formel ent- (I) in kristalliner Form der Modifikation I)

In analoger Weise wird das (R)-Enantiomer ent-(I) isoliert. Jedoch wird noch weiter eingengt um Ausbeuteverluste zu minimieren:
71,00 liter einer ca. 6,2 %igen Lösung aus Beispiel 8 in einer Mischung Acetonitiril/Methanol 40:60 (entspricht 4,00 kg Verbindung ent-(I)) wurden über eine Filterkerze (1,2 um) filtriert und anschließend bei 250 mbar soweit eingeengt, sodaß die Lösung noch rührbar ist. Man fügte 48 1 Ethanol, vergällt mit Toluol zu und destillierte erneut bei 250 mbar bis zur Rührbarkeitsgrenze ab (Umdestillation auf Ethanol). Man gab weitere 48 1 Ethanol, vergällt mit Toluol zu und destillierte anschließend bei Normaldruck bis auf ein Gesamtvolumen von ca. 10 1 ab (Manteltemperatur 98°C). Man kühlte über eine Rampe (4 Stunden) auf 0°C ab, rührte 2 Stunden bei 0°C nach und filtrierte das Produkt ab. Es wurde zweimal mit je 21 kaltem Ethanol nachgewaschen und anschließend im Vakuum bei 50°C getrocknet.
Ausbeute: 3,88 kg (97 % d. Theorie) eines farblosen kristallinen Pulvers
Enantiomerenreinheit: >> 99 % e.e. (HPLC-Methode D); Retentionszeiten/RRT: (4S)- 4-(4-Cyano-2-methoxyphenyl)-5-ethoxy-2,8-dimethyl-1,4-dihydro-1,6-naphthyridin-3-carbox-amid (1) ca. 11 min. RRT: 1,00 ; (4R)- 4-(4-Cyano-2-methoxyphenyl)-5-ethoxy-2,8-dimethyl-1,4-dihydro-1,6-naphthyridin-3-carbox-amid (I) ca. 9 min. RRT: 0,82
Reinheit: > 99,8% (HPLC-Methode B), RT: ca. 6,7 min.
Gehalt: 99,9 % (gegen externen Standard) spezifischer Drehwert (Chloroform, 589 nm, 19.7°C, c = 0.38600 g / 100 ml): + 148.8°.
MS (EIpos): m/z = 379 [M+H]+
1H-NMR (300 MHz, DMSO-d6): □= 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).
Schmelzpunkt: 252 °C

### Chemische Oxidation

### Beispiel 10

### Herstellung von racemischen (XVII) aus racemischen (XIII) durch chemische Methoden

### Rac 4-(4-cyano-2-methoxyphenyl)-5-ethoxy-2,8-dimethyl-1,6-naphthyridin-3-carboxamid

100,00 g (264,25 mmol) 4(R,S)- 4-(4-Cyano-2-methoxyphenyl)-5-ethoxy-2,8-dimethyl-1,4-dihydro-1,6-naphthyridin-3-carboxamid (XIII) wurden in 4 kg Dichlormethan vorgelegt und 68,98 g (303,88 mmol) 2,3-Dichlor-5,6-dicyano-1,4-benzochinon (DDQ) bei 20°C zugegeben. Man rührte 1 h bei 20°C nach. Der ausgefallene Niederschlag wurde abfiltriert und 2-mal mit jeweils 400 g Dichlormethan nachgewaschen. Man engte im Vakuum zur Trockene ein und nahm den Rückstand in 1200 g Ethanol auf. Man erwärmte auf Rückfluss und destillierte ca. 800g Ethanol ab. Man ließ auf Raumtemperatur abkühlen und rührte 1 h bei 20°C nach. Man filtrierte das Produkt ab und wusch mit wenig Ethanol nach (ca. 80 g), trocknete über Nacht im Vakuum (50°C).
Ausbeute: 87,30 g (87,54 % d. Theorie) eines beigen Feststoffes.
MS (EIpos): m/z = 378 [M+H]+
1H NMR (500 MHz, DMSO-d6): δ = 0.72 (t, 3H), 2.50 (s, 3H), 2.70 (s, 3H), 3.65 (s, 1H), 4.00 (m (breit), 2H), 7.30 (d, 1H), 7.45 (d, 1H), 7.50 (s, 2H), 7.69 (s, 1H), 8.05 (s, 1H)

### Beispiel 11a

### Herstellung von M1a(S) aus ent-(I) durch chemische Methoden

### (S)-4-(4-cyano-2-methoxyphenyl)-5-ethoxy-2,8-dimethyl-1,6-naphthyridin-3-carboxamid (M1a(S))

100,00 g (264,25 mmol) 4(R)- 4-(4-Cyano-2-methoxyphenyl)-5-ethoxy-2,8-dimethyl-1,4-dihydro-1,6-naphthyridin-3-carbox-amid (ent-(I)) wurden in 4 kg Dichlormethan vorgelegt und 68,98 g (303,88 mmol) 2,3-Dichlor-5,6-dicyano-1,4-benzochinon (DDQ) bei 20°C zugegeben. Man rührte 1 h bei 20°C nach. Der ausgefallene Niederschlag wurde abfiltriert und 2-mal mit jeweils 400 g Dichlormethan nachgewaschen. Man engte im Vakuum zur Trockene ein und nahm den Rückstand in 1200 g Ethanol auf. Man erwärmte auf Rückfluss und destillierte ca. 800g Ethanol ab. Man ließ auf Raumtemperatur abkühlen und rührte 1 h bei 20°C nach. Man filtrierte das Produkt ab und wusch mit wenig Ethanol nach (ca. 80 g), trocknete über Nacht im Vakuum (50°C).
Ausbeute: 85,80 g (86,04 % d. Theorie) eines beigen Feststoffes.
HPLC: RT ca. 6,08 min. (Chirale Phase: Chiralpak AS-H (250 x 4 mm), Laufmittel: i-Hexan : Ethanol = 50:50)
MS (EIpos): m/z = 378 [M+H]+
1H NMR (500 MHz, DMSO-d6): δ = 0.72 (t, 3H), 2.50 (s, 3H), 2.70 (s, 3H), 3.65 (s, 1H), 4.00 (m (breit), 2H), 7.30 (d, 1H), 7.45 (d, 1H), 7.50 (s, 2H), 7.69 (s, 1H), 8.05 (s, 1H)

### Beispiel 11b

### Herstellung von Mlb(R) aus (I)

### (R)-4-(4-cyano-2-methoxyphenyl)-5-ethoxy-2,8-dimethyl-1,6-naphthyridin-3-carboxamid (M1b(R))

100,00 g (264,25 mmol) 4(S)-4-(4-Cyano-2-methoxyphenyl)-5-ethoxy-2,8-dimethyl-1,4-dihydro-1,6-naphthyridin-3-carbox-amid (I) wurden in 4 kg Dichlormethan vorgelegt und 68,98 g (303,88 mmol) 2,3-Dichlor-5,6-dicyano-1,4-benzochinon (DDQ) bei 20°C zugegeben. Man rührte 1 h bei 20°C nach. Der ausgefallene Niederschlag wurde abfiltriert und 2-mal mit jeweils 400 g Dichlormethan nachgewaschen. Man engte im Vakuum zur Trockene ein und nahm den Rückstand in 1200 g Ethanol auf. Man erwärmte auf Rückfluss und destillierte ca. 800g Ethanol ab. Man ließ auf Raumtemperatur abkühlen und rührte 1 h bei 20°C nach. Man filtrierte das Produkt ab und wusch mit wenig Ethanol nach (ca. 80 g), trocknete über Nacht im Vakuum (50°C).
Ausbeute: 85,80 g (86,04 % d. Theorie) eines beigen Feststoffes.
HPLC : RT ca. 9,03 min. (Chirale Phase: Chiralpak AS-H (250 x 4 mm), Laufmittel: i-Hexan : Ethanol = 50 : 50)
MS (EIpos): m/z = 378 [M+H]+
1H NMR (500 MHz, DMSO-d6): δ = 0.72 (t, 3H), 2.50 (s, 3H), 2.70 (s, 3H), 3.65 (s, 1H), 4.00 (m (breit), 2H), 7.30 (d, 1H), 7.45 (d, 1H), 7.50 (s, 2H), 7.69 (s, 1H), 8.05 (s, 1H)

### Beispiel 12a

### Herstellung von racemischen (XVII) aus ent-(I)

### 4-(4-cyano-2-methoxyphenyl)-5-ethoxy-2,8-dimethyl-1,6-naphthyridin-3-carboxamid

100,00 g (264,25 mmol) 4(R)- 4-(4-Cyano-2-methoxyphenyl)-5-ethoxy-2,8-dimethyl-1,4-dihydro-1,6-naphthyridin-3-carboxamid (ent-(I)) wurden in 4 kg Dichlormethan vorgelegt und 68,98 g (303,88 mmol) 2,3-Dichlor-5,6-dicyano-1,4-benzochinon (DDQ) bei 20°C zugegeben. Man rührte 1 h bei 20°C nach. Der ausgefallene Niederschlag wurde abfiltriert und 2-mal mit jeweils 400 g Dichlormethan nachgewaschen. Man engte im Vakuum zur Trockene ein und nahm den Rückstand in 1200 g Ethanol auf. Man erwärmte im Autoklaven 3 Stunden bei 120°C unter Druck und destillierte anschließend ca. 900g Ethanol ab. Man ließ auf Raumtemperatur abkühlen und rührte 1 h bei 20°C nach. Man filtrierte das Produkt ab und wusch mit wenig Ethanol nach (ca. 40 g), trocknete über Nacht im Vakuum (50°C).
Ausbeute: 92,47 g (92,73 % d. Theorie) eines beigen Feststoffes.
MS (EIpos): m/z = 378 [M+H]+
1H NMR (500 MHz, DMSO-d6): δ = 0.72 (t, 3H), 2.50 (s, 3H), 2.70 (s, 3H), 3.65 (s, 1H), 4.00 (m (breit), 2H), 7.30 (d, 1H), 7.45 (d, 1H), 7.50 (s, 2H), 7.69 (s, 1H), 8.05 (s, 1H)

### Beispiel 12b

### Herstellung von Mla (S) aus ent-(I) durch HNO₃ Oxidation

Durchführung unter N₂. 75,0 g 4(R)- 4-(4-Cyano-2-methoxyphenyl)-5-ethoxy-2,8-dimethyl-1,4-dihydro-1,6-naphthyridin-3-carbox-amid (ent(I)) wurden in 1000 g ACN suspendiert und auf 9°C gekühlt. Dazu wurden 12,68 g rauchende Salpetersäure innerhalb von 10 Min zu getropft. Der Feststoff klumpte kurz zusammen - löste sich danach sofort auf. Man ließ auf RT kommen (Dauer 1 h) und erhielt eine hellgelbe, klare Lösung. Man rührte 4 h bei RT, nach ca. 30 Min bildete sich eine dunkelorangefarbene Lösung, danach eine schmutzig gelbe Suspension. Nach 4 h wurde der Ansatz auf 10°C gekühlt und mit 50 ml Wasser gequenscht. Anschließend wurde mit 80 ml ges. NaHCO₃ - Lösung auf pH 7,2 gestellt (man erhielt eine gelbe Suspension). Feststoff isoliert (erste Teilmenge), mit Wasser gewaschen. Filtrat am Rotationsverdampfer bei 40°C auf ca. 1/3 des Ursprung-Volumens reduziert, 1,5 h im Eisbad gerührt (5°C), den ausgefallenen Feststoff (zweite Teilmenge) isoliert, mit 100 ml kaltem Wasser gewaschen. Die erhaltenen Feststoffe wurden über Nacht im Vakuum getrocknet.
Ausbeute: 59,7 g = 86,7 % d.Th
Analytik 1.Teilmenge
EE: M1a: 83,6 % Mlb: 16,4 %
Gehalt: 98,9 %
Analytik 2. Teilmenge
EE: M1a: 77,4 % Mlb: 22,6 %
Reinheit: 99,2 F1.%
Gehalt: 94,5 %

### Herstellung von racemischen. M1(XVII) aus angereichertem M1a (S)

100 g angereichertes M1a (EE: M1a: 83,6 % Mlb: 16,4 %) wurden in 1000 ml n-Butanol vorgelegt und auf 135°C Badtemperatur erhitzt. Es wurde 6 h unter Rückfluss weitergerührt (gelbe, dünne Suspension). Man kühlt ab und lässt über Nacht bei RT rühren. Die Lösung wurde am Rotationsverdampfer bei ca. 50°C eingeengt (bis zur rührbaren Suspension) und anschließend wurde 1 h bei 5°C gerührt. Man filtrierte die Kristalle ab, wusch mit wenig kaltem Butanol nach und trocknete über Nacht im Vakuum bei 40°C < 200 mbar .
Ausbeute: 85,9 g = 85,9 % d.Th (auf Gehalt Edukt bezogen : 90,9 % d. Th.)
EE: 50,5 % M1a, 49,5 % Mlb

### Elektrochemische Oxidation

### Beispiel 24

### Cyclovoltammetrie von (I) in Abwesenheit von 2,3-Dichlor-5,6-dicyano-1,4-benzochinon (DDQ)

2,17 g (10 mmol) Tetraethylammoniumtetrafluoroborat (Et4NBF4) werden in 100 ml Acetonitril gelöst. Dann werden 378,4 mg (1 mmol) (4S)-4-(4-Cyano-2-methoxyphenyl)-5-ethoxy-2,8-dimethyl-1,4-dihydro-1,6-naphthyridin-3-carboxamid (I) zugegeben.

Die Cyclovoltammetrie wird mit einem Pt-Käfig als Arbeitselektrode und einem Pt-Draht als Gegenelektrode und Ag/Ag+ (10 mmol/1) in Acetonitril als Referenzelektrode über 10 Zyklen mit einer Abtastrate von 250 bzw. 100 mV/s durchgeführt.

### Beispiel 25

### Cyclovoltammetrie von (VI) in Gegenwart von DDQ

2,17 g (10 mmol) Tetraethylammoniumtetrafluoroborat (Et4NBF4) werden in 100 ml Acetonitril gelöst. Dann werden 22,7 mg (0,1 mmol) DDQ und 378,4 mg (1 mmol) der Verbindung der Formel (I) zugegeben. Das DDQ:DHP-Molverhältnis beträgt somit 1:10.

Die Cyclovoltammetrie wird mit einem Pt-Käfig als Arbeitselektrode und einem Pt-Draht als Gegenelektrode und Ag/Ag+ (10 mmol/l) in Acetonitril als Referenzelektrode über 10 Zyklen mit einer Abtastrate von 250 bzw. 100 mV/s durchgeführt.

### Beispiel 26:

### Oxidation von ent-(I) in Gegenwart von DDQ (10 Mol-%)

2,17 g (10 mmol) Tetraethylammoniumtetrafluoroborat (Et4NBF4) werden in 100 ml Acetonitril gelöst. Dann werden 22,7 mg (0,1 mmol) DDQ und 378,4 mg (1 mmol) der Verbindung der Formel ent-(I) (10 mmol) zugegeben. Das DDQ:ent-(I)-Molverhältnis beträgt somit 1:10.

Dann wird die Lösung bei konstantem Potential elektrolysiert, wobei die Anode (Arbeitselektrode) bei einem Potential von +300 mV gegen Ag/Ag+ (10 mmol/l) gehalten wird. Nach Durchgang von 180 C Ladung (entspricht 2,1 F) (über einen Zeitraum von ungefähr 2 h) wird die Reaktion gestoppt. An diesem Punkt betrug die Ausbeute an (XVII) 94% mit einem Atropisomerenverhältnis Mla(S):Mlb(R) = 90:10.

Das Reaktionsprofil wurde durch häufige Entnahme von Proben und Analyse mittels HPLC verfolgt. Das Profil ist in Abbildung 6 angegeben. Das Produkt (XVII) und die Edukte nehmen mit der Zeit zu (ab). Die Produktbildung stimmt mit dem Transfer elektrischer Ladung überein, was eine hohe Stromeffizienz anzeigt.

### Beispiel 27:

### Oxidation von (I) in Gegenwart von DDQ (1 Mol-%)

2,17 g (10 mmol) Tetraethylammoniumtetrafluoroborat (Et4NBF4) werden in 100 ml Acetonitril gelöst. Dann werden 2,3 mg (0,01 mmol) DDQ und 378,4 mg (1 mmol) der Verbindung der Formel (I) zugegeben. Das DDQ:(I)-Molverhältnis beträgt somit 1:100.

Dann wird die Lösung bei konstantem Potential elektrolysiert, wobei die Anode (Arbeitselektrode) bei einem Potential von +300 mV gegen Ag/Ag+ (10 mmol/l) gehalten wird. Nach Durchgang von 180 C Ladung (2,1 F) (über einen Zeitraum von ungefähr 4 h) wird die Reaktion gestoppt. An diesem Punkt betrug die Ausbeute an M1 gemäß HPLC-Analytik 89% (Mla:Mlb = 13:87). Durch anschließende Zugabe von 2,3 mg (0,01 mmol) DDQ (und somit Erhöhung von dessen Anteil auf 2 Mol-%) und anschließende Elektrolyse über einen Zeitraum von 1 h stieg die Ausbeute gemäß HPLC-Analyse auf 96% der Verbindung der Formel (XVII) an (M1a(S):M1b(R) = 13:87).

### Beispiel 28:

### Direkte elektrochemische Oxidation von (XIII)

2,17 g (10 mmol) Tetraethylammoniumtetrafluoroborat (Et4NBF4) werden in 100 ml Acetonitril gelöst. Dann werden 378,4 mg (1 mmol) der Verbindung der Formel (XIII) zugegeben.

Dann wird die Lösung bei konstantem Potential elektrolysiert, wobei die Anode (Arbeitselektrode) bei einem Potential von +1000 mV gegen Ag/Ag+ (10 mmol/1) gehalten wird. Nach Durchgang von 180 C Ladung (2,1 F) (über einen Zeitraum von ungefähr 2 h) wird die Reaktion gestoppt. An diesem Punkt betrug die Ausbeute an (XVII) **<50%.**

### Beispiel 29:

### Racemisierung und Isolierung von (XVII) nach mediierten elektrochemischer Oxidation

Die Lösung aus Beispiel 26 wird in 200 g Ethanol aufgegeben. Man erwärmte im Autoklaven 3 Stunden bei 120°C unter Druck und destillierte ca. 150g Ethanol ab. Man ließ auf Raumtemperatur abkühlen und rührte 1 h bei 20°C nach. Man filtrierte das Produkt ab und wusch mit wenig Ethanol nach (ca. 80 g), trocknete über Nacht im Vakuum (50°C).

### Elektrochemische Reduktion

Als Edukte für die elektrochemische Reduktion wurden die durch die Oxidation der Verbindung der Formel ent-I, der Verbindung der Formel (XIII) bzw. vergleichsweise aus der Verbindung der Formel (I) erhaltenen Atropisomere der Verbindung 4-(4-cyano-2-methoxyphenyl)-5-ethoxy-2,8-dimethyl-1,6-naphthyridine-3-carboxamid, d.h. die Verbindungen Mla(S) bzw. Mlb(R) als auch deren Gemisch (rac.M1) eingesetzt.

Der Umsatz dieser Edukte als auch die Ausbeute des Zielproduktes wurden durch kontinuierliche Probenahme während der elektrochemischen Reduktion und sich anschließende HPLC Analyse bestimmt [HPLC-Methode E]. Das auf 100 normierte Enantiomeren-Verhältnis I zu ent-(I) wurde einmalig am Ende des Versuchs zusätzlich mittels einer chiralen HPLC-Methode ermittelt [HPLC-Methode F].

### Beispiel 13

### Reduktion der Verbindung Mlb(R) (0,2 g Ansatz)

Als Aufbau diente ein Drei-Elektroden-System bestehend aus der Arbeitselektrode [Netzelektrode nach Winkler bestehend aus Platin/Iridium 90/10% (225 Maschen/cm2, Draht-Durchmesser = 0,12 mm, Zylinder-Geometrie), einer Gegenelektrode [Firma ALS: Platin-Draht, gewickelt, 23 cm lang mit 0,5 mm Drahtdurchmesser] und einer Referenzelektrode [Firma ALS: Ag/Ag+ Typ; nicht wässrige Referenzelektrode mit 0,01 M AgNO3 und 0,1 M Tetrabutylammonium Perchlorat in Acetonitril]. Die Gegenelektrode wird in ein am Boden mittels Membran verschlossenes Glasrohr positioniert. Als Membran wurde ein PTFE-Filter [Firma: Sartorius Stedim Biotech GmbH] mit einer Porengröße von 0,45 µm eingesetzt. Als Strom- und Spannungsquelle diente ein Potentiostat der Firma Gamry [Typ: Interface 1000].

0,2 g der Verbindung Mlb(R) (0,53 mmol) erhalten aus Beispiel 11b wurden in 75 g Methanol aufgelöst. Zusätzlich wurden 3,2 g Leitsalz Tetraethylammonium Tetrafluoroborat (14,74 mmol) hinzugefügt. Das Becherglas wurde mit dieser Lösung befüllt. In die durch die Membran abgetrennte Kammer der Gegenelektrode wurde eine Substrat-freie Lösung bestehend aus 0,16 M Tetraethylammonium Tetrafluoroborat in Methanol hinzugegeben.

Für 2 Stunden wurde ein Strom von -30mA geregelt. Danach wurde der Strom auf -180mA eingestellt. Nach weiteren 4 h wurde ein Umsatz von >99% und eine in-situ Ausbeute > 97% ermittelt. Das Enantiomeren-Verhältnis (I):ent-(I) wurde mit 79:21 ermittelt.

### Beispiel 14

### Reduktion der Verbindung M1b(R) (1.0 g Ansatz)

Als Aufbau diente erneut ein Drei-Elektroden-System wie in Beispiel 13 beschrieben.

1,0 g der Verbindung Mlb(R) (2,66 mmol) erhalten aus Beispiel 11b wurden in 80 g Methanol suspendiert, wobei das Substrat nahezu vollständig gelöst wurde. Zusätzlich wurden 4,5 g Leitsalz Tetraethylammonium Tetrafluoroborat (20,73 mmol) hinzugefügt. Das Becherglas wurde mit dieser Lösung befüllt. In die durch die Membran abgetrennte Kammer der Gegenelektrode wurde eine Substrat-freie Lösung bestehend aus 0,21 M Tetraethylammonium Tetrafluoroborat in Methanol hinzugegeben.

Der Versuch wurde potentiostatisch gefahren, wobei das Zielpotential von -3 V gegen die Referenzelektrode nicht erreicht wurde. Über die gesamte Versuchsdauer lag die durch den Interface 1000 einzustellende maximal mögliche Zellspannung an ("compliance voltage" laut Hersteller: 22 V). Nach 6 h Versuchszeit und einem Ladungsfluss von 2650 Coulomb (entspricht einem durchschnittlichen Stromfluss von 122 mA) wurde ein Umsatz von >99% und eine in-situ Zielproduktausbeute > 97% ermittelt. Das Enantiomeren-Verhältnis I:ent-(I) wurde mit 76:24 ermittelt.

### Beispiel 15

### Reduktion der Verbindung Mlb(R) (1,0 g Ansatz)

Als Aufbau diente erneut ein Drei-Elektroden-System wie in Beispiel 13 beschrieben.

1,0 g der Verbindung Mlb(R) (2,66 mmol) erhalten aus Beispiel 11b wurden in 80 g Methanol suspendiert, wobei das Substrat nahezu vollständig gelöst wurde. Zusätzlich wurden 3,5 g Leitsalz Tetraethylammonium Tetrafluoroborat (16,12 mmol) hinzugefügt. Das Becherglas wurde mit dieser Lösung befüllt. In die durch die Membran abgetrennte Kammer der Gegenelektrode wurde eine Substrat-freie Lösung bestehend aus 0,16 M Tetraethylammonium Tetrafluoroborat in Methanol hinzugegeben.

Der Versuch wurde potentiostatisch analog zu Bsp. 14 gefahren.

Nach 4 h Versuchszeit und einem Ladungsfluss von 2193 Coulomb (entspricht einem durchschnittlichen Stromfluss von 152 mA) wurde der Versuch beendet. Der Umsatz betrug zu diesem Zeitpunkt 79% und die in-situ Zielproduktausbeute wurde mit 79% ermittelt. Das Enantiomeren-Verhältnis (I):ent-(I) wurde mit 78:22 bestimmt.

### Beispiel 16

### Reduktion der Verbindung M1a(S) (0.5 g Ansatz)

Als Aufbau diente erneut ein Drei-Elektroden-System wie in Beispiel 13 beschrieben.

0,5 g der Verbindung Mla(S) (1,33 mmol) erhalten aus Beispiel 1 la wurden in 80 g Methanol gelöst. Zusätzlich wurden 3,5 g Leitsalz Tetraethylammonium Tetrafluoroborat (16,12 mmol) hinzugefiigt. Das Becherglas wurde mit dieser Lösung befüllt. In die durch die Membran abgetrennte Kammer der Gegenelektrode wurde eine Substrat-freie Lösung bestehend aus 0,16 M Tetraethylammonium Tetrafluoroborat in Methanol hinzugegeben.

Der Versuch wurde potentiostatisch analog zu Bsp. 14 gefahren.

Nach 5 h Versuchszeit und einem Ladungsfluss von 2132 Coulomb (entspricht einem durchschnittlichen Stromfluss von 118 mA) wurde der Versuch beendet. Der Umsatz betrug zu diesem Zeitpunkt 73% und die in-situ Zielproduktausbeute wurde mit 73% ermittelt. Das Enantiomeren-Verhältnis (I):ent-(I) wurde mit 22:78 bestimmt.

### Beispiel 17

### Herstellung von racemischen (XIII) aus racemischen M1(XVII): Reduktion des Atropisomeren-Gemisches bestehend aus 50gew.% Mlb(R) und 50gew.% Mla(S) (0,5 g Racemat-Ansatz)

Als Aufbau diente erneut ein Drei-Elektroden-System wie in Beispiel 13 beschrieben.

0,5 g des Mla(S)/Mlb(R) Racemates (1,33 mmol) erhalten aus Beispiel 12 wurden in 80 g Methanol gelöst. Zusätzlich wurden 3,5 g Leitsalz Tetraethylammonium Tetrafluoroborat (16,12 mmol) hinzugefügt. Das Becherglas wurde mit dieser Lösung befüllt. In die durch die Membran abgetrennte Kammer der Gegenelektrode wurde eine Substrat-freie Lösung bestehend aus 0,16 M Tetraethylammonium Tetrafluoroborat in Methanol hinzugegeben.

Der Versuch wurde potentiostatisch analog zu Bsp. 14 gefahren.

Nach 4,5 h Versuchszeit und einem Ladungsfluss von 2500 Coulomb (entspricht einem durchschnittlichen Stromfluss von 154 mA) wurde der Versuch beendet. Der Umsatz betrug zu diesem Zeitpunkt 79% und die in-situ Zielproduktausbeute wurde mit 79% ermittelt. Das Enantiomeren-Verhältnis I:ent-(I) wurde mit 50:50 bestimmt.

### Beispiel 18

### Reduktion der Verbindung Mlb(R) (0,6 g Ansatz)

Als Aufbau diente erneut ein Drei-Elektroden-System wie in Beispiel 13 beschrieben.

0,6 g der Verbindung Mlb(R) (1,59 mmol) erhalten aus Beispiel 11b wurden in einem Lösungsmittelgemisch bestehend aus 50 g Methanol und 50 g N,N Dimethylformamid gelöst. Zusätzlich wurden 6 g Leitsalz Tetraethylammonium Tetrafluoroborat (27,64 mmol) hinzugefügt. Das Becherglas wurde mit dieser Lösung befüllt. In die durch die Membran abgetrennte Kammer der Gegenelektrode wurde eine Substrat-freie Lösung bestehend aus 0,24 M Tetraethylammonium Tetrafluoroborat in Methanol hinzugegeben.

Der Versuch wurde potentiostatisch analog zu Bsp. 14 gefahren.

Nach 4,5 h Versuchszeit und einem Ladungsfluss von 1187 Coulomb (entspricht einem durchschnittlichen Stromfluss von 73 mA) betrug der Umsatz 98% und die in-situ Zielproduktausbeute wurde mit 95% ermittelt. Das Enantiomeren-Verhältnis (I):ent-(I) wurde mit 83:17 bestimmt.

### Beispiel 19

### Reduktion der Verbindung M1b(R) (0,6 g Ansatz)

Aufbau und Versuchsbedingungen wurden analog zu Bsp. 18 gewählt mit der Ausnahme, dass als Arbeitselektrode eine poröse Kohlenstoffelektrode zum Einsatz kam (Firma: ALS).

Der Versuch wurde potentiostatisch analog zu Bsp. 14 gefahren.

Nach 3 h und 10 min Versuchszeit und einem Ladungsfluss von 494 Coulomb (entspricht einem durchschnittlichen Stromfluss von 43 mA) betrug der Umsatz 100% und die in-situ Zielproduktausbeute wurde mit 97% ermittelt. Das Enantiomeren-Verhältnis I:ent-(I) wurde mit 52:48 bestimmt.

### Beispiel 20

### Reduktion der Verbindung M1b(R) (Durchflusszelle)

In weiterführenden Beispielen wurde anstelle der Becherglaszelle eine Durchflusszelle (Micro Flow Cell) der Firma Electrocell eingesetzt. Als Arbeitselektrode wurde eine mit Platin beschichtete Titanelektrode eingesetzt. Als Gegenelektrode diente Graphit. Anolyt- und Katholytkammer wurden mittels Kationenaustauscher Membran (Typ fumapem F-9100-PK der Firma Fumatech) voneinander getrennt. Die Membran wurde im Vorfeld in VE-Wasser getaucht und feucht eingebaut. Nach erfolgtem Zusammenbau der Zelle wurde diese mit Methanol gespült. Mittels Schlauchpumpen [Typ: Sci-Q 323; Firma: Watson Marlow] konnte zunächst die Methanol-Spülung und später die Reaktionslösungen durch beide Halbzellen kontinuierlich gefördert werden (jeweils 6 1/h).

Als Strom- und Spannungsquelle diente ein Potentiostat der Firma Gamry [Typ: Reference 3000].

1 g der Verbindung Mlb(R) (2,66 mmol) erhalten aus Beispiel 11b wurden in einem Lösungsmittelgemisch bestehend aus 4 g Methanol und 190 g DMF aufgelöst. Zusätzlich wurden 4,5 g Leitsalz Tetraethylammonium Tetrafluoroborat (20,73 mmol) hinzugefügt. Mit dieser Lösung wurde der Katholytkreislauf über den im Kreislauf integrierten Reservoir-Behälter gefüllt. Eine analoge Lösung ohne die Verbindung Mlb(R) wurde in den Anolytkreislauf gefüllt.

Bei dem Versuch wurde der Stromfluss auf max. 300 mA begrenzt. Nach einem Ladungsfluss von ca. 1000 C (entspricht 4F) betrug der Umsatz 63% und nach insgesamt 3000 C (12F) Umsatz >94%. Es wurden keine signifikanten Nebenprodukte beobachtet.

### Beispiel 21

### Herstellung von racemischen (XIII) aus racemischen M1(XVII)

Als Elektrolyse-Zelle diente erneut, die wie in Beispiel 20 beschriebene Micro Flow Cell der Firma Electrocell. Abweichend zu Beispiel 20 wurden diesmal 10 g (26,6 mmol) der Verbindung rac.M1, erhalten aus Beispiel 12, in einem Lösungsmittelgemisch bestehend aus 4 g Methanol und 190 g DMF aufgelöst. Zusätzlich wurden 4,5 g Leitsalz Tetraethylammonium Tetrafluoroborat (20,73mmol) hinzugefügt. Mit dieser Lösung wurde der Katholytkreislauf gefüllt. Eine analoge Lösung ohne Edukt wurde in den Anolytkreislauf gefüllt.

Nach einem Ladungsfluss von 30000C (12F) wurde die elektrochemische Reduktion gestoppt. Die mittels HPLC (Methode E) ermittelte in situ Ausbeute an rac. (XIII) betrug 95%. Die Katholytlösung wurde anschließend der Aufarbeitung zugeführt.

Isolierung von rac-(XIII): Das Lösungsmittel wird zuerst weitgehend abdestilliert und anschließend das Produkt durch eine Wasser-Fällung (Zugabe von Wasser) ausgefällt, abfiltriert und getrocknet.

Das so erhaltene Rohprodukt kann aus Ethanol oder THF umkristallisiert werden und erneut einer Enantiomeren -Trennung mittels SMB unterzogen werden.

### Beispiel 30

### Herstellung von racemischen(XIII) aus racemischen M1(XVII): Reduktion des Atropisomeren-Gemisches bestehend aus 50gew.% Mlb (R) und 50gew.% Mla (S) (10 g Racemat-Ansatz)

In weiterführenden Beispielen wurde anstelle der Becherglaszelle eine Durchflusszelle (Micro Flow Cell: 10 cm2 Elektrodenoberfläche) der Firma Electrocell eingesetzt.

Als Arbeitselektrode wurde eine mit Platin beschichtete Titanelektrode eingesetzt. Als Gegenelektrode diente Graphit. Anolyt- und Katholytkammer wurden mittels Kationenaustauscher Membran (Typ Nation® N-424 der Firma DUPONT) voneinander getrennt. Die Membran wurde im Vorfeld in VE-Wasser getaucht und feucht eingebaut. Nach erfolgtem Zusammenbau der Zelle wurde diese mit einem Lösungsmittelgemisch bestehend aus 20 gew.-% Methanol und 80 gew.-% DMF gespült. Mittels Schlauchpumpen [Typ: Sci-Q 323; Firma: Watson Marlow] konnte zunächst die Methanol/DMF-Spüllösung und später die Reaktionslösungen durch beide Halbzellen kontinuierlich gefördert werden (jeweils 5 kg/h). Durch einen separat angeschlossenen Kühlkreislauf wurden beide Elektrolyt-Lösungen (Anolyt- und Katholytkreislauf) auf 20°C temperiert. Als Strom- und Spannungsquelle diente ein Potentiostat der Firma Gamry [Typ: Reference 3000].

10 g der Verbindung rac.M1 (26,6 mmol) erhalten aus Beispiel 12b wurden in einem Lösungsmittelgemisch bestehend aus 21,4 g Methanol und 85,6 g DMF aufgelöst. Zusätzlich wurden 1,25 g Leitsalz Tetraethylammonium Tetrafluoroborat (5,76 mmol) und 1,45 g Essigsäure (24,17 mmol) hinzugefügt. Mit dieser Lösung wurde der Katholytkreislauf über den im Kreislauf integrierten Reservoir-Behälter gefüllt. Eine analoge Lösung ohne die Verbindung rac.M1 wurde in den Anolytkreislauf gefüllt (Anolytmasse bei Versuchsstart = 358,7 g). Bei dem Versuch wurde galvanostatisch gefahren. Der konstante Stromfluss betrug 350 mA. Nach 20 h wurde der Versuch beendet und beide Halbzellenkreisläufe entleert. Der Umsatz der Verbindung rac. M1 betrug 99 %. Die in-situ Ausbeute der Zielkomponente (XIII) betrug nach 20 h > 98 %. Es wurden keine signifikanten Nebenprodukte beobachtet. Die Produktselektivität (XIII) liegt bei ca. 99%. Die Produktkonzentration betrug nach Versuchsende ca. 37 mg/g. Die Verdünnung resultiert aus einem Lösungsmitteltransfer vom Anolyt in den Katholyt (Gesamtmassen an Katholyt und Anolyt nach Zell-Entleerung = 264 bzw. 214 g)

Isolierung von rac-(XIII): Nach Entfernung des Lösungsmittelgemischs (DMF/MeOH) und Leitsalzes wurde das Produkt in hoher Ausbeute und Reinheit erhalten.

### Beispiel 31

### Herstellung von racemischen(XIII) aus racemischen M1(XVII): Reduktion des Atropisomeren-Gemisches bestehend aus 50gew.% Mlb (R) und 50gew.% Mla (S) (10 g Racemat-Ansatz)

Es wurde die in Beispiel 30 beschriebene Elektrolyse-Zelle sowie der dort beschriebene Versuchsaufbau verwendet.

Abweichend dazu wurden 10 g der Verbindung rac.M1 (26,6 mmol) erhalten aus Beispiel 12b in einem Lösungsmittelgemisch bestehend aus 16,6 g Methanol und 66,4 g DMF aufgelöst. Zusätzlich wurden 0,97 g Leitsalz Tetraethylammonium Tetrafluoroborat (4,47 mmol) und 1,09 g Essigsäure (18,1 mmol) hinzugefügt. Mit dieser Lösung wurde der Katholytkreislauf über den im Kreislauf integrierten Reservoir-Behälter gefüllt. Eine analoge Lösung ohne die Verbindung rac.M1 wurde in den Anolytkreislauf gefüllt (Anolytmasse bei Versuchsstart = 282 g).

Bei dem Versuch wurde galvanostatisch gefahren. Der konstante Stromfluss betrug 400 mA.

Ab ca. 6 h Versuchszeit wurde der Katholyt trüb und im Reservoir-Behälter wurde die Bildung eines weißen Niederschlags beobachtet. Nach 10 h wurde der Versuch beendet. Der gebildete Niederschlag wurde filtriert (1.7 g) und ohne weitere Reinigungsschritte mittels HPLC-Analytik untersucht. Racemisches (XIII) wurde mit > 99,6 area% analysiert. Im verbleibenden Filtrat betrug das Verhältnis zwischen Zielprodukt (XIII) und Substrat (rac. M1 (XVII)) 89:10 area.%. Es wurden keine signifikanten Nebenprodukte beobachtet. Die Produktselektivität (XIII) liegt bei ca. 99%.

Die Produktkonzentration (Verbindung XIII) im Filtrat betrug ca. 43 mg/g. Die Gesamtmassen an Katholyt und Anolyt nach Zell-Entleerung betrugen: 174 bzw. 197 g.

### Beispiel 32

### Herstellung von racemischen(XIII) aus racemischen M1(XVII): Reduktion des Atropisomeren-Gemisches bestehend aus 50gew.% Mlb (R) und 50gew.% Mla (S) (36 g Racemat-Ansatz)

Als Elektrolyse-Zelle wurde in diesem Beispiel die Multipurpose Cell (MPC, 100 cm2 Elektrodenfläche) der Firma Electrocell eingesetzt. Als Arbeitselektrode wurde eine mit Platin beschichtete Titanelektrode eingesetzt. Als Gegenelektrode diente Graphit. Anolyt- und Katholytkammer wurden mittels Kationenaustauscher Membran (Typ Nation® N-424 der Firma DUPONT) voneinander getrennt. Die Membran wurde im Vorfeld in VE-Wasser getaucht und feucht eingebaut. Nach erfolgtem Zusammenbau der Zelle wurde diese mit einem Lösungsmittelgemisch bestehend aus 20 gew.-% Methanol und 80 gew.-% DMF gespült. Mittels Kreislaufpumpen [Typ: Labor-Reaktionsmischer HMR 050; Firma: Fink] und Cori-Flow Durchflussmesser (Firma Bronkhorst) konnte zunächst die Methanol/DMF-Spüllösung und später die Reaktionslösungen durch beide Halbzellen kontinuierlich gefördert werden (jeweils 50 kg/h). Die Multipurpose Cell wurde zudem an einen Kühlkreislauf angeschlossen, der durch einen Kryostat (Firma Julabo, Typ: FP45) geregelt wurde. Anolyt- und Katholyt-Temperaturen betrugen 22°C.

Als Strom- und Spannungsquelle diente ein Potentiostat der Firma Delta Elektronika [Typ: ES030-10]. Nach dem Spülen der Anolyt- und Katholytkreisläufe für mindestens 15 min wurden folgende Lösungen in die Reservoir-Behälter eingebracht:
Katholyt: 36 g (95,7 mmol) der Verbindung rac.M1 (XVII) erhalten aus Beispiel 12b wurden in einem Lösungsmittelgemisch bestehend aus 100 g Methanol, 400 g DMF, 6 g (27,64 mmol) Tetraethylammonium tetrafluoroborat (Et4NBF4) und 5 g (83,3 mmol) Essigsäure gelöst.
Anolyt: Hier wurde eine Substrat-freie Lösung eingesetzt, bestehend aus 250 g Methanol, 1000 g DMF, 15 g (69,1 mmol) Leitsalz (Et4NBF4) und 12,5 g (208,3 mmol) Essigsäure.

Bei dem Versuch wurde galvanostatisch gefahren. Der konstante Stromfluss betrug 3A. Nach 10h wurde der Versuch beendet und beide Halbzellenkreisläufe entleert. Der Umsatz der Verbindung rac. M1 betrug 95,7% (HPLC area%). Die in-situ Ausbeute der Zielkomponente (XIII) betrug nach 10 h 95,3% (HPLC area%). Es wurden keine signifikanten Nebenprodukte beobachtet (Selektivität zum Zielprodukt > 99,5%). Die Produktkonzentration betrug nach Versuchsende > 2,6 gew. %. Die Verdünnung resultiert aus dem Lösungsmitteltransfer vom Anolyt in den Katholyt. (Gesamtmassen an Katholyt und Anolyt nach Zell-Entleerung = 1296 g bzw. 482 g).

Isolierung von rac-(XIII): Nach Entfernung des Lösungsmittelgemischs (DMF/MeOH) und Leitsalzes wurde das Produkt in hoher Ausbeute und Reinheit erhalten. Das erhaltene Rohprodukt kann zusätzlich aus Ethanol oder THF umkristallisiert und erneut einer Enantiomeren-Trennung mittels SMB unterzogen werden.

### Beispiel 22

### Einkristall-Röntgenstrukturanalyse der Verbindung der Formel M1b(R): (R)- 4-(4-Cyan-2-methoxyphenyl)-5-ethoxy-2,8-dimethyl-1,6-naphthyridin-3-carboxamid

Analysenmethode: Einkristall-Röntgenstrukturanalyse
Vermessener Kristall: Farbloser Block, 0.40 x 0.20 x 0.20 mm³

### Experimentell:

Die Kristallstrukturbestimmung wurde mit Hilfe eines Diffraktometers (Oxford Diffraction, Xcalibur Serie), ausgestattet mit einem CCD Flächendetektor (Model Ruby), einer sogenannten "sealed tube" Röntgenröhre mit CuKa Strahlung, Osmium-Reflektor als Monochromator und einer Cryojet Kühlvorrichtung für Niedrigtemperaturmessungen (T =100 K), durchgeführt.

360° Datensammlung Omega und Phi Scan. Eingesetzte Programme: Datenaufnahme und -reduktion mit Crysalis (Oxford Diffraction 2007). Die Kristallstrukturlösung wurde mittels direkter Methoden, wie in SHELXTL Version 6.10 (Sheldrick, Universität Göttingen (Germany), 2000) implementiert, durchgeführt, und mittels des XP-Programms visualisiert. Fehlende Atome wurden anschließend mit Hilfe der Differenz-Fourier-Synthese lokalisiert und zu der Atomliste hinzugefügt. Die Verfeinerung nach der Methode der kleinsten Quadrate auf F2 wurde mit allen gemessenen Intensitäten durchgeführt und mit Hilfe des Programms SHELXTL Version 6.10 (Sheldrick, Universität Göttingen (Germany), 2000) durchgeführt. Alle Nichtwasserstoffatome wurden einschließlich anisotroper Auslenkungsparameter verfeinert.
Kristalldaten und Strukturverfeinerung der **Verbindung** der Formel Mlb (R): (R)-4-(4-Cyan-2-methoxyphenyl)-5-ethoxy-2,8-dimethyl-1,6-naphthyridin-3-carboxamid
Identifikationskode: Mlb
Summenformel: C21 H20 N4 O3
Molekularmasse: 376.41
Temperatur: 100 K
Wellenlänge: 1.54178 Å
Kristallsystem: Orthorhombisch
Raumgruppe: P2(1)2(1)2(1)
Gitterkonstanten: a = 9.70950(10) Å
b= 10.67390(10) Å
c= 18.9480(2) Å
Volumen: 1963.74(3) Å
Z 4
Spezifische Dichte (berechnet): 1.273 Mg/m3
Absorptionskoeffizient: 0.714 mm-1
F(000) 792
Kristallmaße: 0.40 x 0.20 x 0.20 mm3
Theta-Bereich für die Datenaufnahme: 4.67 bis 65.66°.
Index-Bereich: -11 ≤ h ≤ 9, -12 ≤ k ≤ 12, -19 ≤ l ≤ 22
Aufgenommene Reflexe: 15493
Unabhängige Reflexe: 3367 [R(int) = 0.0230]
Vollständigkeit bei Theta= 65.66° 99.5 %
Absorption-Korrektur: Crysalis
Verfeinerungsmethode: Volle-Matrix Methode der kleinsten Quadrate auf F2
Daten / Einschränkungen / Parameter: 3367 / 0 / 257
Güte der Anpassung auf F2: 1.048
Finale R Werte: [I>2sigma(I)] R1 = 0.0242, wR2 = 0.0636
R Werte (alle Daten): R1 = 0.0249, wR2 = 0.0641
Absoluter Strukturparameter: -0.18(13)
Grösste und kleinste Differenzdichte: 0.142 und -0.139 e.Ä-3

### Röntgenstrukturanalyse:

Die Röntgenstrukturanalyse zeigte, dass wenn das 1,6-Naphthyridin-3-carboxamid-Ringsystem in der Papierebene liegt, der 4-Cyan-2-methoxyphenyl-Substitunet hierzu quersteht, wobei dann die Methoxygruppe hinter die Papierebene zeigt.

**Bestimmung der Absolut-Konfiguration**

| **Chiralitätsprüfung*** | **Korrekte Struktur** | **Invertierte Struktur** |
|---|---|---|
| **Flack-Parameter (Standardabweichung)** | -0.1838(0.1347) | 1.1745(0.1364) |
| **Twin Basf (Standardabweichung)** | 0.0000(0.1348) | 1.1855(0.1347) |
| **wR2-Wert (mit Flack-Parameter)** | 0.0641 | 0.0649 |
| **Chiralität** | ***Ra*** | ***Sa*** |

H. D. Flack, Acta Cryst., 1983, A39, 876-881
H. D. Flack, G. Bernardinelli, Acta Cryst., 1999, A55, 908-915
H. D. Flack, G. Bernardinelli, J. Appl. Cryst., 2000, 33, 1143-1148.

### Die Verbindung der Formel Mlb(R) besitzt somit die absolute Konfiguration R (Ra)

Die Benennung der absoluten Konfiguration erfolgt nach den Regeln von Cahn-Ingold-Prelog für axial chirale Verbindungen

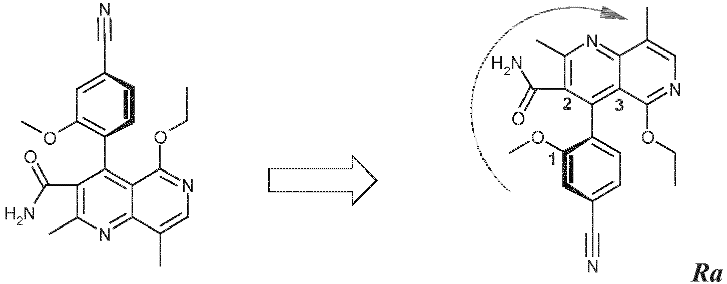

### Beispiel 23

### Bestimmung der Absolutkonfiguration der Mb-Reihe durch Korrelation der CD-Spektren

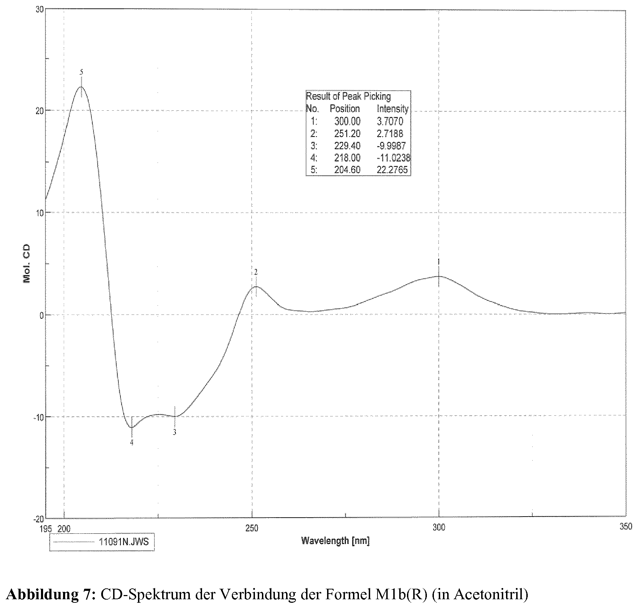

### (Siehe Abbildung 7)

### Physikochemische Charakterisierung von Verbindung der Formel (I) in kristalliner Form der Modifikation I

Verbindung der Formel (I) in kristalliner Form der Modifikation **I** schmilzt bei 252°C, ΔH = 95 - 113 Jg⁻¹ (Heizrate 20 Kmin⁻¹).

Eine Depression des Schmelzpunktes wurde in Abhängigkeit der Heizrate beobachtet.

Der Schmelzpunkt sinkt bei einer geringeren Heizrate (z.B. 2 Kmin⁻¹) weil Zersetzung eintritt.

Es wurden keine weiteren Phasenübergänge beobachtet. Ein Massenverlust von ca. 0,1 % wurde bis zu einer Temperatur von 175°C beobachtet.

### Stabilität und Feuchtigkeits-Einlagerung

Proben von Verbindung der Formel (I) in kristalliner Form der Modifikation **I** wurden bei 85 % and 97 % rel. Luftfeuchtigkeit (25°C) eingelagert. Die Proben wurden nach 12 Monaten mittels DSC, TGA und XRPD ausgewertet. Nach 12 Monaten wird in beiden Fällen eine Massenänderung von < 0,1 % festgestellt. D.h. Verbindung der Formel (I) in kristalliner Form der Modifikation **I** zeigt keine signifikante Wasserabsorption unter diesen Lagerbedingungen. Laut DSC, TGA und XRPD besteht kein Unterschied zu Verbindung der Formel (I) in kristalliner Form der Modifikation I.

### HPLC-Bedingungen/Methoden

### Methode A

YMC Hydrosphere C18
150*4,6 mm, 3,0 µm
25°C, 1 ml/min , 270 nm, 4 nm
0' : 70% TFA 0,1%*; 30% Acetonitril
17': 20% TFA 0,1% ; 80% Acetonitril
18': 70% TFA 0,1 %; 30% Acetonitril
*: TFA in Wasser

### Methode B

YMC Hydrosphere C18
150*4,6 mm, 3,0 µm
25°C, 1 ml/min , 255 nm, 6 nm
0' : 90% TFA 0,1%; 10% Acetonitril
20': 10% TFA 0,1% ; 90% Acetonitril
18': 10% TFA 0,1 %; 90% Acetonitril

### Methode C

Nucleodur Gravity C18
150*2 mm, 3,0 µm
35°C, 0,22 ml/min. ,255 nm, 6 nm
Lösung A: 0,58 g Ammoniumhydrogenphosphat und 0,66 g Ammoniumdihydrogenphosphat in 1 L
Wasser (Ammoniumphosphat-Puffer pH 7,2)
Lösung B: Acetonitril
0' : 30% B ; 70% A
15' : 80% B ; 20% A
25' : 80% B ; 20% A

### Methode D

Säulen-Länge: 25 cm
Innendurchmesser: 4,6 mm
Füllung: Chiralpak IA, 5 µm
Reagenzien: 1. Acetonitril, für die HPLC
2. Methyl-tert-butyl-ether (MTBE), p.a.
Prüflösung Die Probe wird in einer Konzentration von 1,0 mg/mL
in Acetonitril gelöst.
(z. B. ca. 25 mg Probe, genau gewogen, in Acetonitril
zu 25,0 mL lösen.)
Elutionsmittel A. Acetonitril
B. Methyl-tert-butyl-ether (MTBE), p.a.
Durchflussrate 0,8 mL/min
Temperatur des Säulenofens 25 °C
Detektion Messwellenlänge: 255 nm
Bandbreite: 6 nm
Injektionsvolumen 5 µL
Zusammensetzung des Eluenten A und B im Volumenverhältnis 90:10 mischen.
Laufzeit des Chromatogramms 30 min
Retentionszeiten/RRT:
   (4S)-4-(4-Cyano-2-methoxyphenyl)-5-ethoxy-2,8-dimethyl-1,4-dihydro-1,6-naphthyridin-3-carbox-amid (1) ca. 11 min. RRT: 1,00
   (4R)-4-(4-Cyano-2-methoxyphenyl)-5-ethoxy-2,8-dimethyl-1,4-dihydro-1,6-naphthyridin-3-carbx-amid (1) ca. 9 min. RRT: 0,82

### Methode E

YMC Hydrosphere C18
150*4,6 mm, 3 µm Korngröße
25°C, 1 ml/min , typischer Anfangsdruck: ca. 160 bar
Messwellenlänge: 255 nm, Bandbreite: 6 nm

### Gradient:

0': 90% Ameisensäure 0,1%*; 10% Acetonitril
20': 10% Ameisensäure 0,1% ; 90% Acetonitril
25': 90% Ameisensäure 0,1 %; 10% Acetonitril
*: Ameisensäure in Wasser

### Retentionszeiten:

| | |
|---|---|
| Verbindung I bzw. ent-(I): | ca. 9,9 min |
| Verbindung M1a bzw. M1b: | ca. 15,5 min |

### Methode F

Chiralpak IA
150*4,6 mm, 5 µm Korngröße
25°C, 0,8 ml/min
Messwellenlänge: 255 nm, Bandbreite: 6 nm
Mobile Phase: Acetonitril+tert-Butylmethylether (MTBE) im Volumenverhältnis 90:10 gemischt

### Retentionszeiten:

| | |
|---|---|
| Verbindung der Formel M1b(R): | ca. 5,1 min |
| Verbindung der Formel M1a(S): | ca. 5,5 min |
| Verbindung der Formel (I): | ca. 8,6 min |
| Verbindung der Formel ent-(I): | ca. 10,8 min |

### Beschreibung der Abbildungen:

**Abbildung 1****:** Thermische Racemisierung der Verbindung der Formel ent-(I) in 1-Butanol ohne Zusatz einer katalytischen Menge Säure.
**Abbildung 2****:** Thermische Racemisierung der Verbindung der Formel ent-(I) in 1-Butanol mit und ohne Zusatz einer katalytischen Menge Säure.
**Abbildung 3****:** Gängige Typen elektrochemischer Zellen. Becherglas-Zelle, "H"-Zelle und Filterpressen-Durchflusszelle.
**Abbildung 4****:** Reaktionsschema der mediierten elektrochemischen Oxidation von ent-(I) zu (XVII) durch DDQ.
**Abbildung 5****:** Cyclovoltammetrie von DDQ, DHP und der Verbindung der Formel ent-(I) und der Mischung DDQ:DHP (V) 1:10 gemäß den Beispielen 24 und 25
**Abbildung 6****:** Entwicklung von mittels HPLC gemessenem Edukt DHP ent-(I) und Produkt PYR (XVII) als Funktion der Zeit gemäß Beispiel 26. Die Linien stellen den nur auf Elektronenfluss und 100% Stromeffizienz basierenden berechneten Wert dar.
**Abbildung 7****:** CD-Spektrum der Verbindung der Formel Mlb(R) (in Acetonitril)

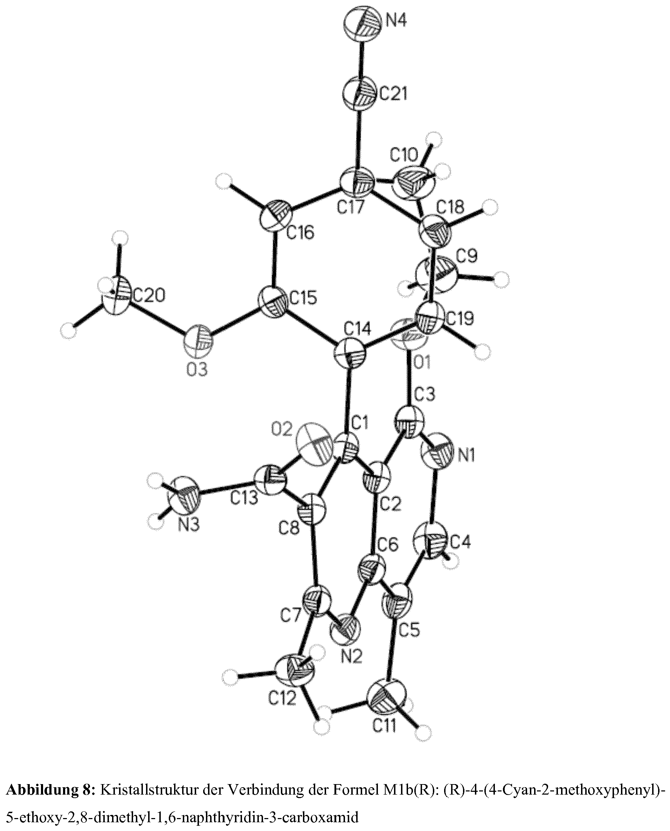

**Abbildung 8****:** Kristallstruktur der Verbindung der Formel M1b(R): (R)-4-(4-Cyan-2-methoxyphenyl)-5-ethoxy-2,8-dimethyl-1,6-naphthyridin-3-carboxamid

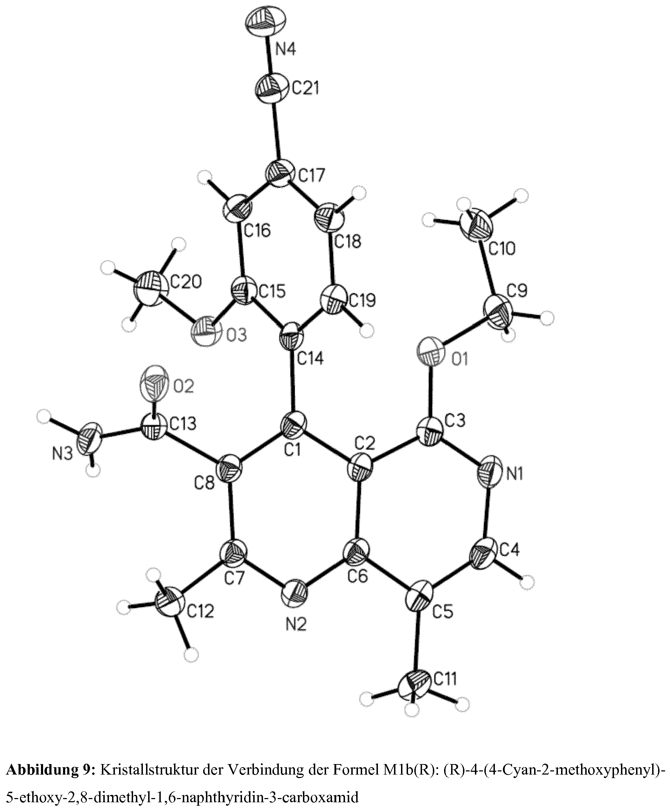

**Abbildung 9****:** Kristallstruktur der Verbindung der Formel M1b(R): (R)-4-(4-Cyan-2-methoxyphenyl)-5-ethoxy-2,8-dimethyl-1,6-naphthyridin-3-carboxamid

## Patentansprüche

1. Verfahren zur Herstellung von Verbindungen der Formeln M1a(S) und M1b(R) 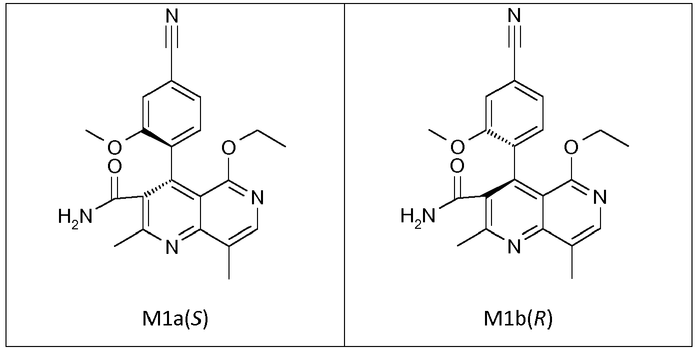 **dadurch gekennzeichnet, dass** die Verbindung der Formel ent-(I) 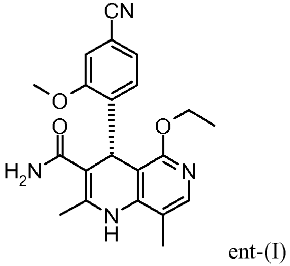 oxidiert wird.

2. Verfahren zur Herstellung von Verbindungen der Formeln Mla(S) und Mlb(R) gemäß Anspruch 1, **dadurch gekennzeichnet, dass** die Oxidation mit chemischen Oxidationsmitteln durchgeführt wird.

3. Verfahren zur Herstellung der racemischen Verbindung der Formel (XVII) 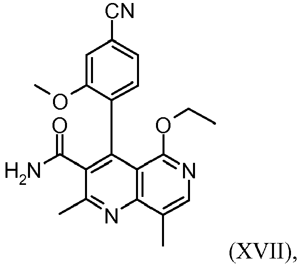 **dadurch gekennzeichnet, dass** ein Gemisch aus den Verbindungen der Formeln M1a(S) und Mlb(R) 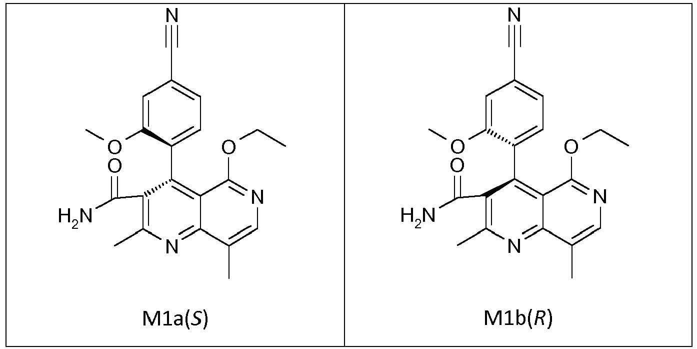 thermisch racemisiert wird.

4. Verfahren zur Herstellung der Verbindung der Formel (XVII) gemäß Anspruch 3, **dadurch gekennzeichnet, dass** ein Gemisch aus den Verbindungen der Formeln Mla(S) und Mlb(R) bei einer Temperatur von 70 bis 110 °C mit oder ohne Zugabe einer Säure racemisiert wird.

5. Verfahren zu Herstellung von Verbindungen der Formel (I) und ent-(I) 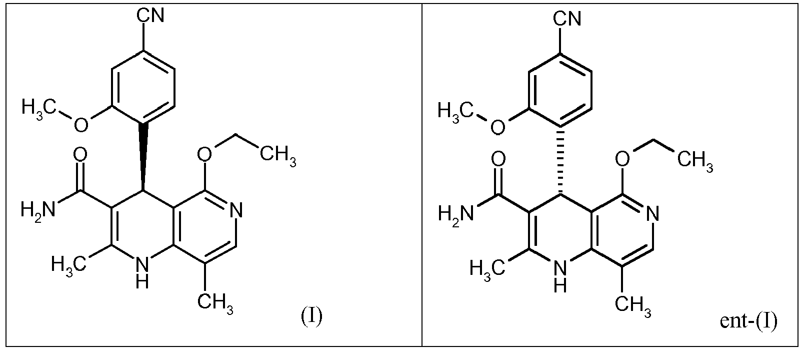 **dadurch gekennzeichnet, dass** Verbindungen der Formeln (XVII) oder Mla(S) oder Mlb(R) oder ein Gemisch aus Mla(S) und Mlb(R) 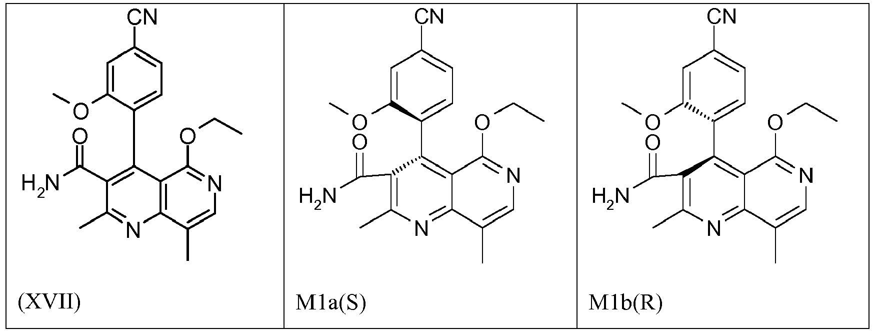 elektrochemisch reduziert wird.

6. Verfahren zu Herstellung der Verbindungen der Formeln (I) und ent-(I) gemäß Anspruch 5, **dadurch gekennzeichnet, dass** die elektrochemische Reduktion in einer Becherglaszelle oder Durchflusszelle in Anwesenheit von Methanol durchgeführt wird.

7. Verfahren zur Herstellung von Verbindungen der Formel (I) und ent-(I) gemäß Anspruch 5 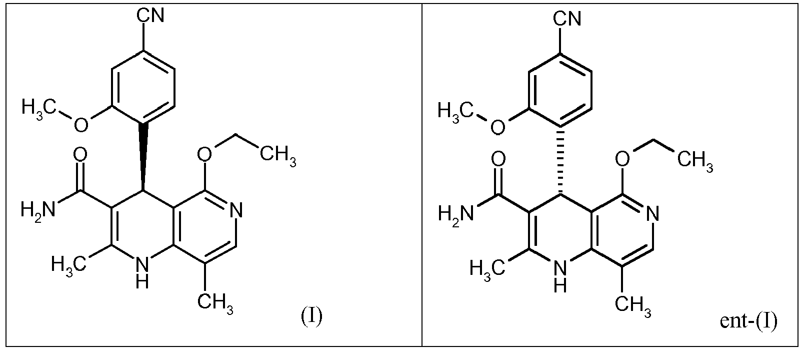 **dadurch gekennzeichnet, dass** Verbindungen der Formeln (XVII) oder Mla(S) oder Mlb(R) oder ein Gemisch aus Mla(S) und Mlb (R) 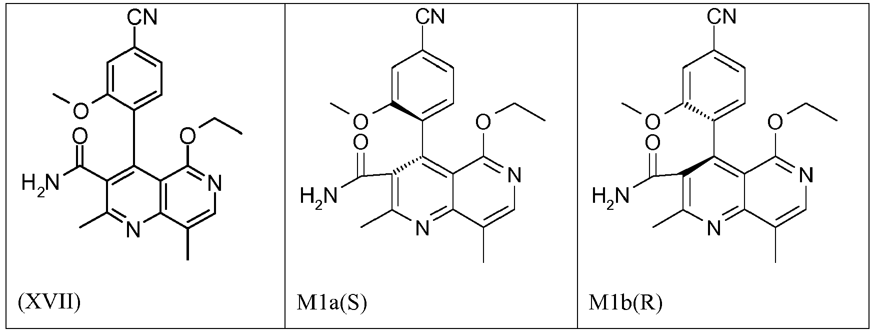 elektrochemisch reduziert werden,
und **dadurch gekennzeichnet, dass** die Verbindungen der Formeln (XVII), Mla(S) und Mlb(R) durch thermische Isomerisierung von Verbindungen der Formeln Mla(S) und Mlb(R) 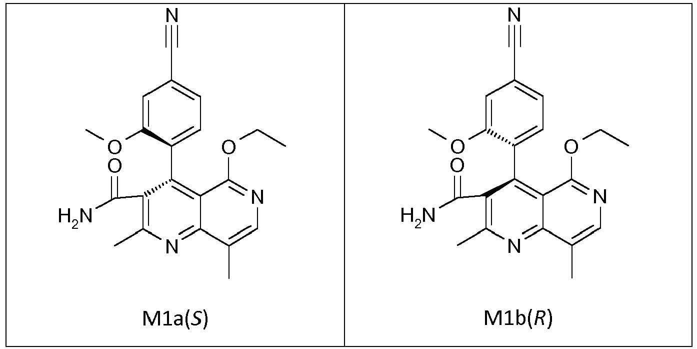 erhalten werden, und **dadurch gekennzeichnet, dass** man die Verbindung der Formel ent-(I) 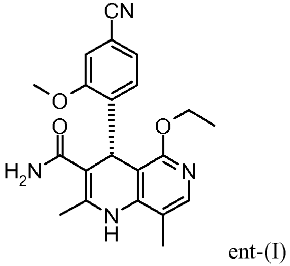 oxidiert.

8. Verfahren zur Herstellung von Verbindungen der Formel (I) und ent-(I) gemäß Anspruch 7 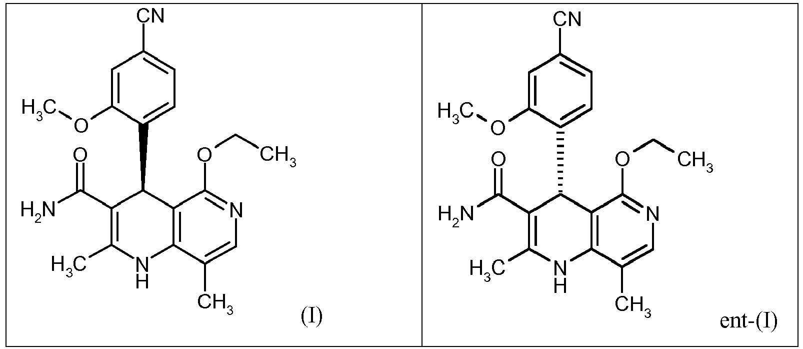 **dadurch gekennzeichnet, dass** Verbindungen der Formeln (XVII) oder Mla(S) oder Mlb(R) oder ein Gemisch aus Mla(S) und Mlb(R) 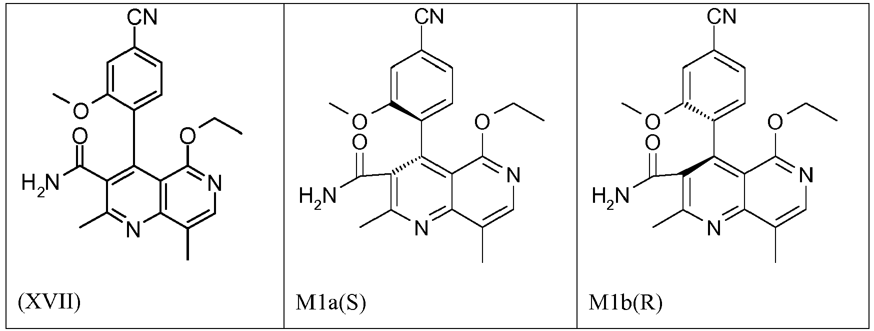 elektrochemisch in einer Becherglaszelle oder Durchflusszelle in Anwesenheit von Methanol reduziert werden,
und **dadurch gekennzeichnet, dass** die Verbindungen der Formeln (XVII), Mla(S) und Mlb(R) durch thermische Isomerisierung von Verbindungen der Formeln Mla(S) und Mlb(R) 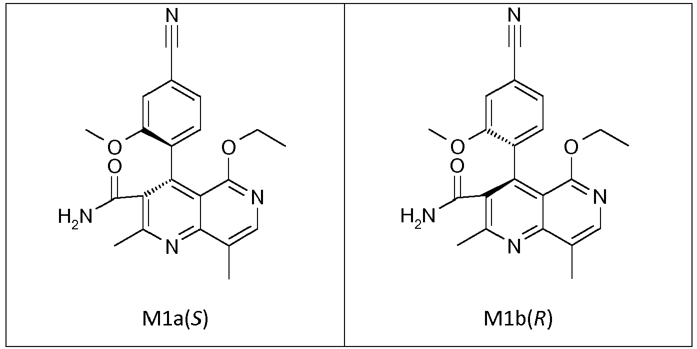 erhalten werden, und **dadurch gekennzeichnet, dass** man die Verbindung der Formel ent-(I) 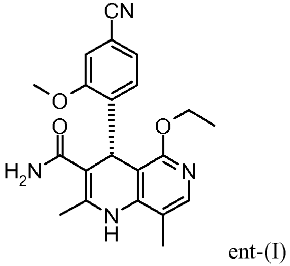 mit chemischen Oxidationsmitteln oxidiert.

## Claims

1. Process for preparing compounds of the formulae M1a(S) and Mlb(R) 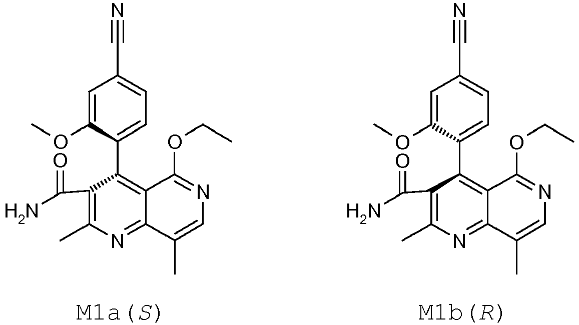 **characterized in that** the compound of the formula ent-(I) 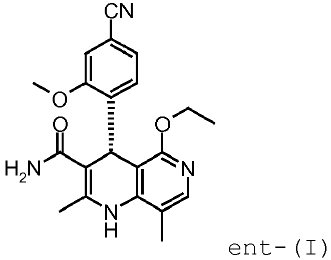 is oxidized.

2. Process for preparing compounds of the formulae M1a(s) and Mlb(R) according to Claim 1, **characterized in that** the oxidation is conducted with chemical oxidizing agents.

3. Process for preparing the racemic compound of the formula (XVII) 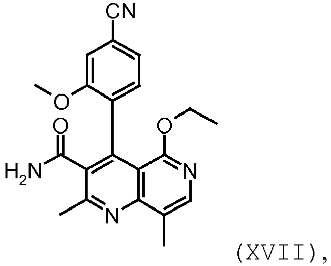 **characterized in that** a mixture of the compounds of the formulae Mla(S) and Mlb(R) 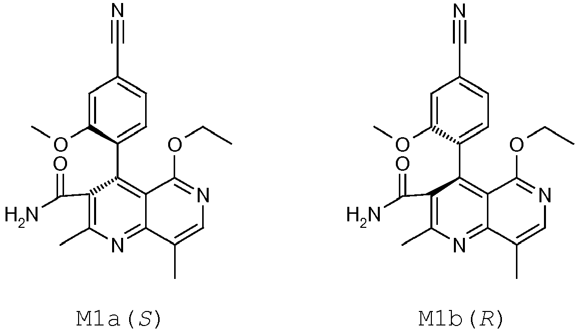 is thermally racemized.

4. Process for preparing the compound of the formula (XVII) according to Claim 3, **characterized in that** a mixture of the compounds of the formulae M1a(S) and Mlb(R) is racemized at a temperature of 70 to 110°C with or without addition of an acid.

5. Process for preparing compounds of the formula (I) and ent-(I) 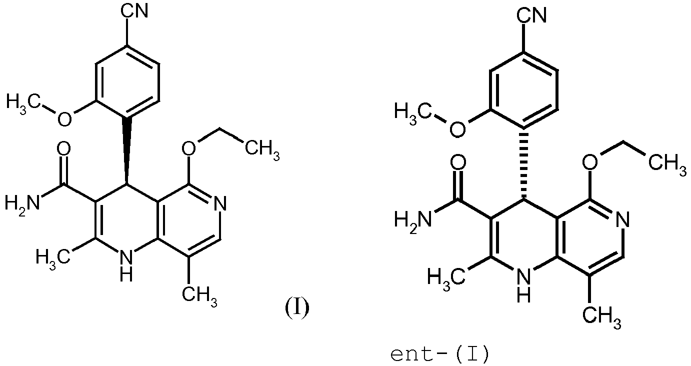 **characterized in that** compounds of the formulae (XVII) or Mla(S) or Mlb(R) or a mixture of Mla(S) and Mlb(R) 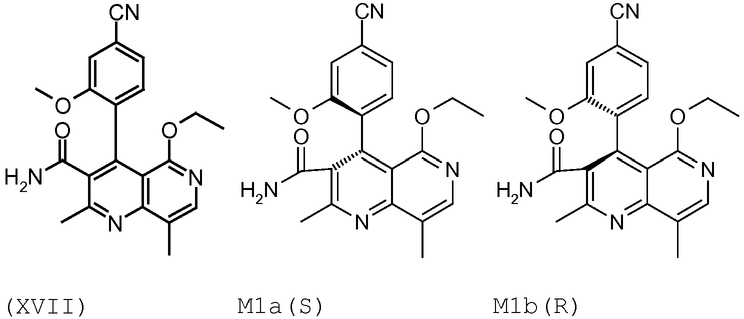 are electrochemically reduced.

6. Process for preparing the compounds of the formulae (I) and ent-(I) according to Claim 5, **characterized in that** the electrochemical reduction is conducted in a beaker cell or flow cell in the presence of methanol.

7. Process for preparing compounds of the formulae (I) and ent-(I) according to Claim 5 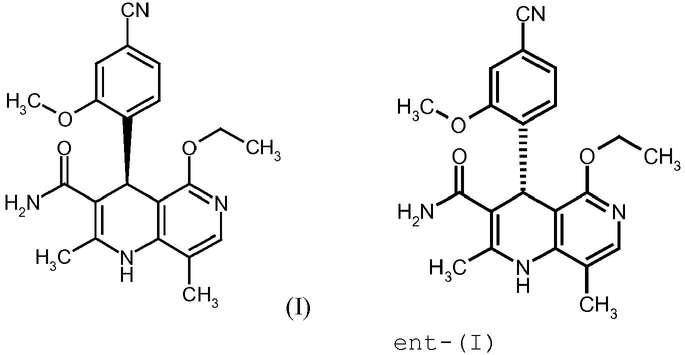 **characterized in that** compounds of the formulae (XVII) or Mla(S) or Mlb(R) or a mixture of Mla(S) and Mlb(R) 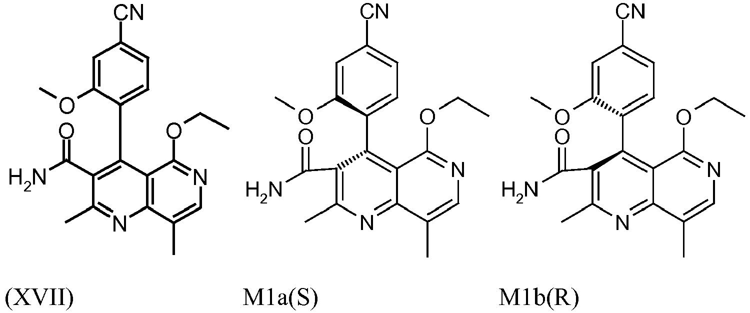 are electrochemically reduced,
and **characterized in that** the compounds of the formulae (XVII), Mla(S) and Mlb(R) are obtained by thermal isomerization of compounds of the formulae M1a(S) and M1b (R) 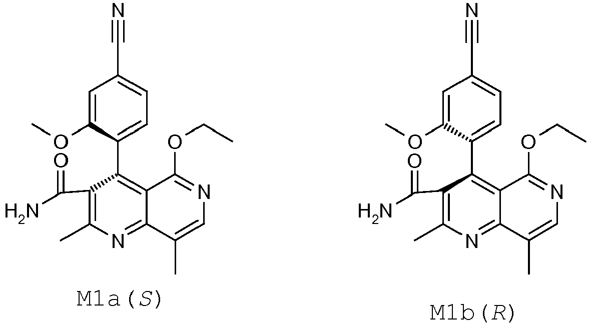 and **characterized in that** the compound of the formula ent-(I) 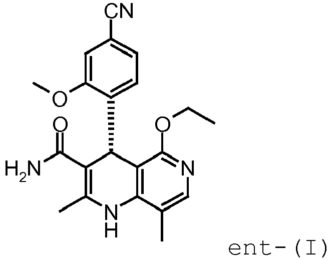 is oxidized.

8. Process for preparing compounds of the formulae (I) and ent-(I) according to Claim 7 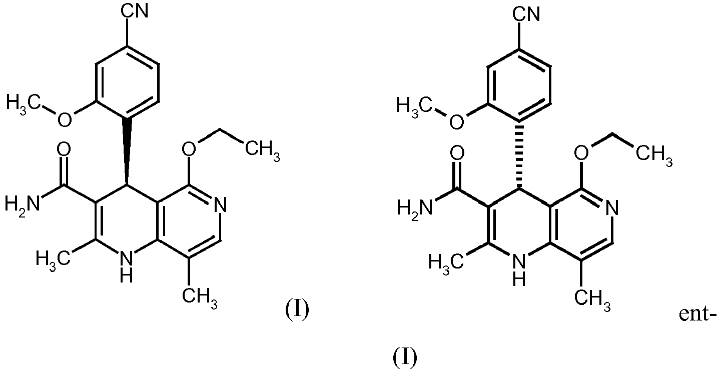 **characterized in that** compounds of the formulae (XVII) or Mla(S) or Mlb(R) or a mixture of Mla(S) and Mlb(R) 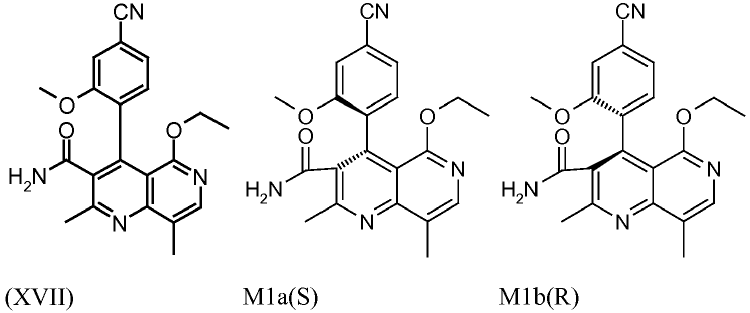 are electrochemically reduced in a beaker cell or flow cell in the presence of methanol,
and **characterized in that** the compounds of the formulae (XVII), Mla(S) and Mlb(R) are obtained by thermal isomerization of compounds of the formulae M1a(S) and M1b (R) 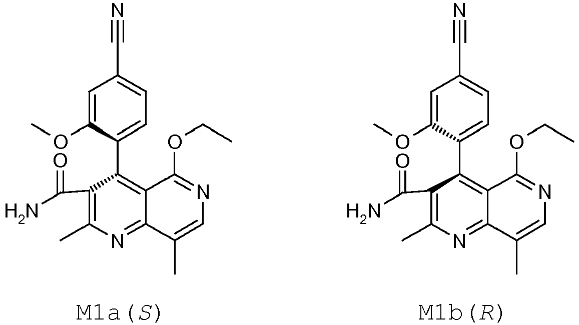 and **characterized in that** the compound of the formula ent-(I) 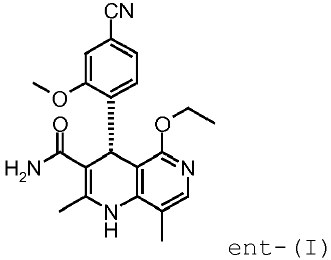 is oxidized with chemical oxidizing agents.

## Revendications

1. Procédé de fabrication de composés des formules Mla(S) et Mlb(R) 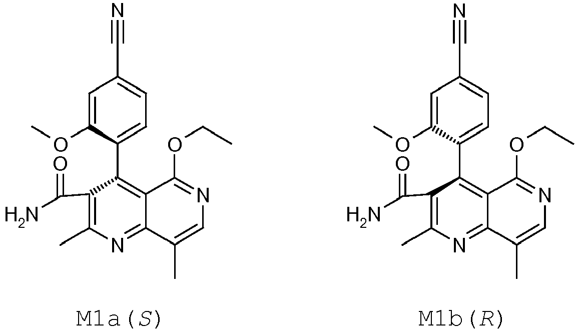 **caractérisé en ce que** le composé de formule ent-(I) 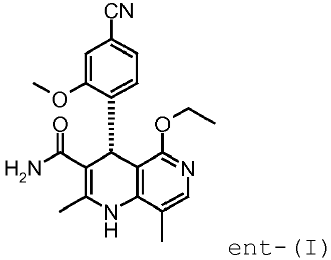 est oxydé.

2. Procédé de fabrication de composés des formules M1a(S) et M1b(R) selon la revendication 1, **caractérisé en ce que** l'oxydation est réalisée avec des oxydants chimiques.

3. Procédé de fabrication du composé racémique de formule (XVII) 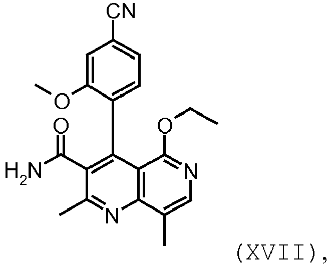 **caractérisé en ce qu'**un mélange des composés des formules Mla(S) et Mlb(R) 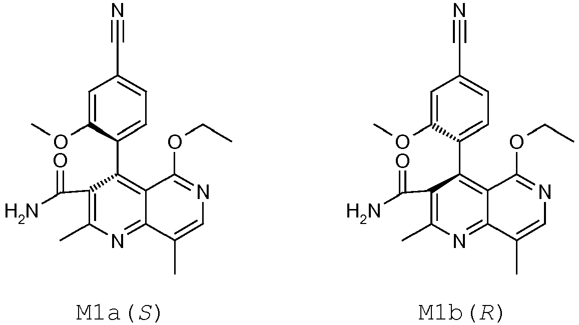 est racémisé thermiquement.

4. Procédé de fabrication du composé de formule (XVII) selon la revendication 3, **caractérisé en ce qu'**un mélange des composés des formules Mla(S) et M1b(R) est racémisé à une température de 70 à 110 °C avec ou sans ajout d'un acide.

5. Procédé de fabrication de composés de formule (I) et ent-(I) 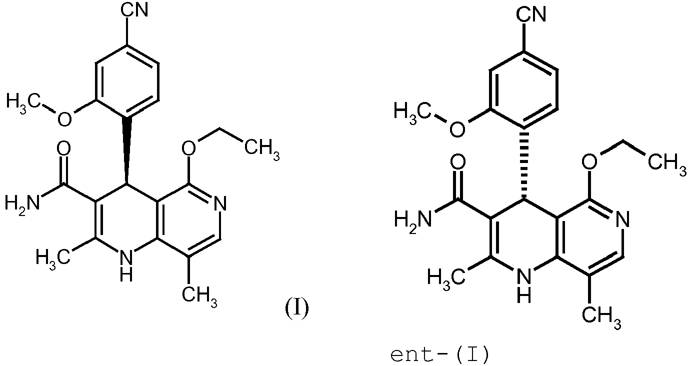 **caractérisé en ce qu'**un composé de formule (XVII) ou Mla(S) ou Mlb(R) ou un mélange de Mla(S) et Mlb(R) 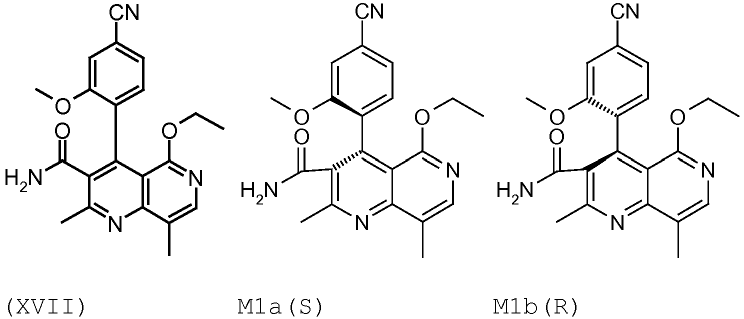 est réduit électrochimiquement.

6. Procédé de fabrication des composés des formules (I) et ent-(I) selon la revendication 5, **caractérisé en ce que** la réduction électrochimique est réalisée dans une cellule de bécher ou une cellule à débit en présence de méthanol.

7. Procédé de fabrication de composés de formule (I) et ent-(I) selon la revendication 5 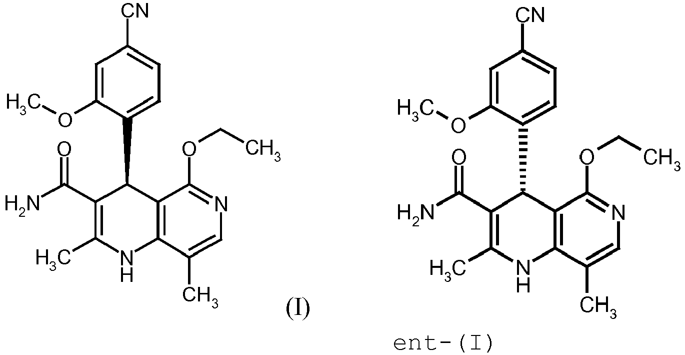 **caractérisé en ce que** des composés des formules (XVII) ou Mla(S) ou Mlb(R) ou un mélange de Mla(S) et Mlb(R) 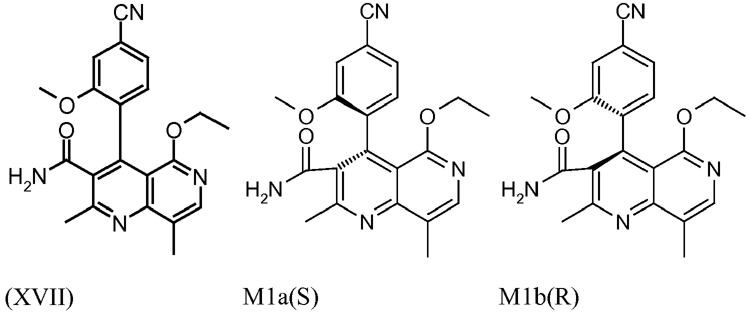 sont réduits électrochimiquement,
et **caractérisé en ce que** les composés des formules (XVII), Mla(S) et Mlb(R) sont obtenus par isomérisation thermique de composés des formules Mla(S) et Mlb(R) 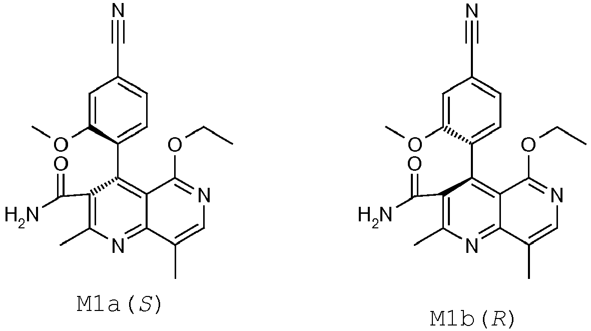 et **caractérisé en ce que** le composé de formule ent-(I) 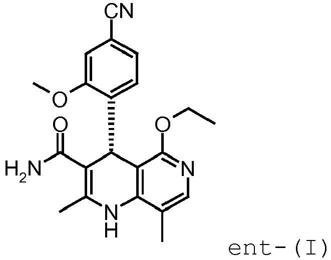 est oxydé.

8. Procédé de fabrication de composés de formule (I) et ent-(I) selon la revendication 7 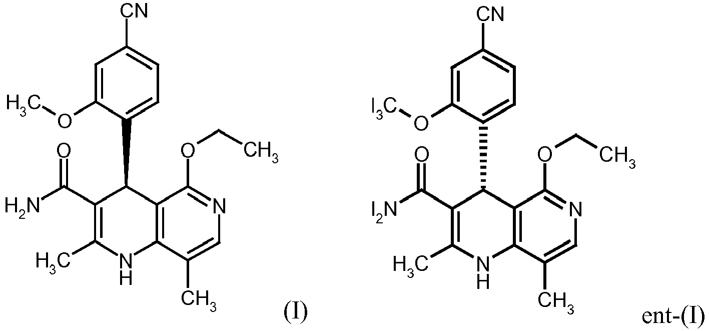 **caractérisé en ce que** des composés des formules (XVII) ou Mla(S) ou Mlb(R) ou un mélange de Mla(S) et Mlb(R) 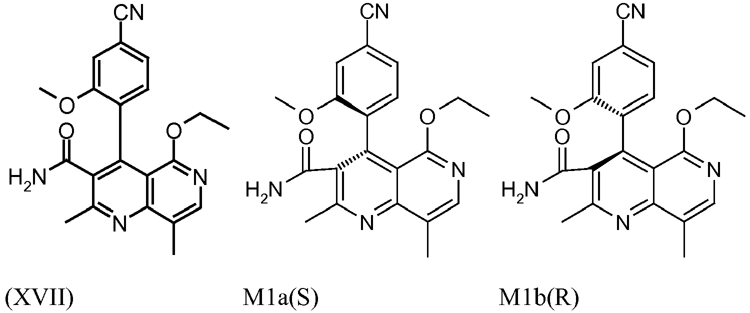 est réduit électrochimiquement dans une cellule de bécher ou une cellule à débit en présence de méthanol, et **caractérisé en ce que** les composés des formules (XVII), Mla(S) et Mlb(R) sont obtenus par isomérisation de composés des formules Mla(S) et Mlb(R) 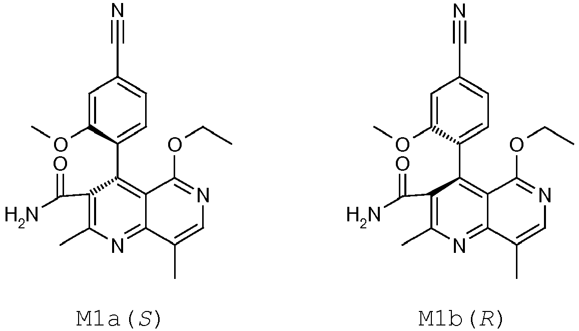 et **caractérisé en ce que** le composé de formule ent-(I) 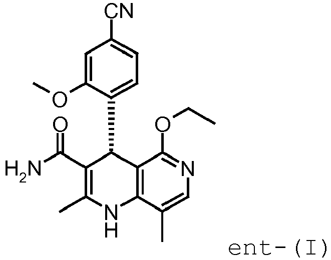 est oxydé avec des oxydants chimiques.
